# EUROPEAN PATENT APPLICATION

(11) **EP 4 847 496 A1**
(43) Date of publication of application: **02.12.2026**
(21) Application number: 25744737.5
(22) Date of filing: 24.01.2025
(51) Int. Cl.: C07D 401/12, C07D 401/14, C07D 487/04, C07D 413/14, C07D 413/12, A61K 31/506, A61K 31/4439, A61K 31/444, A61P 35/00

(54) **RING-SUBSTITUTED AMIDE COMPOUNDS, COMPOSITION AND USE THEREOF**

(30) Priority: 25.01.2024 CN 202410107219; 13.03.2024 CN 202410288450; 19.06.2024 CN 202410793797
(71) Applicant: Simcere Zaiming Pharmaceutical Co., Ltd., Haikou, Hainan 570311 (CN)
(72) Inventor: YU, Jian, Shanghai 201318 (CN); YANG, Yang, Shanghai 201318 (CN); ZHOU, Muxing, Shanghai 201318 (CN); ZHU, Wei, Shanghai 201318 (CN); SUN, Tianwen, Shanghai 201318 (CN); GAO, Donglin, Shanghai 201318 (CN); ZHOU, Feng, Shanghai 201318 (CN); ZHOU, Lei, Shanghai 201318 (CN); LI, Zhengtao, Shanghai 201318 (CN)
(74) Representative: Potter Clarkson
(86) International application number: PCT/CN2025/074827
(87) International publication number: WO 2025/157274

(57) **Abstract**

Provided are novel ring-substituted amide compounds or pharmaceutically acceptable salts thereof, a pharmaceutical composition containing same, and the use thereof as a KIF18A inhibitor in the prevention or treatment of related diseases.

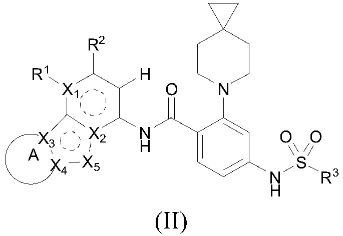

## Description

### CROSS-REFERENCE TO RELATED APPLICATIONS

The present disclosure claims the benefit of and priority to the following three Chinese Patent Applications for Invention, the contents of which are incorporated herein by reference in their entirety:
Chinese Patent Application No. CN202410107219.5 filed with China National Intellectual Property Administration on Jan. 25, 2024,
Chinese Patent Application No. CN202410288450.9 filed with China National Intellectual Property Administration on Mar. 13, 2024, and
Chinese Patent Application No. CN202410793797.9 filed with China National Intellectual Property Administration on Jun. 19, 2024.

### TECHNICAL FIELD

The present disclosure relates to a ring-substituted amide compound or a pharmaceutically acceptable salt thereof, a pharmaceutical composition containing same, and use thereof as a KIF 18A inhibitor in the prevention or treatment of related diseases.

### BACKGROUND

Cancer is generally characterized by unregulated cell proliferation. Damage to one or more genes of the cell proliferation pathway may lead to the loss of normal regulation of cell proliferation. These deregulated genes can encode various tumor suppressors or oncogene proteins, leading to unchecked cell cycle progression and cell proliferation. Various kinases and kinesins have been identified as key regulators of cell cycle, mitotic regulation, and progression of normal cells and cancer cells.

Kinesins are molecular motors that play important roles in cell division and intracellular transport of vesicles and organelles. Mitotic kinesins play a role in spindle assembly, chromosome segregation, centrosome segregation, and many other aspects. Based on sequence homology within the so-called "motor domain", human kinesins are classified into 14 subfamilies. The activity of ATPase of the domain enables unidirectional movement along microtubules. The non-motor domains of these proteins are responsible for attachment to the "cargo"; the "cargo" may be one of a variety of membranous organelles, signal transduction scaffold systems, and chromosomes. Kinesins use the energy resulting from ATP hydrolysis to move the "cargo" along polarized microtubules. Therefore, kinesins are often referred to as "plus-end-directed" or "minus-end-directed" motor proteins. The KIF18A gene belongs to the kinesin-8 subfamily and is a plus-end-directed motor protein. KIF18A is believed to affect the movement of the plus end of the kinetochore microtubules to control proper chromosome positioning and maintain spindle tension. Removal of human KIF18A results in longer spindles in HeLa cervical cancer cells, increases chromosome oscillation in the metaphase of mitosis, and results in activation of the spindle assembly checkpoint. Thus, KIF18A may be a viable target for cancer therapy, with KIF18A being overexpressed in various types of cancer. In addition, in cancer cells, the deletion, knockout, or inhibition of the KIF18A gene affects the mitotic spindle. Specifically, it has been found that inhibiting KIF18A induces mitotic arrest, ultimately leading to mitotic catastrophe of cancer cells or mitotic slippage in interphase and thereby resulting in apoptosis of cancer cells.

Therefore, inhibitors of KIF18A can be developed into promising anticancer drugs, and research institutions are highly interested in discovering inhibitors of the KIF 18A protein.

### SUMMARY

The present disclosure provides a compound represented by formula (II) or a pharmaceutically acceptable salt thereof: 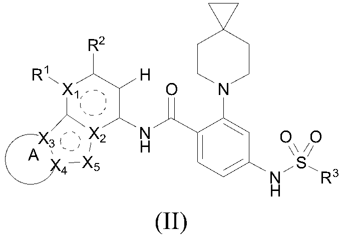 wherein in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, X₃, X₄, and X₅ are each independently CR^{a} or nitrogen, wherein R^{a} are each independently selected from being absent, H, or halogen;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl or C₂-C₆ alkenyl, wherein the C₁-C₆ alkyl is optionally substituted with one or more halogen or OH.

In some embodiments, R³ is selected from C₁-C₆ alkyl optionally substituted with one or more halogen or OH.

In some embodiments, in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, X₃, X₄, and X₅ are each independently CR^{a} or nitrogen, wherein R^{a} are each independently selected from being absent, H, or halogen;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, C₁-C₆ alkyl, cyano, C₁-C₆ alkoxy, C(O)R^{d}, halogen, C₁-C₆ haloalkyl, or C₁-C₆ haloalkoxy; R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl optionally substituted with one or more halogen or OH.

In some embodiments, in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, X₃, X₄, and X₅ are each independently CR^{a} or nitrogen, wherein R^{a} are each independently selected from being absent, H, or halogen;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl optionally substituted with one or more halogen or OH.

In some embodiments, in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, X₃, X₄, and X₅ are each independently CR^{a} or nitrogen, wherein R^{a} are each independently selected from being absent, H, or halogen;
R¹ is absent, H, C₁-C₆ alkyl, cyano, C₁-C₆ alkoxy, OH, halogen, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl, wherein the C₁-C₆ alkyl is optionally substituted with one or more halogen or OH.

In some embodiments, in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₄, and X₅ are each independently CR^{a} or nitrogen, wherein R^{a} are each independently selected from being absent, H, or halogen;
one of X₂ and X₃ is C, and the other is N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl, wherein the C₁-C₆ alkyl is optionally substituted with one or more halogen or OH.

In some embodiments, X₁ is C, one of X₂ and X₃ is C, and the other is N.

In some embodiments, R¹ is absent, H, C₁-C₆ alkyl, cyano, C₁-C₆ alkoxy, (CH₂)ᵣ-C(O)R^{d}, halogen, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂.

In some embodiments, the compound represented by formula (II) or the pharmaceutically acceptable salt thereof of the present disclosure is selected from a compound represented by formula (III') or a pharmaceutically acceptable salt thereof: 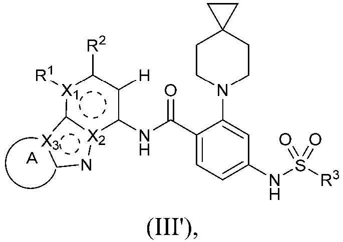 wherein in formula (III'):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl or C₂-C₆ alkenyl, wherein the C₁-C₆ alkyl is optionally substituted with one or more halogen or OH.

In some embodiments, R³ is selected from C₁-C₆ alkyl optionally substituted with one or more halogen or OH.

In some embodiments, in formula (III'):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, C₁-C₆ alkyl, cyano, C₁-C₆ alkoxy, C(O)R^{d}, halogen, C₁-C₆ haloalkyl, or C₁-C₆ haloalkoxy; R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl optionally substituted with one or more halogen or OH.

In some embodiments, in formula (III'):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl optionally substituted with one or more halogen or OH.

In some embodiments, in formula (III'):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, OH, halogen, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl, wherein the C₁-C₆ alkyl is optionally substituted with one or more halogen or OH.

In some embodiments, in formula (III'):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁ is independently C or N; one of X₂ and X₃ is C, and the other is N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl, wherein the C₁-C₆ alkyl is optionally substituted with one or more halogen or OH.

In some embodiments, in formula (II), formula (III'), and its subformula (III) below, X₁ is C or N, X₂ is N, and X₃ is C.

In some embodiments, in formula (II), formula (III'), and its subformula (III) below, X₁ is C, X₂ is N, and X₃ is C; A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl).

In some embodiments, the compounds represented by formula (II), formula (III'), and its subformula (III) below do not include the following compounds:

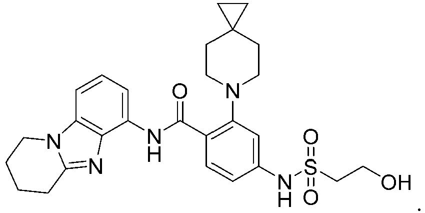

In some embodiments, the compounds represented by formula (II), formula (III'), and its subformula (III) below do not include the following compounds:

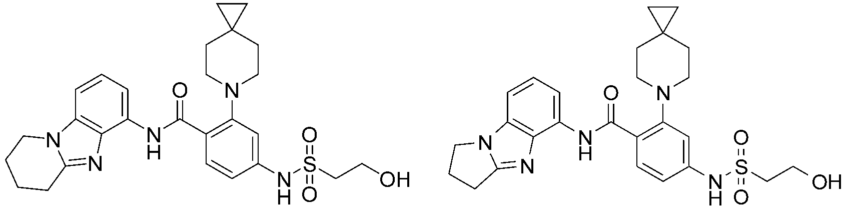

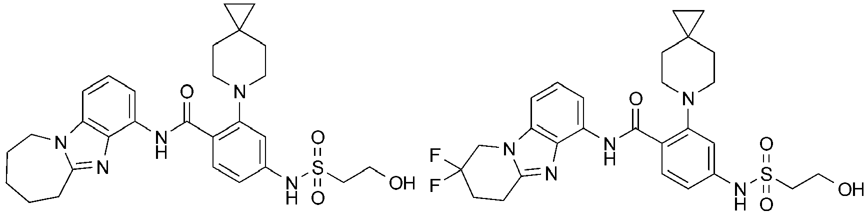

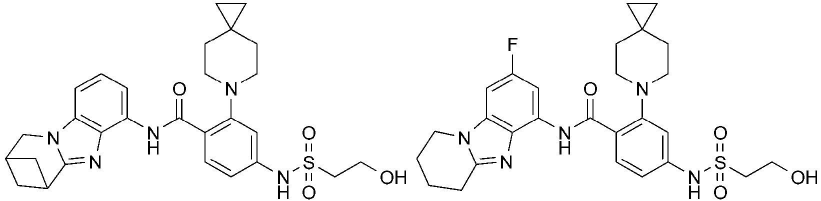

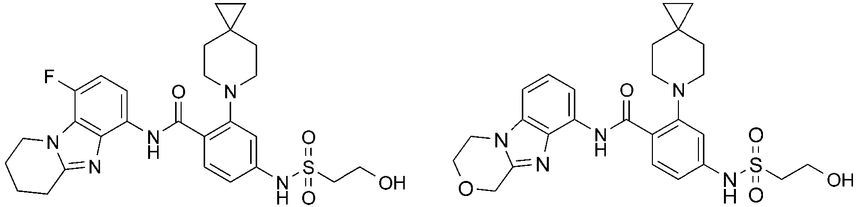

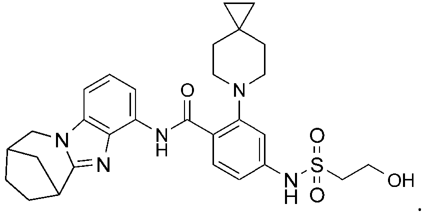

In some embodiments, the compounds represented by formula (II), formula (III'), and its subformula (III) below do not include the following compounds:

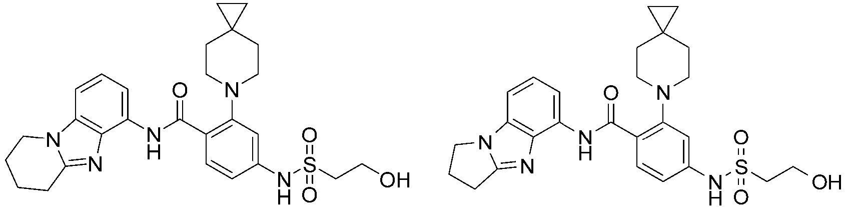

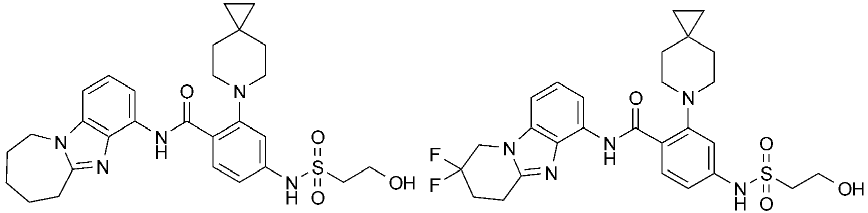

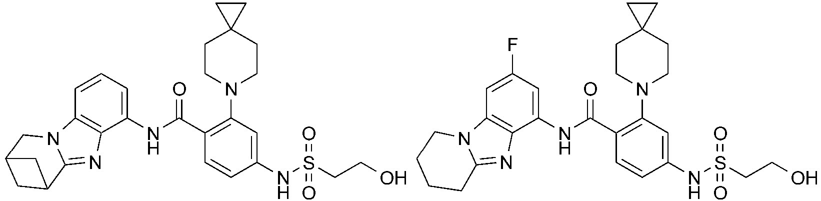

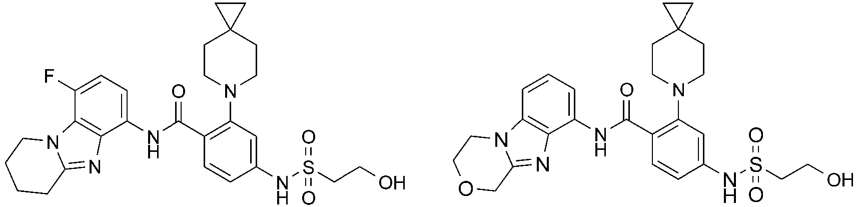

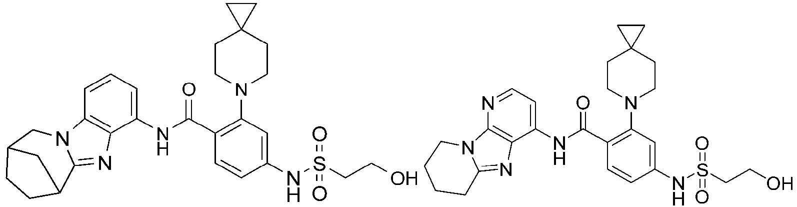

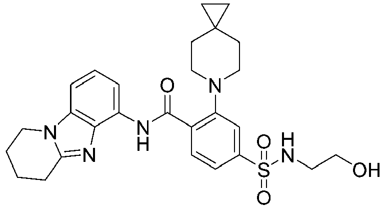

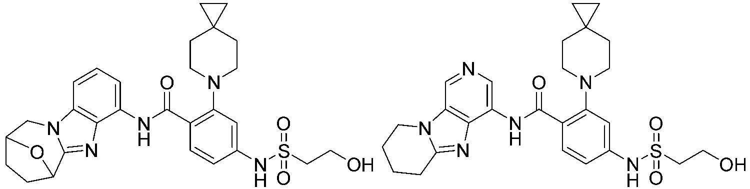

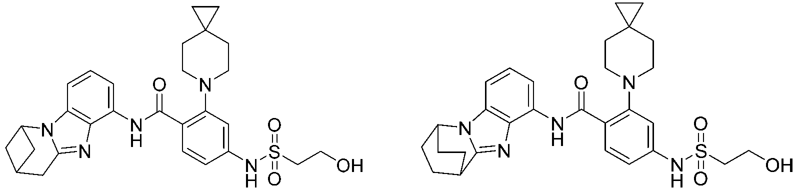

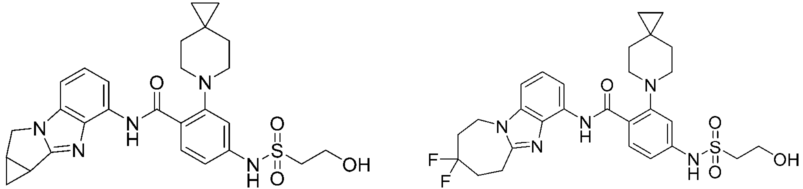

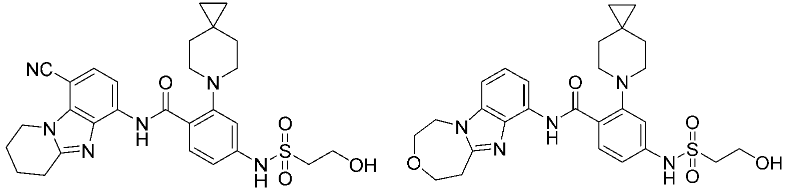

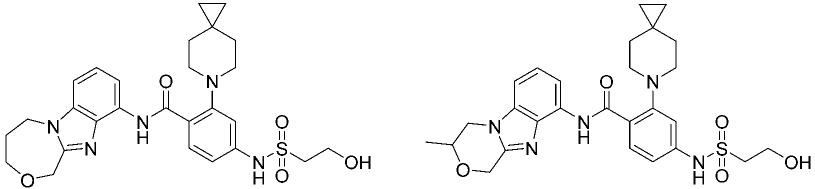

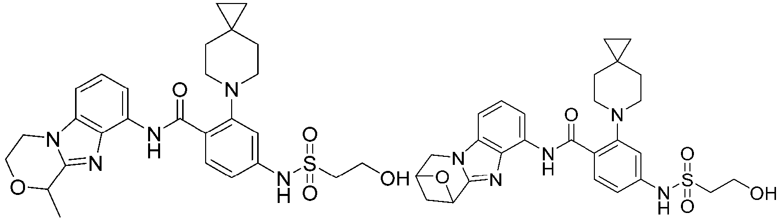

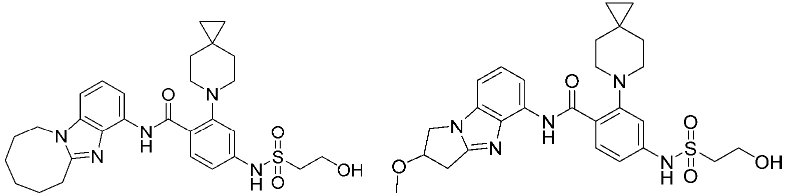

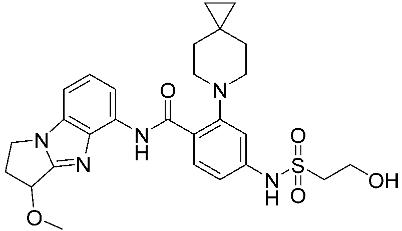

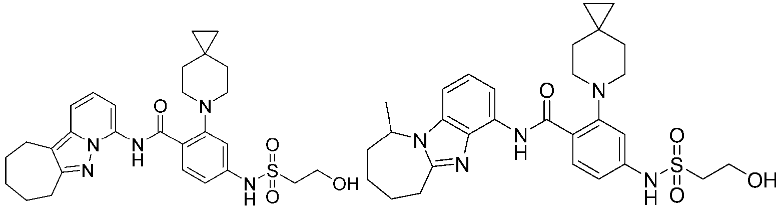

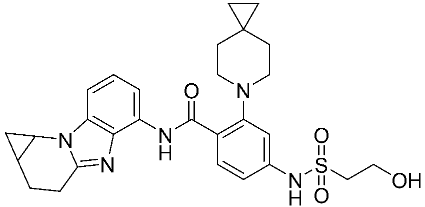

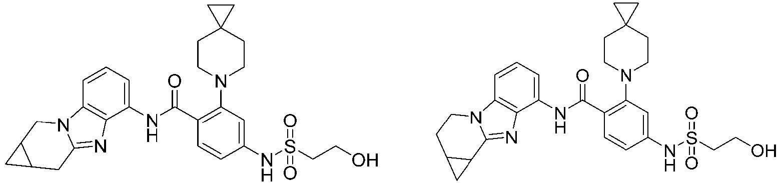

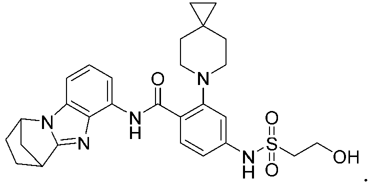

In some embodiments, the compounds represented by formula (II), formula (III'), and its subformula (III) below do not include the following compounds: 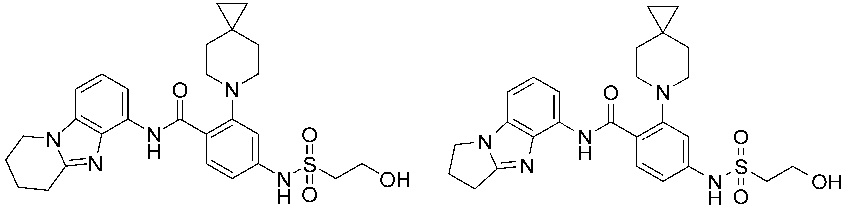 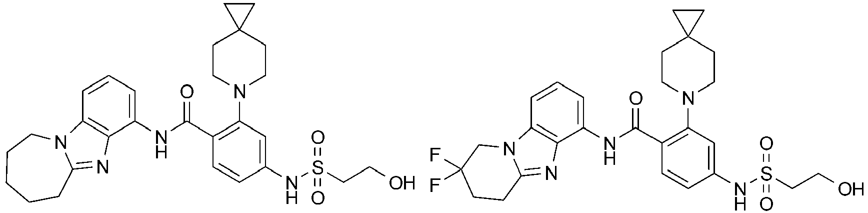 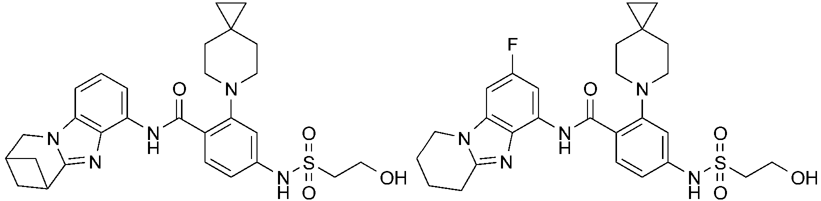 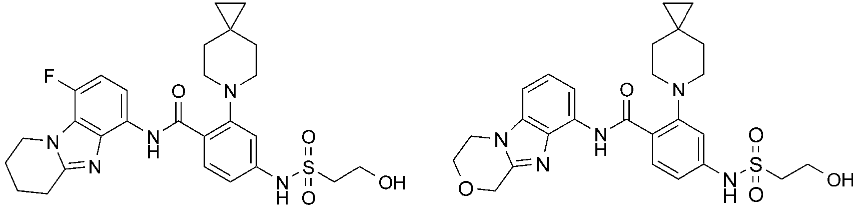 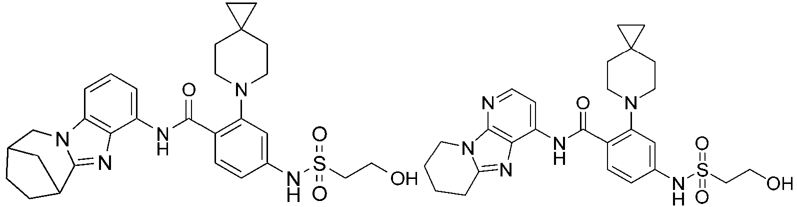 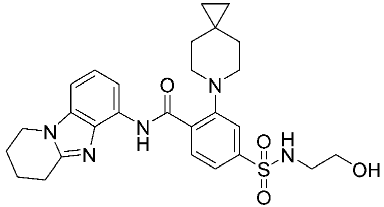 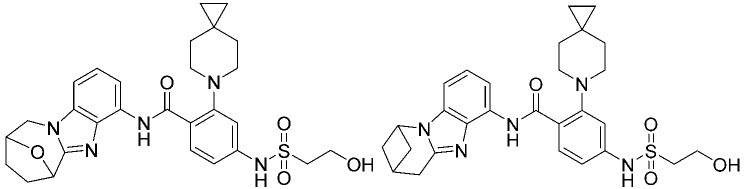 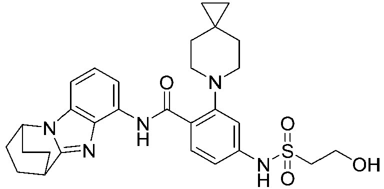 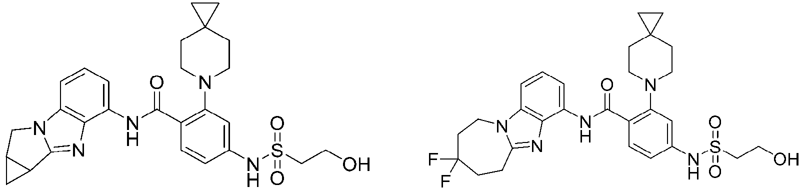 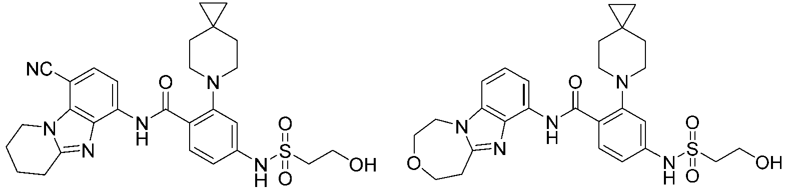 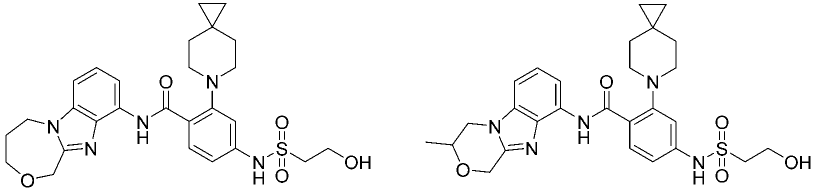 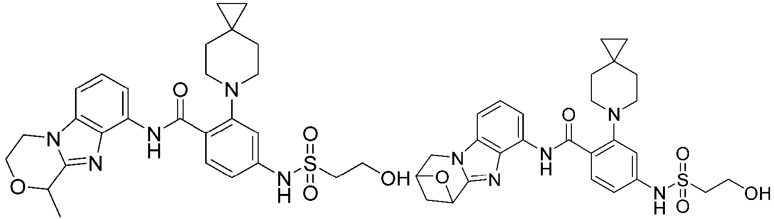 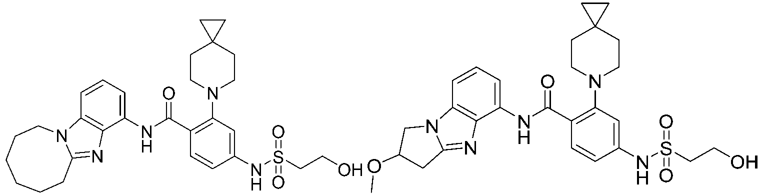 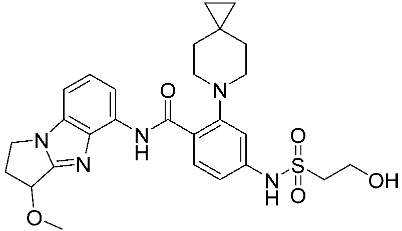 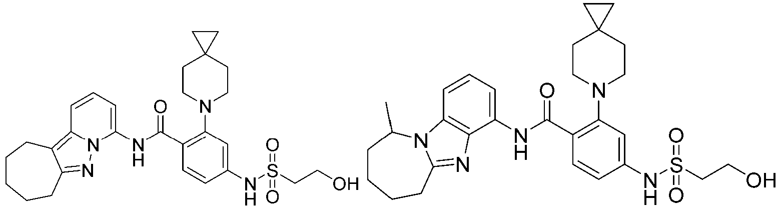 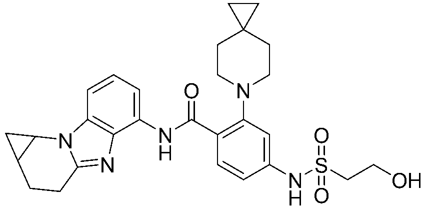 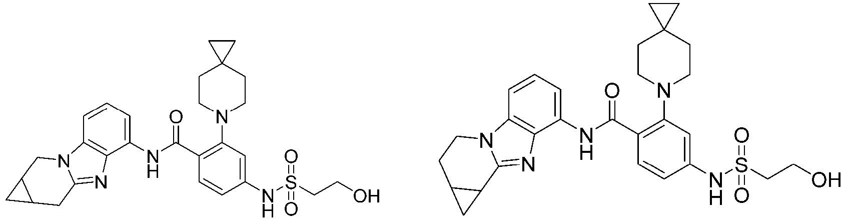 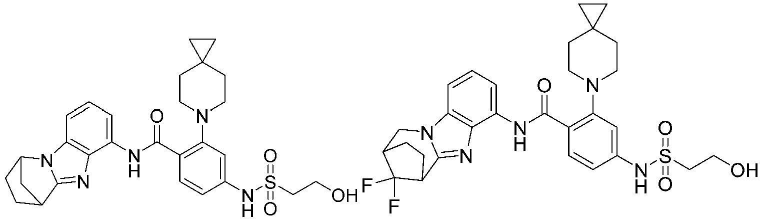 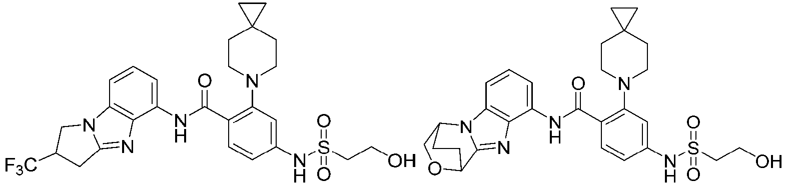 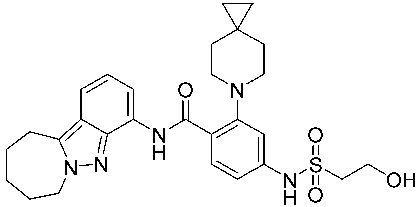 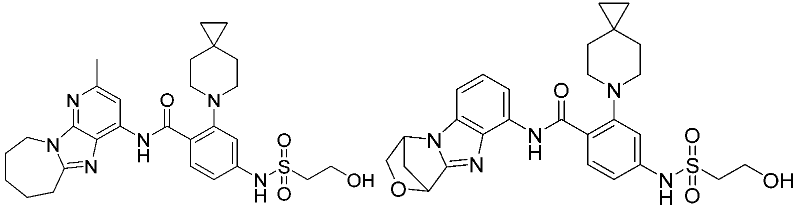 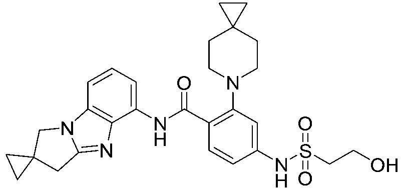 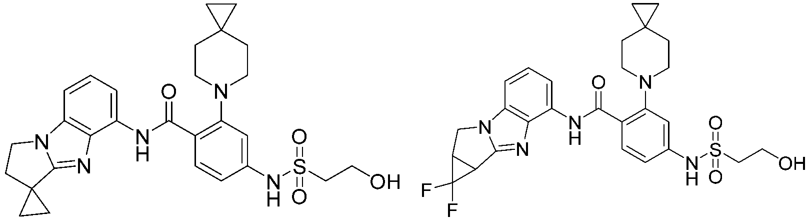 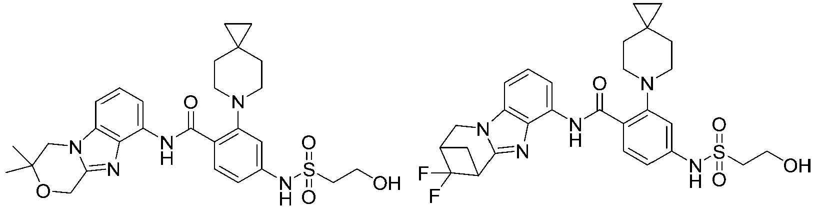 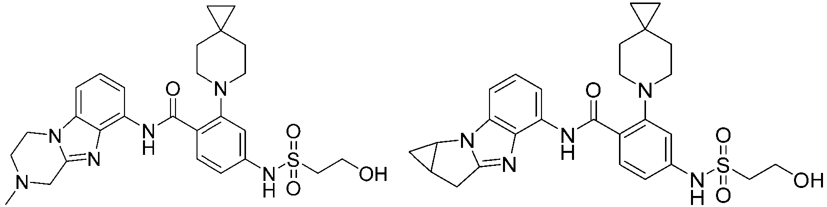 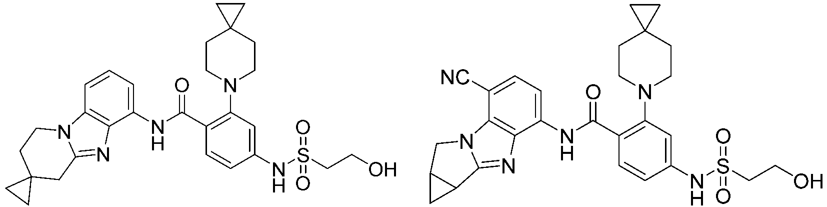 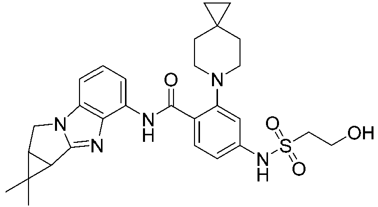 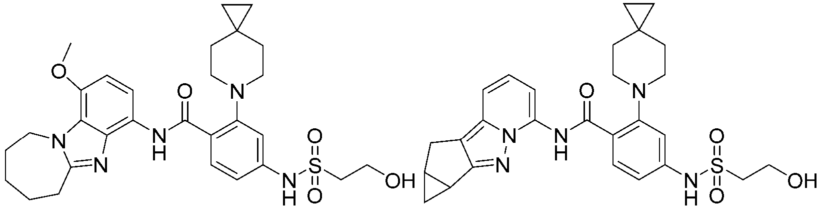 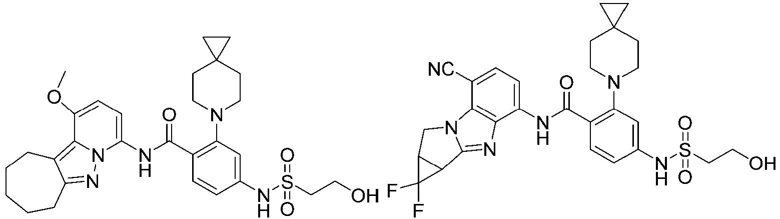 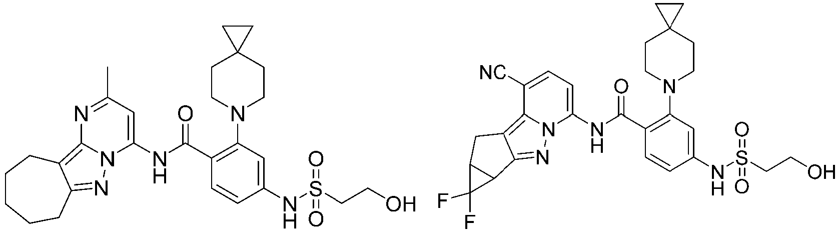 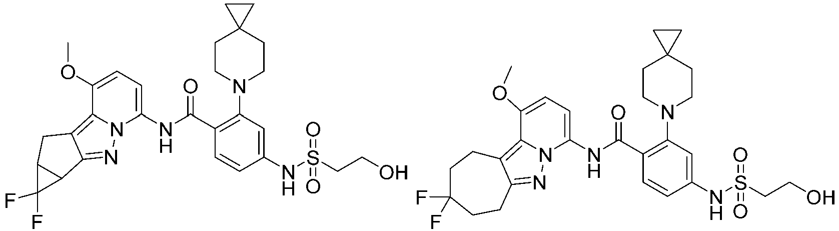 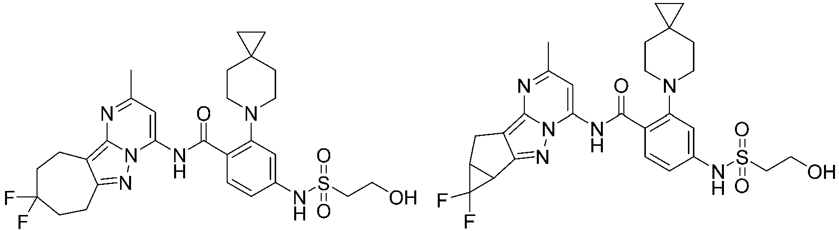 and

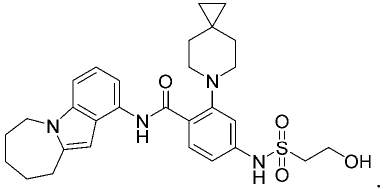

In some embodiments, in the compounds represented by formula (II), formula (III'), and its subformula (III) below, the structural unit 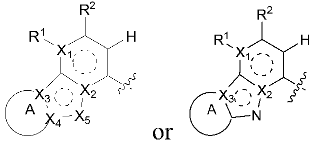 is not the following structures:

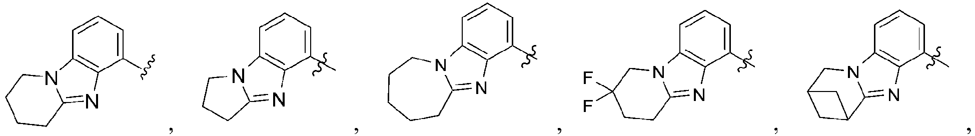

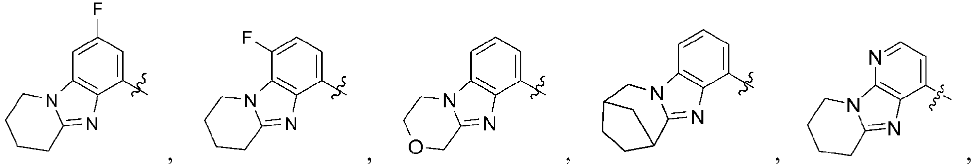

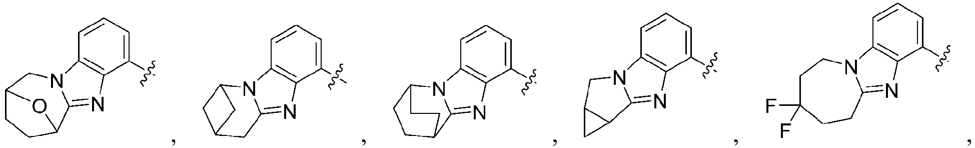

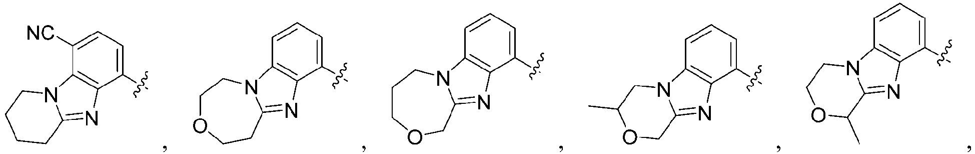

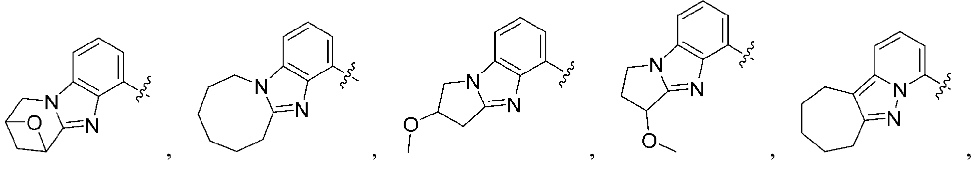

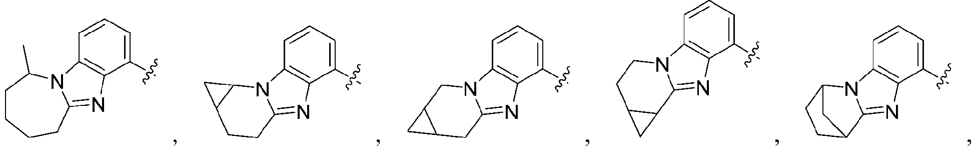

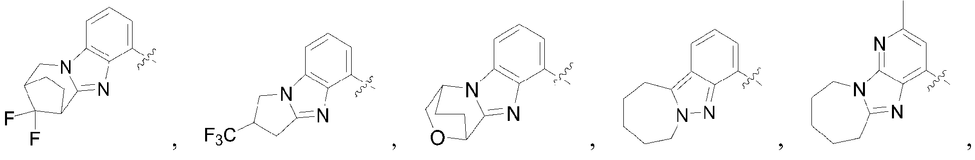

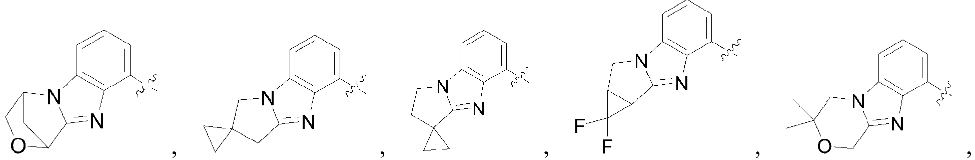

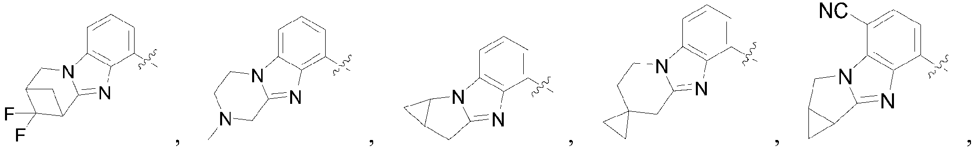

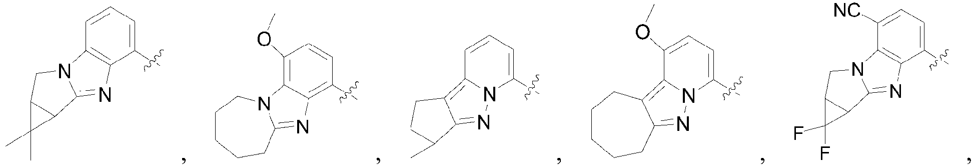

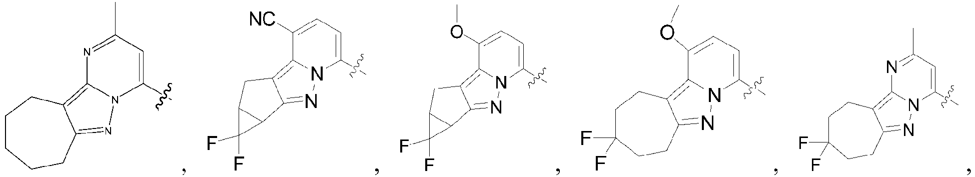

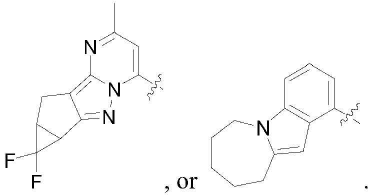

In some embodiments, in the compounds represented by formula (II), formula (III'), and its subformula (III) below:
when A is a monocyclic 5-membered heterocyclic ring containing only one N atom as a heteroatom, the monocyclic 5-membered heterocyclic ring containing only one N atom as a heteroatom is substituted with one or more R^{b}, wherein R^{b} is as defined in formula (II), but R^{b} is not methoxy or -CF₃;
when A is a monocyclic 6-membered heterocyclic ring containing only one N atom as a heteroatom, and X₁ is C, the compound satisfies at least one of the following conditions (1) and (2): (1) R¹ and R² are not both hydrogen, and R² is not F when R¹ is hydrogen, and R¹ is not F or cyano when R² is hydrogen; (2) ring A is substituted with one or more R^{b}, and R^{b} is not F;
when A is a monocyclic 6-membered heterocyclic ring containing only one N atom as a heteroatom and X₁ is N, the compound satisfies at least one of the following conditions (1) and (2): (1) R² is not hydrogen; (2) ring A is substituted with one or more R^{b};
when A is a monocyclic 6-membered heterocyclic ring containing one N and one O or containing two N as heteroatoms, the compound satisfies at least one of the following conditions (1) and (2): (1) R¹ and R² are not both hydrogen; (2) ring A is substituted with one or more R^{b}, and R^{b} is not methyl;
when A is a bicyclic 6-membered carbocyclic or heterocyclic ring containing only one N atom as a heteroatom, and the bicyclic 6-membered carbocyclic or heterocyclic ring is a fused ring of a 3-membered ring and a 5-membered ring, the compound satisfies at least one of the following conditions (1) and (2): (1) R¹ and R² are not both hydrogen, and R¹ is not cyano when R² is hydrogen; (2) the bicyclic 6-membered carbocyclic or heterocyclic ring is substituted with one or more R^{b}; when the bicyclic 6-membered heterocyclic ring is substituted with two F or two methyl groups as R^{b}, the bicyclic 6-membered heterocyclic ring is additionally substituted with another one or more R^{b}, and/or R¹ is not hydrogen or cyano; when the bicyclic 6-membered carbocyclic ring is substituted with two F as R^{b}, the bicyclic 6-membered carbocyclic ring is additionally substituted with another one or more R^{b}, and/or R¹ is not absent, cyano, or methoxy; the another one or more R^{b} are as defined in formula II;
when A is a 7-membered ring, the 7-membered ring is selected from a monocyclic 7-membered carbocyclic ring or 7-membered heterocyclic ring, a bicyclic 7-membered carbocyclic ring, or a fused bicyclic 7-membered heterocyclic ring; and the fused bicyclic 7-membered heterocyclic ring is a bicyclic ring formed by fusing a 4-membered ring with a 5-membered ring;
when A is a monocyclic 7-membered heterocyclic ring containing one N and one O as heteroatoms, the compound satisfies at least one of the following conditions (1) and (2): (1) ring A is substituted with one or more R^{b}; (2) R¹ and R² are not both hydrogen;
when A is an 8-membered ring, the 8-membered ring is selected from a monocyclic 8-membered carbocyclic ring or 8-membered heterocyclic ring, a bicyclic 8-membered carbocyclic ring, or a fused bicyclic 8-membered heterocyclic ring;
when A is a monocyclic 8-membered heterocyclic ring containing one N as a heteroatom, the compound satisfies at least one of the following conditions (1) and (2): (1) ring A is substituted with one or more R^{b}; (2) R¹ and R² are not both hydrogen.

In some embodiments, in formula (II) or formula (III'), when one of R¹ and R² is H, the other is not H, methyl, CN, or halogen.

In some embodiments, in formula (II) or formula (III'), A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from OH, F, or C₁-C₄ alkyl, wherein the C₁-C₄ alkyl is optionally substituted with one or more groups independently selected from F or O(C₁-C₄ alkyl).

In some embodiments, in formula (II) or formula (III'), A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from F or C₁-C₄ alkyl, wherein the C₁-C₄ alkyl is optionally substituted with one or more groups independently selected from F or O(C₁-C₄ alkyl).

In some embodiments, in formula (II) or formula (III'), the structural unit 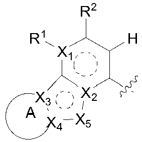 or 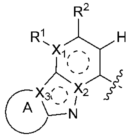 is selected from 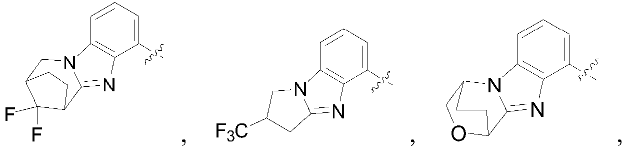 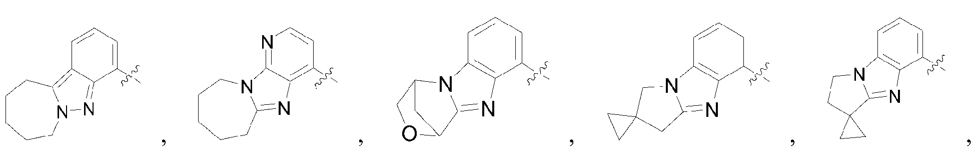 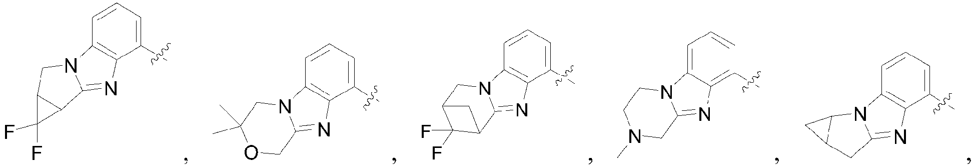 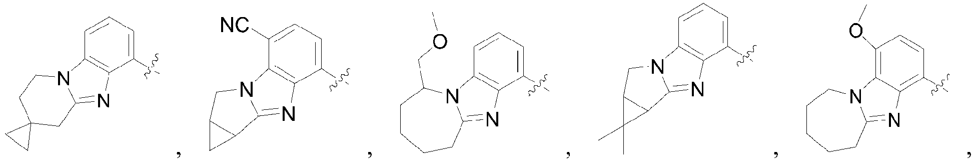 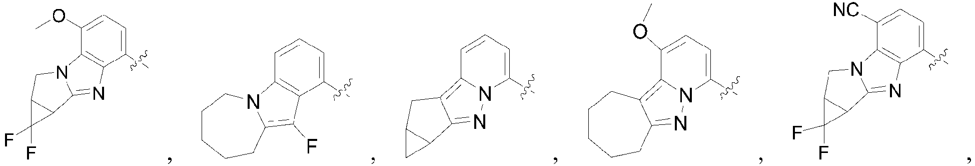 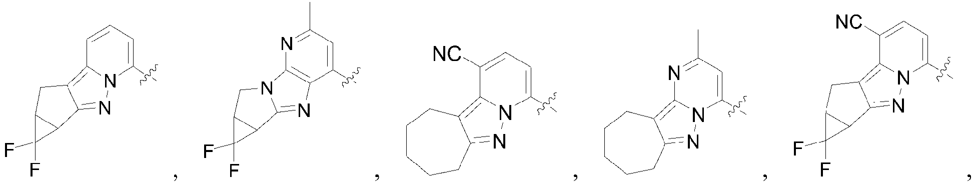 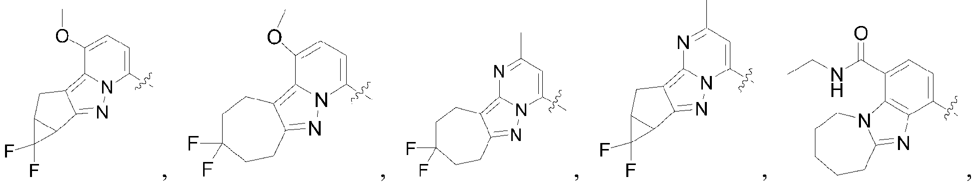 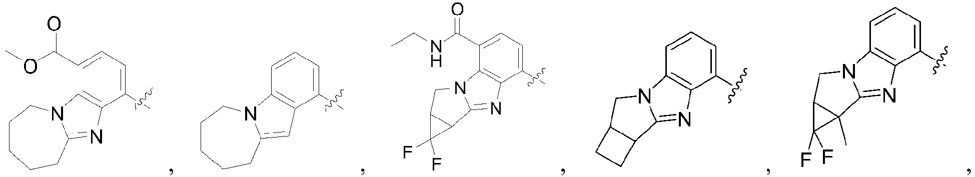 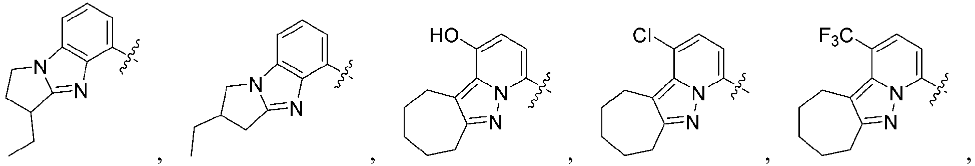 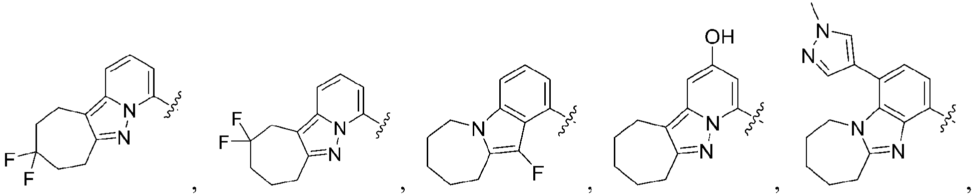 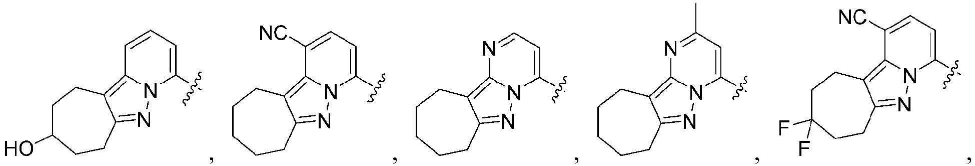 or

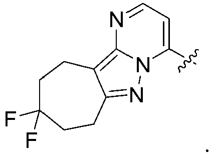

In some embodiments, in formula (II) or formula (III'), the structural unit 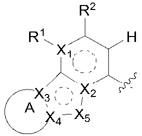 or 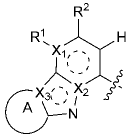 is selected from 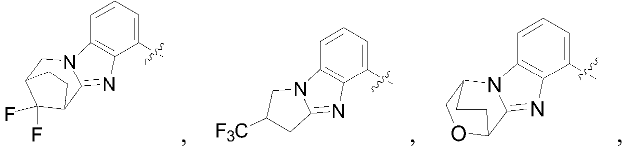 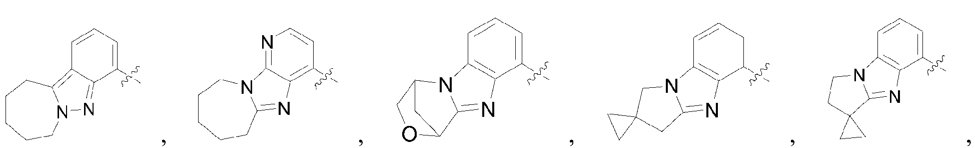 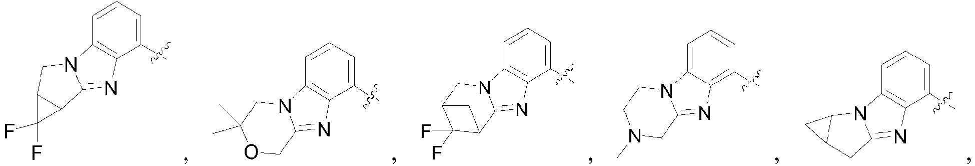 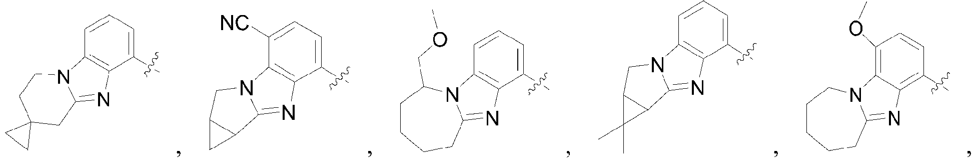 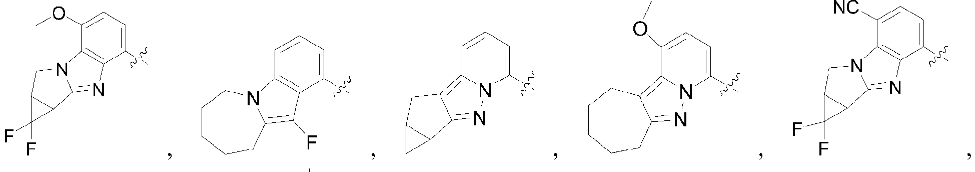 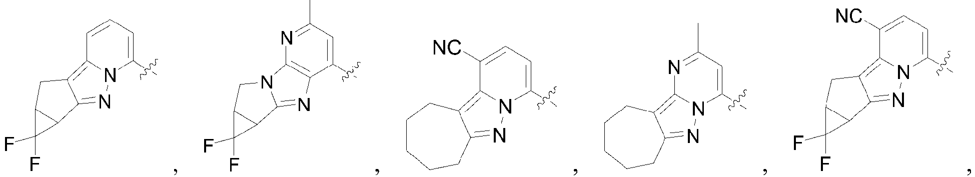 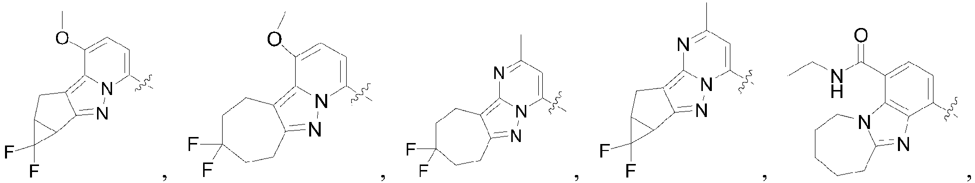 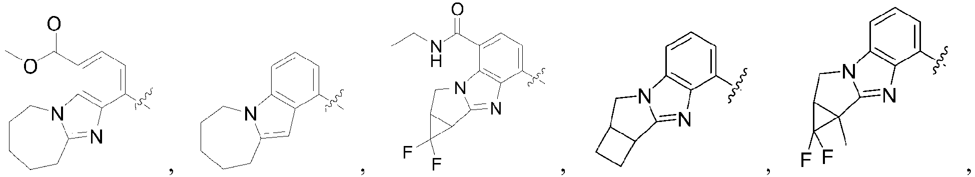 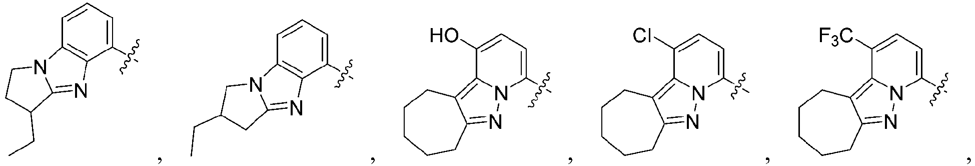 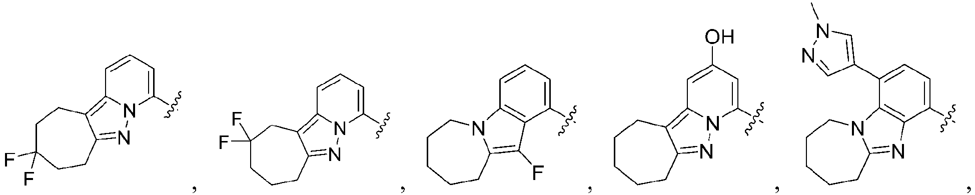 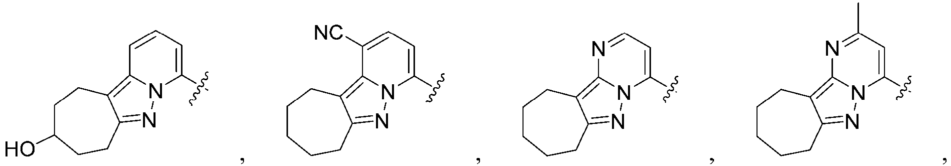 or

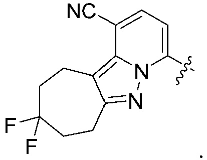

In some embodiments, in formula (II) or formula (III'), the structural unit 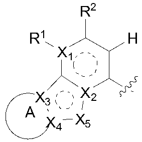 is selected from

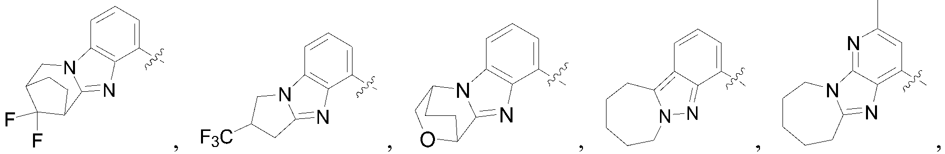

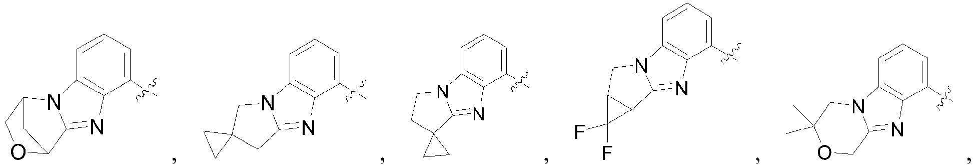

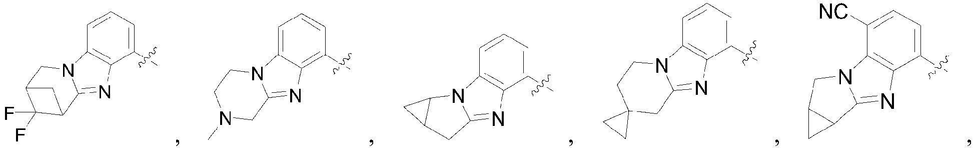

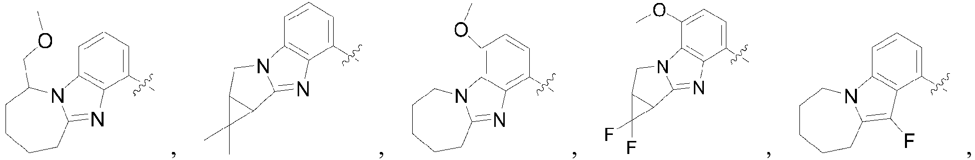

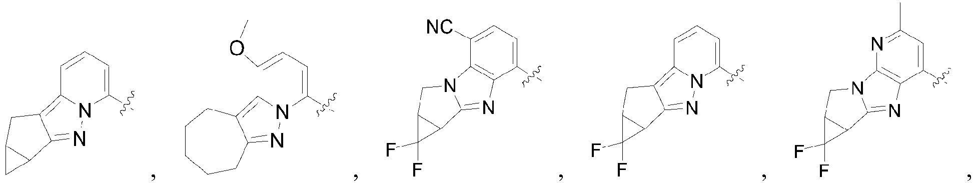

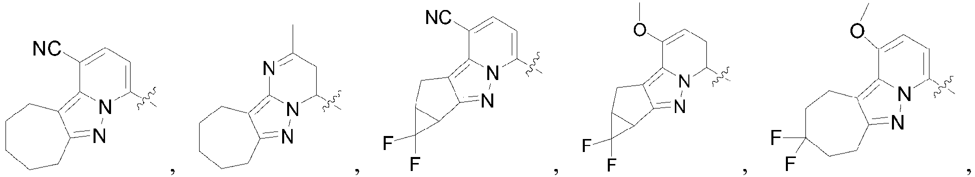

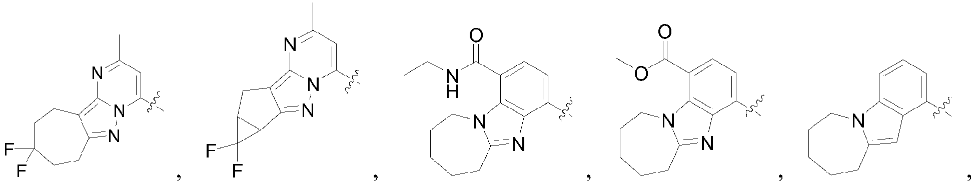

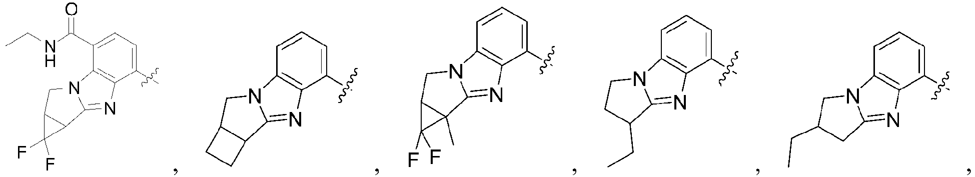

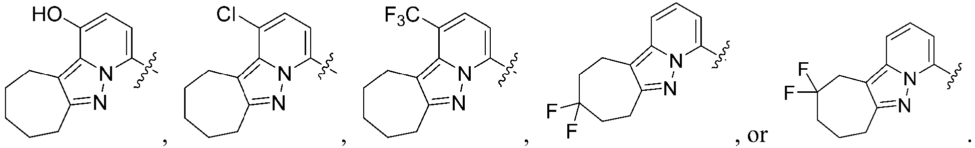

In some embodiments, in formula (II), X₁, X₂, X₃, X₄, and X₅ are each independently CR^{a} or N, wherein R^{a} are each independently selected from being absent, H, or F.

In some embodiments, in formula (II), X₁, X₂, X₃, X₄, and X₅ are each independently C or N.

In some embodiments, in formula (II), X₅ is N.

In some embodiments, in formula (II), at least one of X₁, X₂, X₃, X₄, and X₅ is N.

In some embodiments, in formula (II), one, two, or three of X₁, X₂, X₃, X₄, and X₅ are N.

In some embodiments, in formula (II), two of X₁, X₂, X₃, X₄, and X₅ are N.

In some embodiments, in formula (II), three of X₁, X₂, X₃, X₄, and X₅ are N.

In some embodiments, in formula (II), X₂ and X₅ are N; in some embodiments, in formula (II), X₃ and X₅ are N; in some embodiments, in formula (II), X₄ and X₅ are N.

In some embodiments, in formula (II), X₄ is C, X₅ is N, one of X₂ and X₃ is C, and the other is N.

In some embodiments, in formula (II), X₁ is C or N; one of X₂ and X₃ is C, and the other is N.

In some embodiments, in formula (II), X₄ is C, and X₅ is C or N.

In some embodiments, in formula (II), X₁, X₃, and X₄ are C, and X₂ and X₅ are N.

In some embodiments, in formula (II), X₁, X₂, and X₄ are C, and X₃ and X₅ are N.

In some embodiments, in formula (II), X₃ and X₄ are C, and X₁, X₂, and X₅ are N.

In some embodiments, in formula (II), X₁, X₂, X₄, and X₅ are C, and X₃ is N.

In some embodiments, the compound represented by formula (II) or (III') or the pharmaceutically acceptable salt thereof of the present disclosure is selected from a compound represented by formula (III) or a pharmaceutically acceptable salt thereof: 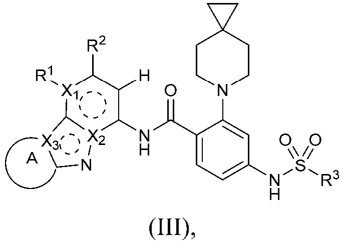 wherein in formula (III):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂, and when one of R¹ and R² is H, the other is not H, cyano, or halogen; and
R³ is selected from C₁-C₆ alkyl or C₂-C₆ alkenyl, wherein the C₁-C₆ alkyl is optionally substituted with one or more halogen or OH.

In some embodiments, R³ is selected from C₁-C₆ alkyl optionally substituted with one or more halogen or OH.

In some embodiments, in formula (III):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, C₁-C₆ alkyl, cyano, C₁-C₆ alkoxy, C(O)R^{d}, halogen, C₁-C₆ haloalkyl, or C₁-C₆ haloalkoxy; R^{d} is selected from OH, C₁-C₆ alkyl, O(C₁-C₄ alkyl), NH₂, NH(C₁-C₄ alkyl), or N(C₁-C₄ alkyl)₂, and when one of R¹ and R² is H, the other is not H, cyano, or halogen; and
R³ is selected from C₁-C₆ alkyl optionally substituted with one or more halogen or OH.

In some embodiments, in formula (III):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, and X₃ are each independently C or N;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂, and when one of R¹ and R² is H, the other is not H, cyano, or halogen; and
R³ is selected from C₁-C₆ alkyl optionally substituted with one or more halogen or OH.

In some embodiments, in formula (II), formula (III'), or formula (III), R² is H, C₁-C₆ alkyl, cyano, C₁-C₆ alkoxy, C(O)R^{d}, halogen, C₁-C₆ haloalkyl, or C₁-C₆ haloalkoxy.

In some embodiments, in formula (II), formula (III'), or formula (III), R¹ is absent, H, C₁-C₆ alkyl, cyano, C₁-C₆ alkoxy, (CH₂)ᵣ-C(O)R^{d}, halogen, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂.

In some embodiments, in formula (II), formula (III'), or formula (III), R¹ is absent, H, OH, C₁-C₆ alkyl, halogen, cyano, C₁-C₆ alkoxy, 5- to 10-membered heteroaryl, or C(O)R^{d}, wherein the C₁-C₆ alkyl is optionally substituted with one or more halogen, and the 5- to 10-membered heteroaryl is optionally substituted with one or more C₁-C₆ alkyl.

In some embodiments, in formula (II), formula (III'), or formula (III), R¹ is absent, H, OH, C₁-C₆ alkyl, halogen, cyano, C₁-C₆ alkoxy, or 5- to 10-membered heteroaryl, wherein the C₁-C₆ alkyl is optionally substituted with one or more halogen, and the 5- to 10-membered heteroaryl is optionally substituted with one or more C₁-C₆ alkyl.

In some embodiments, in formula (II), formula (III'), or formula (III), R¹ is absent, H, OH, C₁-C₆ alkyl, halogen, cyano, C₁-C₆ alkoxy, or C(O)R^{d}, wherein the C₁-C₆ alkyl is optionally substituted with one or more halogen.

In some embodiments, in formula (II), formula (III'), or formula (III), R¹ is absent, H, OH, C₁-C₆ alkyl, halogen, cyano, or C₁-C₆ alkoxy, wherein the C₁-C₆ alkyl is optionally substituted with one or more halogen.

In some embodiments, in formula (II), formula (III'), or formula (III), R¹ is absent, H, OH, methoxy, halogen, CN, CF₃, or

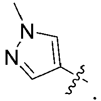

In some embodiments, in formula (II), formula (III'), or formula (III), R¹ is absent, H, OH, methoxy, halogen, CN, or CF₃.

In some embodiments, in formula (II), formula (III'), or formula (III), R¹ is H, OH, methoxy, halogen, or CF₃.

In some embodiments, in formula (II), formula (III'), or formula (III), R¹ is OH.

In some embodiments, in formula (II), formula (III'), or formula (III), R² is H, OH, C₁-C₆ alkyl, cyano, C₁-C₆ alkoxy, or C(O)R^{d}.

In some embodiments, in formula (II), formula (III'), or formula (III), R² is H, C₁-C₆ alkyl, cyano, C₁-C₆ alkoxy, or C(O)R^{d}.

In some embodiments, in formula (II), formula (III'), or formula (III), R² is H, OH, C₁-C₆ alkyl, cyano, or C₁-C₆ alkoxy.

In some embodiments, in formula (II), formula (III'), or formula (III), R² is H, OH, or C₁-C₆ alkyl.

In some embodiments, in formula (II), formula (III'), or formula (III), R² is H, OH, or C₁-C₄ alkyl.

In some embodiments, in formula (II), formula (III'), or formula (III), R² is H.

In some embodiments, in formula (II), formula (III'), or formula (III), R² is OH.

In some embodiments, in formula (II), formula (III'), or formula (III), R³ is selected from C₁-C₄ alkyl or C₂-C₃ alkenyl, wherein the C₁-C₄ alkyl is optionally substituted with one or more halogen or OH.

In some embodiments, in formula (II), formula (III'), or formula (III), R³ is selected from C₁-C₄ alkyl optionally substituted with one or more halogen or OH.

In some embodiments, in formula (II), formula (III'), or formula (III), R³ is selected from C₁-C₄ alkyl or C₂-C₃ alkenyl, wherein the C₁-C₄ alkyl is optionally substituted with one or more OH.

In some embodiments, in formula (II), formula (III'), or formula (III), R³ is selected from C₁-C₄ alkyl optionally substituted with one or more OH.

In some embodiments, in formula (II), formula (III'), or formula (III), R³ is selected from methyl, ethyl, vinyl, or

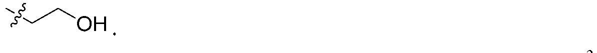

In some embodiments, in formula (II), formula (III'), or formula (III), R³ is selected from methyl, ethyl, or

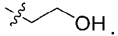

In some embodiments, in formula (II), formula (III'), or formula (III), R³ is selected from methyl or

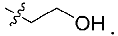

In some embodiments, in formula (II), formula (III'), or formula (III), R³ is selected from

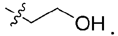

In some embodiments, in formula (II), formula (III'), or formula (III), X₁ is C, and X₂ and X₃ are each independently C or N.

In some embodiments, in formula (II), formula (III'), or formula (III), X₁ is C, one of X₂ and X₃ is C, and the other is N.

In some embodiments, in formula (II), formula (III'), or formula (III), A is selected from a monocyclic or bicyclic 4- to 10-membered carbocyclic ring or 4- to 10-membered heterocyclic ring, wherein the 4- to 10-membered carbocyclic ring or 4- to 10-membered heterocyclic ring is optionally substituted with one or more R^{b}, and each R^{b} is independently selected from OH, F, or C₁-C₄ alkyl.

In some embodiments, in formula (II), formula (III'), or formula (III), A is selected from a monocyclic or bicyclic 4- to 10-membered carbocyclic ring or 4- to 10-membered heterocyclic ring, wherein the 4- to 10-membered carbocyclic ring or 4- to 10-membered heterocyclic ring is optionally substituted with one or more R^{b}, and each R^{b} is independently selected from F or C₁-C₄ alkyl.

In some embodiments, each R^{b} is independently selected from OH, F, methyl, or ethyl.

In some embodiments, each R^{b} is independently selected from F, methyl, or ethyl.

In some embodiments, in formula (II) or formula (III'), the structural unit 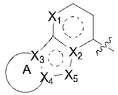 is selected from the following groups optionally substituted with one or more R^{b}:

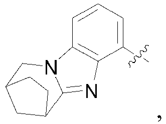

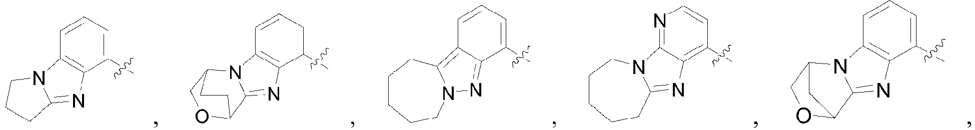

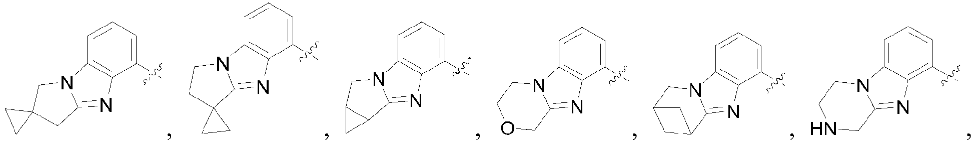

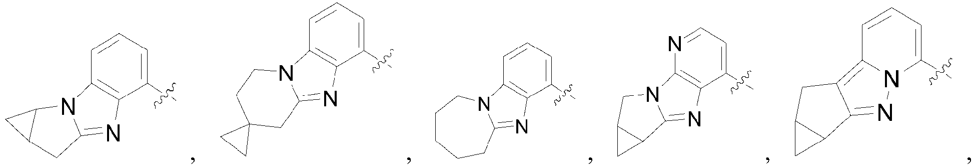

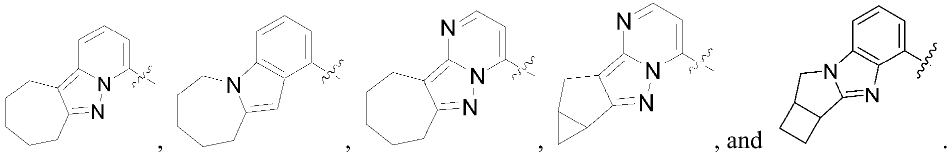

In some embodiments, the structural unit 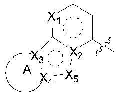 in formula (II) or the structural unit 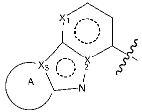 in formula (III') or formula (III) is selected from the following groups optionally substituted with one or more R^{b}:

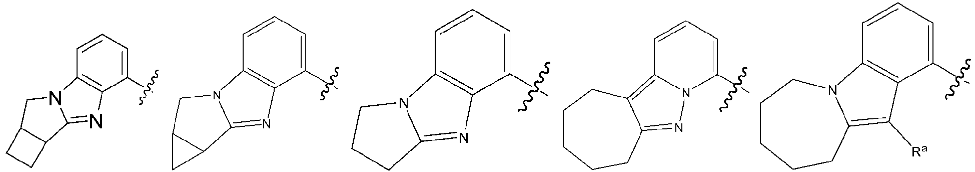

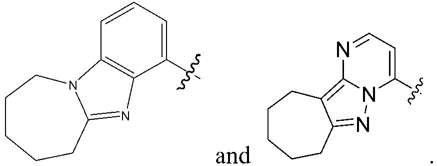

In some embodiments, the structural unit 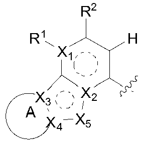 in formula (II) or the structural unit 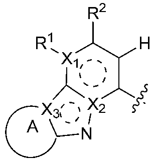 in formula (III') or formula (III) is selected from 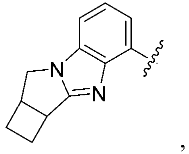 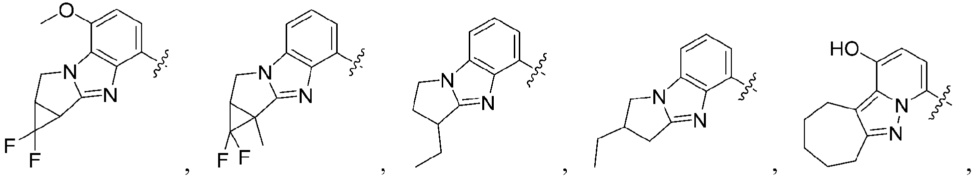 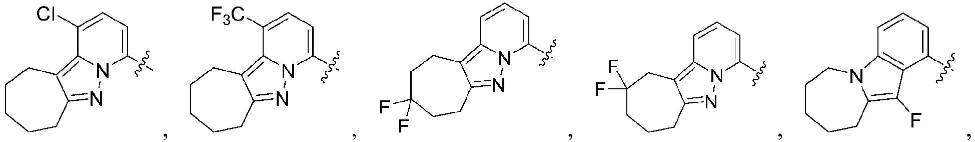 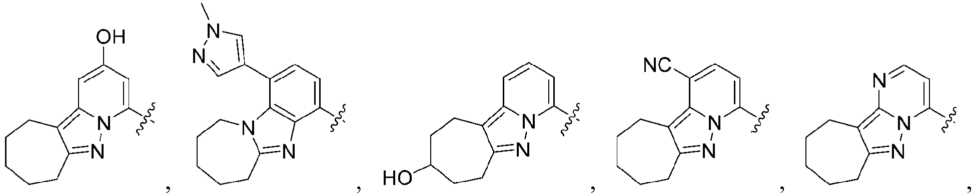 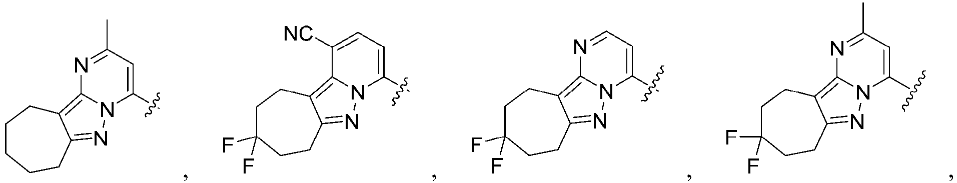 or

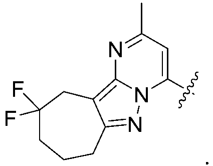

In some embodiments, the structural unit 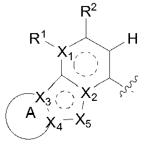 in formula (II) or the structural unit 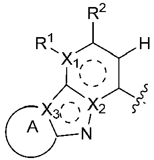 in formula (III') or formula (III) is selected from

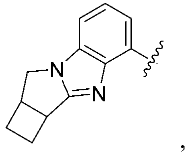

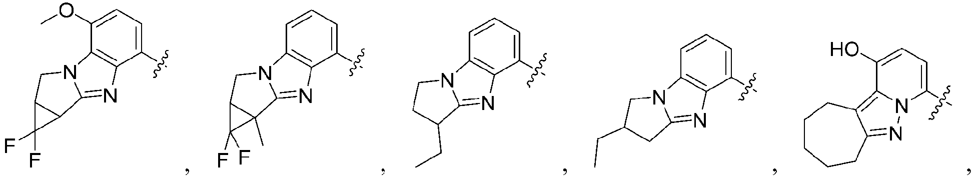

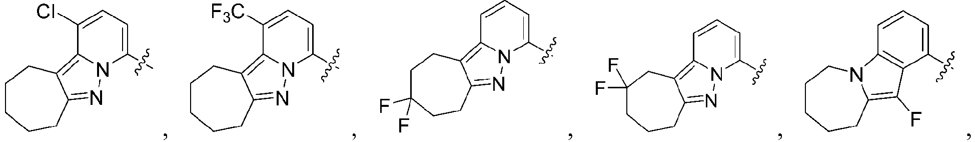

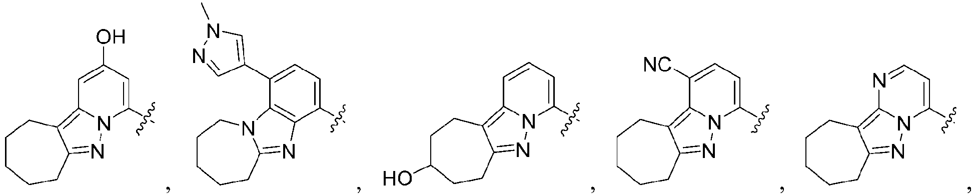

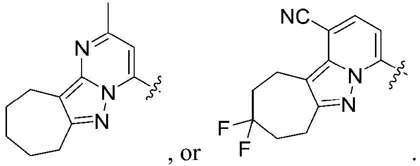

In some embodiments, the structural unit 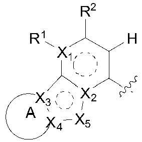 in formula (II) or the structural unit 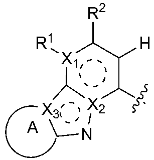 in formula (III') or formula (III) is selected from

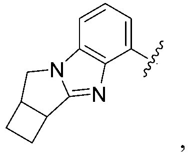

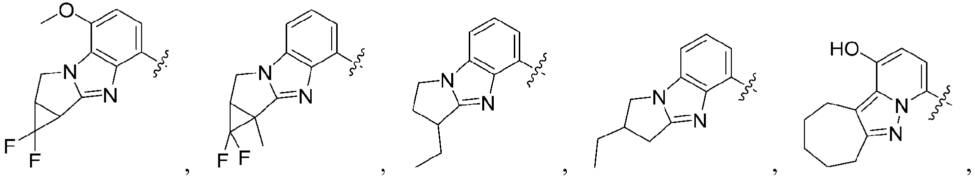

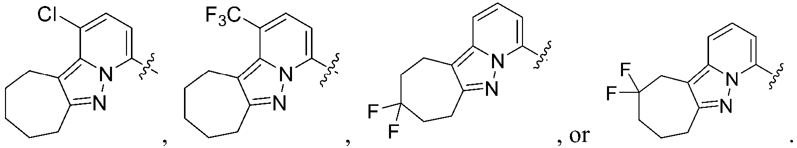

In some embodiments, the compound represented by formula (II), formula (III'), or formula (III) or the pharmaceutically acceptable salt thereof of the present disclosure is selected from the following compounds or pharmaceutically acceptable salts thereof:

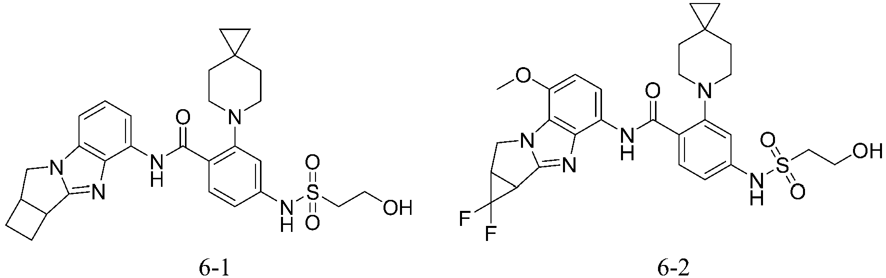

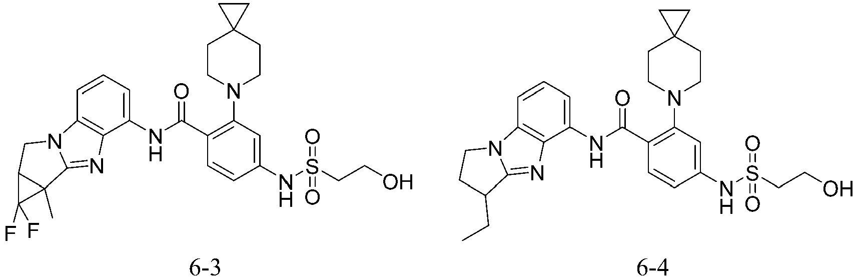

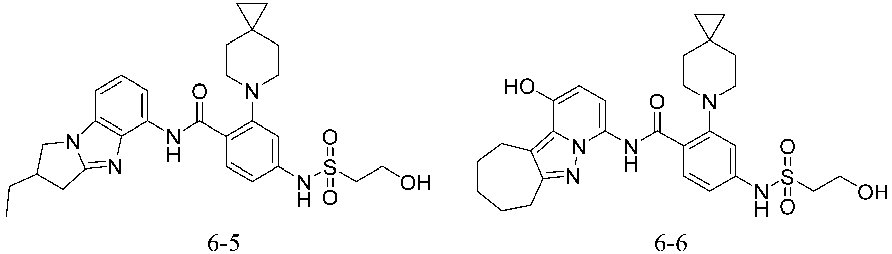

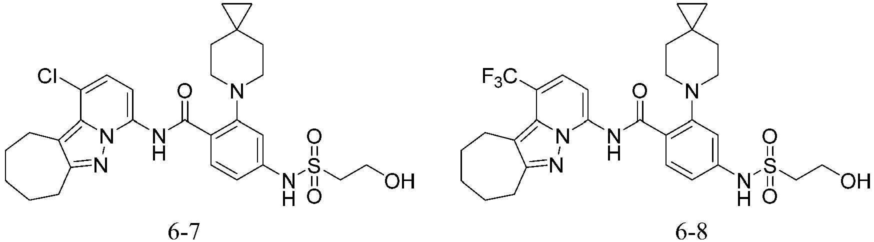

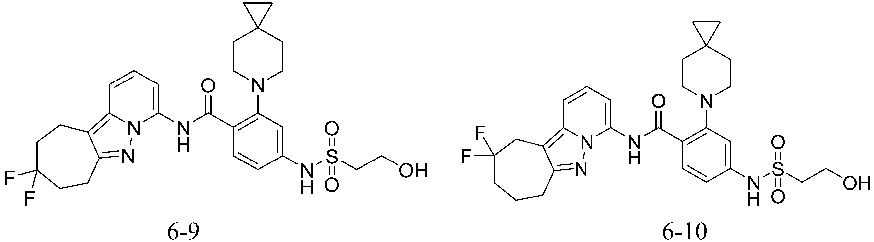

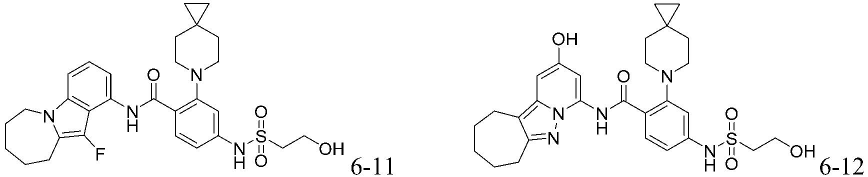

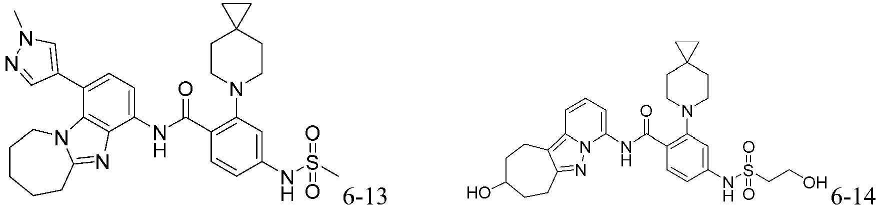

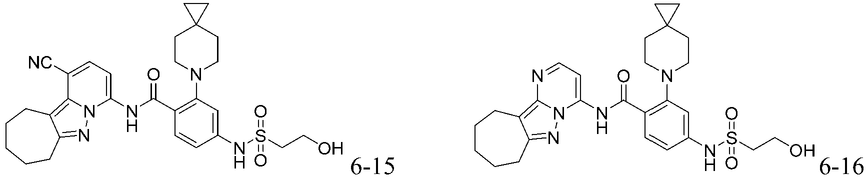

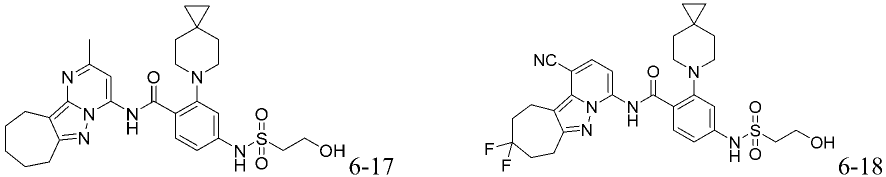

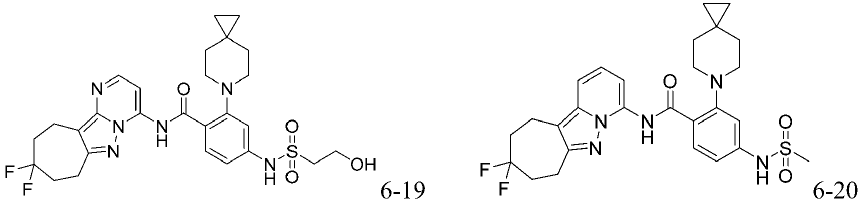

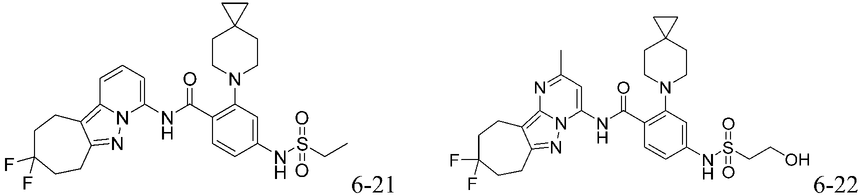

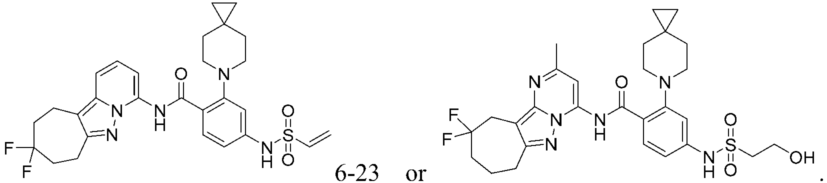

The present disclosure also provides a pharmaceutical composition comprising the compound represented by formula (II), formula (III'), or formula (III) or the pharmaceutically acceptable salt thereof and a pharmaceutically acceptable excipient.

Further, the present disclosure relates to use of the compound represented by formula (II), formula (III'), or formula (III) or the pharmaceutically acceptable salt thereof or the pharmaceutical composition thereof in the preparation of a medicament for preventing or treating a KIF18A-related disease.

Further, the present disclosure relates to use of the compound represented by formula (II), formula (III'), or formula (III) or the pharmaceutically acceptable salt thereof or the pharmaceutical composition thereof in the preparation of a medicament for preventing or treating a tumor.

Further, the present disclosure relates to use of the compound represented by formula (II), formula (III'), or formula (III) or the pharmaceutically acceptable salt thereof or the pharmaceutical composition thereof in preventing or treating a KIF18A-related disease.

Further, the present disclosure relates to use of the compound represented by formula (II), formula (III'), or formula (III) or the pharmaceutically acceptable salt thereof or the pharmaceutical composition thereof in preventing or treating a tumor.

Further, the present disclosure relates to the compound of formula (II), formula (III'), or formula (III) or the pharmaceutically acceptable salt thereof or the pharmaceutical composition thereof for use in preventing or treating a KIF18A-related disease.

Further, the present disclosure relates to the compound of formula (II), formula (III'), or formula (III) or the pharmaceutically acceptable salt thereof or the pharmaceutical composition thereof for use in preventing or treating a tumor.

The present disclosure also relates to a method for treating a KIF18A-related disease, which comprises administering to a patient a therapeutically effective amount of the compound of formula (II), formula (III'), or formula (III) or the pharmaceutically acceptable salt thereof described herein, or the pharmaceutical composition comprising the compound of formula (II), formula (III'), or formula (III) or the pharmaceutically acceptable salt thereof described herein.

The present disclosure also relates to a method for treating a tumor, which comprises administering to a patient a therapeutically effective amount of the compound of formula (II), formula (III'), or formula (III) or the pharmaceutically acceptable salt thereof described herein, or the pharmaceutical composition comprising the compound of formula (II), formula (III'), or formula (III) or the pharmaceutically acceptable salt thereof described herein.

In some embodiments, the KIF18A-related disease is selected from a tumor.

In some embodiments, the tumor is selected from melanoma, breast cancer, ovarian cancer, endometrial carcinoma, cervical cancer, brain cancer, head and neck cancer, thyroid cancer, lung cancer, bronchogenic carcinoma, esophageal cancer, gastric cancer, liver cancer, renal cancer, pancreatic cancer, gallbladder cancer, colon cancer, bladder cancer, prostate cancer, testicular cancer, skin cancer, bone cancer, and hematologic tumors; the lung cancer includes, but is not limited to, small cell lung cancer and non-small cell lung cancer; the hematologic tumors include, but are not limited to, leukemia, lymphoma, and myeloma; the brain cancer includes, but is not limited to, neuroglioma, neuroblastoma, astrocytoma, and meningioma.

### Terms and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be interpreted according to its common meaning in the field. When referring to a trade name, it is intended to refer to its corresponding commercial product or its active ingredient.

Herein, " 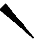 " denotes a linking site.

The term "tautomer" refers to functional isomers resulting from the rapid movement of an atom in a molecule between two positions. The compounds of the present disclosure may exhibit tautomerism. Tautomeric compounds may have two or more interconvertible forms. Tautomers generally exist in equilibrium. Attempts to isolate a single tautomer usually lead to a mixture whose physicochemical properties are consistent with a mixture of compounds. The position of the equilibrium depends on the chemical properties of the molecule. For example, in many aliphatic aldehydes and ketones, such as acetaldehyde, the keto form predominates, and in phenols, the enol form predominates. The present disclosure includes all tautomeric forms of the compounds.

The term "stereoisomer" refers to isomers resulting from different spatial arrangements of atoms in a molecule, including cis-trans isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may be present in the form of a particular geometric isomer or stereoisomer. The form of a particular geometric isomer or stereoisomer may be cis and trans isomers, E and Z geometric isomers, (-)- and (+)-enantiomers, (R)- and (S)-enantiomers, diastereoisomers, (D)-isomers, (L)-isomers, and racemic mixtures or other mixtures thereof, such as an enantiomer or diastereoisomer enriched mixture, and all of the above isomers, as well as mixtures thereof, fall within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The term "substituted" means that any one or more (e.g., 1, 2, 3, 4, 5, or 6) hydrogen atoms on a specific atom are substituted with substituents, as long as the valence of the specific atom is normal and the compound resulting from the substitution is stable. When the substituent is oxo (i.e., =O), it means that two hydrogen atoms are replaced, and oxo will not be present on an aromatic group. The term "optional" or "optionally" means that the subsequently described event or circumstance may or may not occur. The description includes instances where the event or circumstance occurs and instances where the event or circumstance does not occur. For example, ethyl being "optionally" substituted with one or more halogen means that the ethyl may be unsubstituted (CH₂CH₃), monosubstituted (CH₂CH₂F, CH₂CH₂Cl, or the like), polysubstituted (CHFCH₂F, CH₂CHF₂, CHFCH₂Cl, CH₂CHCl₂, or the like), or fully substituted (CF₂CF₃, CF₂CCl₃, CCl₂CCl₃, or the like). Those skilled in the art will appreciate that for any group containing one or more substituents, no substitution or substituting pattern that is spatially impossible and/or cannot be synthesized will be introduced.

When any variable (e.g., R^{a} or R^{b}) occurs one or more times in the constitution or structure of a compound, the variable is independently defined in each case. For example, if a group is substituted with two R^{b}, the definition of each R^{b} is independent.

When the number of a linking group is 0, such as -(CH₂)₀-, it means that the linking group is a bond.

When one of the variables is selected from a chemical bond or is absent, it means that the two groups that it links are linked directly. For example, when L in A-L-Z represents a bond, it means that the structure is actually A-Z.

When the linking direction of a linking group referred to herein is not specified, the linking direction is arbitrary. For example, when L¹ in the structural unit L¹-L²-R^{y} is selected from "-NR⁵SO₂-", L¹ may be linked to L²-R^{y} in the left-to-right direction to form "-NR⁵SO₂-L²-R^{y}" or linked to L²-R^{y} in the right-to-left direction to form "-SO₂NR⁵-L²-R^{y}".

When a chemical bond is cross-linked to two atoms on a ring, it means that the chemical bond can be bonded to any atom on the ring. For example, the structural unit 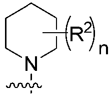 indicates that the chemical bond can be bonded to any position on the ring.

Cₘ-Cₙ used herein means that the portion has an integer number of carbon atoms in the range of m-n. For example, the "C₁-C₁₀" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to a hydrocarbyl group with a general formula of CₙH₂ₙ₊₁. The alkyl may be linear or branched. The term "C₁-C₁₀ alkyl" may be interpreted as a linear or branched saturated hydrocarbyl group having 1, 2, 3, 4, 5, 6, 7, 8, 9, or 10 carbon atoms. Specific examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or the like. The term "C₁-C₆ alkyl" may be understood to represent alkyl having 1 to 6 carbon atoms; specific examples include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, or the like. The term "C₁-C₄ alkyl" may be understood to represent a linear or branched saturated alkyl group having 1 to 4 carbon atoms. The "C₁-C₁₀ alkyl" may include ranges such as "C₁-C₆ alkyl" or "C₁-C₄ alkyl", and the "C₁-C₆ alkyl" may further include "C₁-C₄ alkyl".

The term "alkenyl" refers to a linear or branched unsaturated aliphatic hydrocarbyl group consisting of carbon atoms and hydrogen atoms and having at least one double bond. The term "C₂-C₆ alkenyl" may be understood to represent a linear or branched unsaturated hydrocarbyl group comprising one or more double bonds and having 2, 3, 4, 5, or 6 carbon atoms. It will be appreciated that when the alkenyl comprises more than one double bond, the double bonds may be separated from one another or conjugated. Specific examples of the alkenyl include, but are not limited to, vinyl, allyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, or the like.

The term "cycloalkyl" refers to a fully saturated carbocyclic ring group that exists in the form of a monocyclic ring, a fused ring, a bridged ring, a spiro ring, or the like. Unless otherwise indicated, the carbocyclic ring is generally a 3- to 20-membered ring. The term "C₃-C₁₀ cycloalkyl" refers to a cycloalkyl group having 3, 4, 5, 6, 7, 8, 9, or 10 ring carbon atoms. The term "C₃-C₆ cycloalkyl" refers to a cycloalkyl group having 3, 4, 5, or 6 ring carbon atoms.

The term "C₃-C₁₀ cycloalkenyl" refers to a non-aromatic carbocyclic ring group that is not fully saturated, has at least one carbon-carbon double bond, exists in the form of a monocyclic ring, a fused ring, a bridged ring, a spiro ring, or the like, and has 3-10 ring carbon atoms.

The term "heterocyclyl" refers to a fully saturated or partially saturated (non-aromatic heteroaromatic group on the whole) monocyclic, fused cyclic, spiro cyclic, or bridged cyclic group, and ring atoms of the group include 1-5 (e.g., 1-3 or 1-2) heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), -S(=O)₂-, -S(=O)-, -P(=O)₂-, -P(=O)-, -NH-, -S(=O)(=NH)-, -C(=O)NH-, -NHC(=O)NH-, and the like. The fused ring, spiro ring, or bridged ring may be bicyclic, tricyclic, tetracyclic, or pentacyclic. The term "12- to 20-membered tetracyclic heterocyclyl" refers to a tetracyclic heterocyclyl group having 12, 13, 14, 15, 16, 17, 18, 19, or 20 ring atoms, and 1, 2, 3, 4, or 5 ring atoms of the group are independently selected from the heteroatoms or heteroatom groups described above, wherein the tetracyclic ring exists in the form of a fused ring, a spiro ring, a bridged ring, or a combination thereof. The term "10- to 16-membered tricyclic heterocyclyl" refers to a tricyclic heterocyclyl group having 10, 11, 12, 13, 14, 15, or 16 ring atoms, and 1, 2, 3, 4, or 5 ring atoms of the group are independently selected from the heteroatoms or heteroatom groups described above, wherein the tricyclic ring exists in the form of a fused ring, a spiro ring, a bridged ring, or a combination thereof. The "10- to 16-membered tricyclic heterocyclyl" may include "12-to 15-membered tricyclic heterocyclyl". The term "4- to 14-membered heterocyclyl" refers to a heterocyclyl group having 4, 5, 6, 7, 8, 9, 10, 11, 12, 13, or 14 ring atoms, and 1, 2, 3, 4, or 5 ring atoms of the group are independently selected from the heteroatoms or heteroatom groups described above. The term "4- to 10-membered heterocyclyl" refers to a heterocyclyl group having 4, 5, 6, 7, 8, 9, or 10 ring atoms, and 1, 2, 3, 4, or 5 ring atoms of the group are independently selected from the heteroatoms or heteroatom groups described above. The "4- to 14-membered heterocyclyl" may include "4- to 10-membered heterocyclyl" and "4- to 7-membered heterocyclyl". The "4- to 10-membered heterocyclyl" may include "4- to 7-membered heterocyclyl". The term "4- to 7-membered heterocyclyl" refers to a heterocyclyl group having 4, 5, 6, or 7 ring atoms, and 1, 2, 3, 4, or 5 ring atoms of the group are independently selected from the heteroatoms or heteroatom groups described above. The term "4- to 7-membered monoheterocyclyl" refers to a 4- to 7-membered heterocyclyl group in a monocyclic form. Specific examples of 4-membered heterocyclyl groups include, but are not limited to, azetidinyl or oxetanyl; specific examples of 5-membered heterocyclyl groups include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1H-pyrrolyl; specific examples of 6-membered heterocyclyl groups include, but are not limited to, tetrahydropyranyl, piperidinyl, morpholinyl, dithianyl, thiomorpholinyl, piperazinyl, trithianyl, tetrahydropyridinyl, or 4H-[1,3,4]thiadiazinyl; specific examples of 7-membered heterocyclyl groups include, but are not limited to, diazepanyl. The heterocyclyl may also be a bicyclic group, wherein specific examples of 5,5-membered bicyclic groups include, but are not limited to, hexahydrocyclopenta[c]pyrrol-2(1H)-yl; specific examples of 5,6-membered bicyclic groups include, but are not limited to, hexahydropyrrolo[1,2-a]pyrazin-2(1H)-yl, 5,6,7,8-tetrahydro-[1,2,4]triazolo[4,3-a]pyrazinyl, or 5,6,7,8-tetrahydroimidazo[1,5-a]pyrazinyl. Optionally, the heterocyclyl may be a benzo-fused ring group of the 4- to 7-membered heterocyclyl described above. Specific examples include, but are not limited to, dihydroisoquinolyl and the like. The "4- to 10-membered heterocyclyl" may include the ranges of "5- to 10-membered heterocyclyl", "4- to 7-membered heterocyclyl", "5- to 6-membered heterocyclyl", "6- to 8-membered heterocyclyl", "4- to 10-membered heterocycloalkyl", "5- to 10-membered heterocycloalkyl", "4- to 7-membered heterocycloalkyl", "5- to 6-membered heterocycloalkyl", "6- to 8-membered heterocycloalkyl", and the like, and the "4- to 7-membered heterocyclyl" may further include the ranges of "4- to 6-membered heterocyclyl", "5- to 6-membered heterocyclyl", "4- to 7-membered heterocycloalkyl", "4- to 6-membered heterocycloalkyl", "5- to 6-membered heterocycloalkyl", and the like. Although some bicyclic heterocyclyl groups herein comprise, in part, one benzene ring or one heteroaromatic ring, the heterocyclyl is still non-aromatic on the whole.

The term "heterocycloalkyl" refers to a fully saturated cyclic group that exists in the form of a monocyclic ring, a fused ring, a bridged ring, a spiro ring, or the like, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), -S(=O)₂-, -S(=O)-, -NH-, -S(=O)(=NH)-, -C(=O)NH-, -NHC(=O)NH-, or the like. The term "4- to 10-membered heterocycloalkyl" refers to a heterocycloalkyl group having 4, 5, 6, 7, 8, 9, or 10 ring atoms, and 1-5 ring atoms of the group are independently selected from the heteroatoms or heteroatom groups described above. "4- to 10-membered heterocycloalkyl" includes "4- to 7-membered heterocycloalkyl", wherein specific examples of 4-membered heterocycloalkyl include, but are not limited to, azetidinyl, oxetanyl, or thietanyl; specific examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, or tetrahydropyrazolyl; specific examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, or 1,4-dithianyl; specific examples of 7-membered heterocycloalkyl include, but are not limited to, azepanyl, oxepanyl, or thiepanyl.

The term "heteroaryl" refers to an aromatic cyclic group having an aromatic monocyclic or fused polycyclic system, which contains at least one ring atom selected from N, O, and S, with the remaining ring atoms being C, typically has 5, 6, 7, 8, 9, 10, 11, 12, 13, or 14 ring atoms, and comprises 1-8, preferably 1-5, heteroatoms independently selected from N, O, and S. The term "11-to 14-membered tricyclic heteroaryl" refers to an aromatic cyclic group having an aromatic fused tricyclic system with 11, 12, 13, or 14 ring atoms, wherein at least one ring atom is selected from N, O, and S, and the remaining ring atoms are C. The term "5- to 6-membered heteroaryl" refers to an aromatic ring system having 5 or 6 ring atoms, which comprises 1-3, e.g., 1-2, heteroatoms independently selected from N, O, and S. It includes, but is not limited to, thienyl, furanyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, triazinyl, or the like.

The term "halo" or "halogen" refers to fluorine, chlorine, bromine, or iodine.

The term "hydroxy" refers to the -OH group.

The term "cyano" refers to the -CN group.

The term "sulfhydryl" refers to the -SH group.

The term "amino" refers to the -NH₂ group.

The term "nitro" refers to the -NO₂ group.

The term "therapeutically effective amount" means an amount of a compound of the present disclosure for (i) treating a specific disease, condition, or disorder; (ii) palliating, ameliorating, or eliminating one or more symptoms of a specific disease, condition, or disorder; or (iii) delaying the onset of the one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the content of the present disclosure.

The term "prevent", "preventing", or "prevention" means administering the compound or formulation described herein to prevent a disease or one or more symptoms associated with the disease, and includes: preventing the occurrence of the disease or disease state in an individual (e.g., a mammal), particularly when such an individual (e.g., a mammal) is predisposed to the disease state but has not yet been diagnosed with it.

The term "individual" includes mammals and non-mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, and guinea pigs. Examples of non-human mammals include, but are not limited to, birds, fish, and the like. In one embodiment associated with the methods and compositions provided herein, the mammal is a human. The terms "patient" and "individual" are used interchangeably.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, and water.

The word "comprise" and variations thereof such as "comprises" or "comprising" may be interpreted in an open and non-exclusive sense, i.e., "including but not limited to".

The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl. Certain isotopically labeled compounds of the present disclosure (e.g., those labeled with ³H and ¹⁴C) can be used to analyze compounds and/or substrate tissue distribution. Tritiation (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation, such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present disclosure include, but are not limited to, oral, rectal, topical, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional methods of mixing, dissolving, granulating, emulsifying, freeze-drying, and the like.

In some embodiments, the pharmaceutical composition is in an oral form. For oral administration, the pharmaceutical composition can be formulated by mixing the active compound with pharmaceutically acceptable excipients well known in the art. These excipients enable the compounds of the present disclosure to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compound with a solid excipient, optionally grinding the resulting mixture, adding additional suitable excipients if desired, and processing the mixture into granules to get the core parts of tablets or dragees. Suitable excipients include, but are not limited to, binders, diluents, disintegrants, lubricants, glidants, flavoring agents, and the like.

The pharmaceutical composition may also be suitable for parenteral administration, such as a sterile solution, suspension, or lyophilized product in a suitable unit dosage form.

In all of the administration methods for the compound of general formula (II), formula (III'), or formula (III) described herein, the daily dose administered is 0.01 mg/kg body weight to 200 mg/kg body weight, preferably 0.05 mg/kg body weight to 50 mg/kg body weight, and more preferably 0.1 mg/kg body weight to 30 mg/kg body weight, given in a single dose or divided doses.

The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

The following abbreviations are used in the present disclosure:

| Abbreviation | Chemical name | Abbreviation | Chemical name |
|---|---|---|---|
| BPin | 4,4,5,5-Tetramethyl-1,3,2-dioxabora n-2-yl | B₂Pin₂ | 4,4,5,5-Tetramethyl-2-(4,4,5, 5-tetramethyl-1,3,2-dioxabor an-2-yl)-1,3,2-dioxaborane |
| DCM | Dichloromethane | DMF | N,N-Dimethylformamide |
| Pd(dppf)Cl₂ | [1,1'-Bis(diphenylphosphino)ferroce ne]dichloropalladium(II) | HCl | Hydrochloric acid |
| DMAP | 4-Dimethylaminopyridine | TFA | Trifluoroacetic acid |
| THF | Tetrahydrofuran | MeCN | Acetonitrile |
| TEA | Triethylamine | NBS | N-Bromosuccinimide |
| M | mol/L | Boc | tert-Butyloxycarbonyl |
| DIEA | N,N-Diisopropylethylamine | MeOH | Methanol |
| DMSO | Dimethyl sulfoxide | TLC | Thin layer chromatography |
| AcOK | Potassium acetate | DIPEA | *N,N-*Diisopropylethylamine |
| PE | Petroleum ether | EA | Ethyl acetate |
| ACN | Acetonitrile | Pd₂(Ph₃P)₄ | Tetrakis(triphenylphosphine) palladium(0) |
| AIBN | Azobisisobutyronitrile | Pd₂dba₃ | Tris(dibenzylideneacetone)di palladium(0) |
| Xantphos | 4,5-Bis(diphenylphosphino)-9,9-di methylxanthene | Cs₂CO₃ | Cesium carbonate |
| XPhos-Pd-G2 | Chloro(2-dicyclohexylphosphino-2', 4',6'-triisopropyl-1,1'-biphenyl)[2-(2 '-amino-1,1'-biphenyl)]palladium(II) | XPhos-Pd-G3 | Methanesulfonato(2-dicyclo hexylphosphino-2',4',6'-triiso propyl-1,1'-biphenyl)(2'-ami no-1,1'-biphenyl-2-yl)palladi um(II) |
| K₃PO₄·3H₂O | Potassium phosphate trihydrate | CaCO₃ | Calcium carbonate |
| THF | Tetrahydrofuran | NaH | Sodium hydride |
| HCHO | Formaldehyde | AcOK | Potassium acetate |
| K₂CO₃ | Potassium carbonate | X-Phos | Dicyclohexyl(2,4,6-triisopro pyl-[1,1-biphenyl]-2-yl)phos phine |
| HATU | O-(7-Azabenzotriazol-1-yl)-N,N,N', N'-tetramethyluronium hexafluorophosphate | TBS | tert-Butyldimethylsilyl |
| TCFH | N,N,N',N'-Tetramethylchloroforma midinium hexafluorophosphate | NMI | N-Methylimidazole |
| PyBOP | 1H-Benzotriazole-1-yloxytripyrrolid inyl hexafluorophosphate | DBU | 1,8-Diazabicyclo[5.4.0]unde c-7-ene |
| RuPhos | 2-Dicyclohexylphosphino-2',6'-diiso propoxy-1,1'-biphenyl | | |

### DETAILED DESCRIPTION

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios.

Unless otherwise stated, % refers to weight percentage wt%.

Compounds are named either manually or by ChemDraw^{®} software, and suppliers' catalog names are used for commercially available compounds.

The structures of the compounds are determined by nuclear magnetic resonance (NMR) and/or mass spectrometry (MS). NMR shifts are given in 10⁻⁶ (ppm). The solvents for NMR determination are deuterated dimethyl sulfoxide, deuterated chloroform, deuterated methanol, and the like, and the internal standard is tetramethylsilane (TMS). The "IC₅₀" refers to the half maximal inhibitory concentration, which is the concentration at which half of the maximal inhibitory effect is achieved. The eluent or mobile phase may be a mixed eluent or mobile phase composed of two or more solvents, and the ratio of the mixed eluent or mobile phase is the volume ratio of the solvents.

### Example 1: Synthesis of Compound 6-1

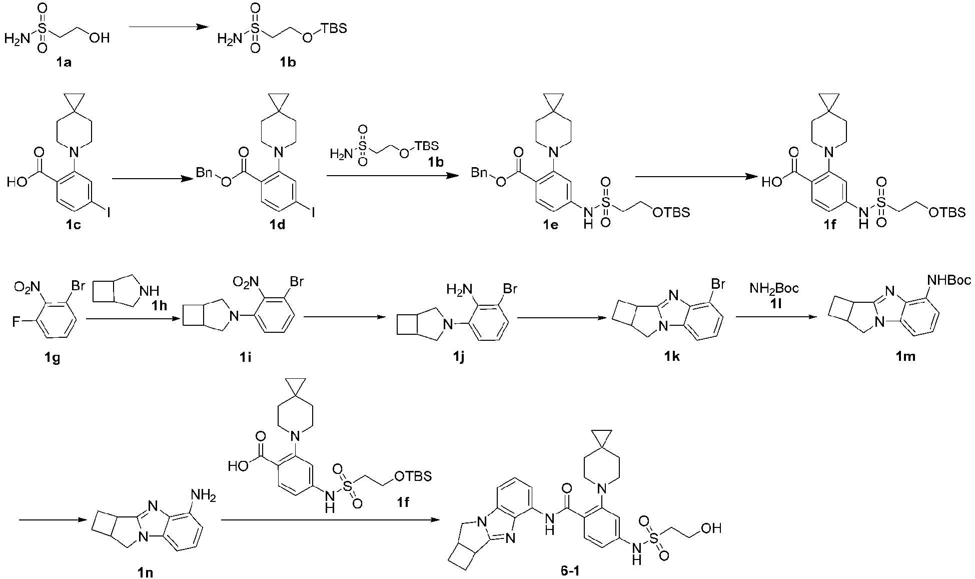

### Step 1: synthesis of compound 1b

Compound **1a** (2.00 g, 15.9 mmol) and imidazole (1.31 g, 19.1 mmol) were added to DMF (20 mL), followed by the addition of tert-butyldimethylsilyl chloride (2.89 g, 19.1 mmol) at 0 °C. The reaction mixture was allowed to react sufficiently at room temperature. A saturated aqueous ammonium chloride solution (50 mL) was added, and ethyl acetate (30 mL × 3) was added for extraction. The combined organic phases were washed with saturated brine, dried over anhydrous magnesium sulfate, and filtered, and the filtrate was concentrated and then slurried with petroleum ether to give compound **1b** (3.20 g).

### Step 2: synthesis of compound 1d

Compound **1c** (1.00 g, 2.80 mmol) and potassium carbonate (464 mg, 3.36 mmol) were added to DMF (20 mL), followed by the addition of benzyl bromide (526 mg, 3.08 mmol) at 0 °C. The reaction mixture was allowed to react sufficiently at room temperature. Water (50 mL) was added to the system, and ethyl acetate (30 mL × 3) was added for extraction. The organic phases were combined, washed with saturated brine, dried over magnesium sulfate, and filtered, and the filtrate was concentrated to dryness by rotary evaporation. The residue was purified by flash column chromatography (petroleum ether:ethyl acetate = 8:1) to give compound **1d** (1.0 g).

MS m/z (ESI): 448.2 [M+H]⁺.

### Step 3: synthesis of compound 1e

Compound **1d** (500 mg, 1.12 mmol), compound **1b** (400 mg, 1.68 mmol), potassium phosphate (635 mg, 3 mmol), and (1R,2R)-N,N'-dimethyl-1,2-cyclohexanediamine (160 mg, 1.12 mmol) were added to DMF (5 mL), followed by the addition of cuprous iodide (381 mg, 2 mmol). The reaction mixture was allowed to react sufficiently at 95 °C in a nitrogen atmosphere. After the completion of the reaction, the reaction mixture was cooled and directly purified by flash reversed-phase column chromatography (acetonitrile:water, 10-85%) to give compound **1e** (680 mg).

MS m/z (ESI): 559.3 [M+H]⁺.

### Step 4: synthesis of compound 1f

Compound **1e** (0.60 g, 1.07 mmol) was added to methanol (6 mL) and ethyl acetate (4 mL), followed by the addition of palladium on carbon (0.20 g, 10% loading). The reaction mixture was allowed to react sufficiently at 25 °C in a hydrogen atmosphere. The reaction mixture was filtered, and the filtrate was concentrated to dryness by rotary evaporation. The residue was slurried with petroleum ether (5 mL) to give compound **1f** (314 mg).

MS m/z (ESI): 469.3 [M+H]⁺.

### Step 5: synthesis of compound 1i

Compound **1g** (617 mg, 2.81 mmol), compound **1h** hydrochloride (250 mg, 1.87 mmol), and cesium carbonate (1.83 g, 5.61 mmol) were added to acetonitrile (4 mL), and the mixture was allowed to react sufficiently at 80 °C. Water (20 mL) was added to the system, and ethyl acetate (20 mL × 3) was added for extraction. The organic phases were combined, washed with saturated brine, dried over anhydrous sodium sulfate, and filtered, and the filtrate was concentrated to dryness by rotary evaporation to give a crude product of **1i** (550 mg).

MS m/z (ESI): 296.9/298.9 [M+H]⁺.

### Step 6: synthesis of compound 1j

Compound **1i** (550 mg, 1.85 mmol) and ammonium chloride (953 mg, 18.51 mmol) were added to water (5 mL) and ethanol (25 mL), followed by the addition of iron powder (1.03 g, 18.51 mmol). The mixture was allowed to react sufficiently at 80 °C. The reaction mixture was filtered, and the filter cake was washed with ethyl acetate. The filtrate was concentrated, and the residue was purified by column chromatography (petroleum ether:ethyl acetate = 100:0 to 80:20) to give compound **1j** (460 mg).

MS m/z (ESI): 267.0/269.0 [M+H]⁺.

### Step 7: synthesis of compound 1k

A solution of compound **1j** (450 mg, 1.68 mmol) in water (2 mL) and acetonitrile (10 mL) was heated to 80 °C, and iodine (2.13 g, 8.40 mmol) was added to the mixture. The mixture was allowed to react sufficiently at 80 °C. The reaction mixture was poured into a saturated aqueous sodium bicarbonate solution (50 mL), and ethyl acetate (50 mL × 3) was added for extraction. The organic phase was washed with a saturated sodium thiosulfate solution (50 mL × 3), dried over anhydrous sodium sulfate, and filtered, and the filtrate was concentrated. The residue was purified by column chromatography (petroleum ether:ethyl acetate = 75:25) to give compound **1k** (340 mg).

MS m/z (ESI): 263.0/265.0 [M+H]⁺.

### Step 8: synthesis of compound 1m

Pd₂dba₃ (139 mg, 152 µmol), cesium carbonate (619 mg, 1.90 mmol), and Xantphos (88 mg, 152 µmol) were added to a solution of compound **1l** (356 mg, 3.04 mmol) and compound **1k** (200 mg, 761 µmol) in dioxane (4 mL) in a nitrogen atmosphere, and the mixture was allowed to react sufficiently at 100 °C. The reaction mixture was filtered, and the filtrate was concentrated to dryness by rotary evaporation. The residue was purified by column chromatography to give compound **1m** (210 mg). MS m/z (ESI): 300.1 [M+H]⁺.

### Step 9: synthesis of compound 1n

Trifluoroacetic acid (1 mL) was added dropwise to a solution of compound **1m** (200 mg, 668 µmol) in dichloromethane (5 mL), and the mixture was allowed to react sufficiently at room temperature. The reaction mixture was concentrated, and the residue was dissolved in water, followed by extraction with ethyl acetate (20 mL × 3). The organic phase was discarded. The aqueous phase was adjusted to pH = 10 and extracted three times with ethyl acetate (20 mL × 3). The organic phases were combined, dried over anhydrous sodium sulfate, and filtered, and the filtrate was concentrated to give compound **1n** (85 mg).

MS m/z (ESI): 200.1 [M+H]⁺.

### Step 10: synthesis of compound 6-1

Compound **1f** (47 mg, 100 µmol), N-methylimidazole (NMI, 44 mg, 533 µmol), and N,N,N',N'-tetramethylchloroformamidinium hexafluorophosphate (TCFH, 45 mg, 160 µmol) were added to acetonitrile (2 mL), and the mixture was stirred at room temperature for 15 min. A solution of compound **1n** (20 mg, 100 µmol) in acetonitrile (0.5 mL) was added dropwise to the reaction mixture. The mixture was heated to 60 °C and allowed to react sufficiently. The reaction mixture was cooled to room temperature, and pyridine hydrofluoride (29 mg, 300 µmol) was added. The reaction mixture was allowed to react sufficiently at room temperature. The reaction mixture was directly purified by flash reversed-phase column chromatography (acetonitrile:water = 10:90 to 90:10) to give compound **6-1** (33 mg).

¹H-NMR (400 MHz, DMSO-*d*₆) δ 12.37 (s, 1H), 10.06 (s, 1H), 8.34 (dd, *J* = 7.0, 2.0 Hz, 1H), 8.02 (d, *J =* 8.6 Hz, 1H), 7.25 (d, *J* = 2.1 Hz, 1H), 7.22 - 7.13 (m, 2H), 7.07 (dd, *J* = 8.6, 2.1 Hz, 1H), 4.91 (s, 1H), 4.17 (dd, *J =* 10.6, 6.2 Hz, 1H), 4.06 (d, *J* = 10.7 Hz, 1H), 3.80 - 3.69 (m, 4H), 3.34 (t, *J =* 6.5 Hz, 2H), 3.08 - 2.91 (m, 4H), 2.65 - 2.57 (m, 1H), 2.47 - 2.39 (m, 1H), 2.11 - 1.98 (m, 2H), 1.94 - 1.49 (m, 4H), 0.34 - 0.21 (m, 4H).

MS m/z (ESI): 536.2 [M+H]⁺.

### Example 2: Synthesis of Compound 6-2

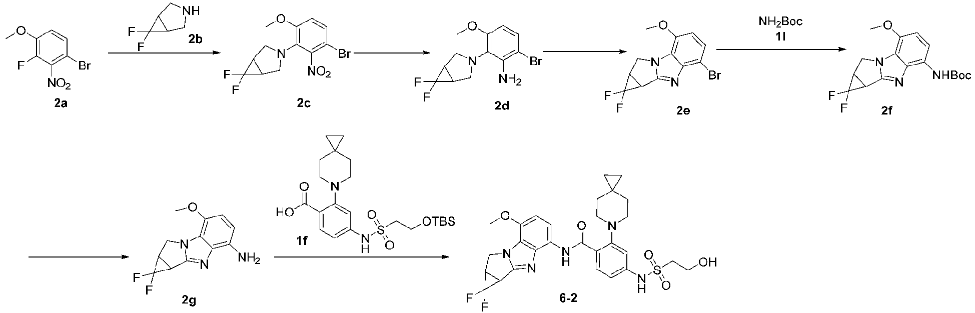

### Step 1: synthesis of compound 2c

Compound **2c** (352 mg) was obtained through synthesis and purification using a method identical to that described in step 5 of Example 1, with compounds **2a** (0.3 g, 1.2 mmol) and **2b** hydrochloride (560 mg, 3.6 mmol) as starting materials.

### Step 2: synthesis of compound 2d

Compound **2d** (205 mg) was obtained through synthesis and purification using a method identical to that described in step 6 of Example 1, with compound **2c** (311 mg, 0.89 mmol) as starting material.

### Step 3: synthesis of compound 2e

Compound **2e** (151 mg) was obtained through synthesis and purification using a method identical to that described in step 7 of Example 1, with compound **2d** (202 mg, 0.63 mmol) as starting material.

### Step 4: synthesis of compound 2f

Compound **2f** (30 mg) was obtained through synthesis and purification using a method identical to that described in step 8 of Example 1, with compound **2e** (80 mg, 0.25 mmol) as starting material.

### Step 5: synthesis of compound 2g

Compound **2g** (17 mg) was obtained through synthesis and purification using a method identical to that described in step 9 of Example 1, with compound **2f** (30 mg, 0.085 mmol) as starting material. MS m/z (ESI): 252.1 [M+H]⁺.

### Step 6: synthesis of compound 6-2

Compound **6-2** (3.1 mg) was obtained through synthesis and purification using a method identical to that described in step 10 of Example 1, with compound **2g** (10 mg, 0.04 mmol) and compound **1f** (17 mg, 0.04 mmol) as starting materials.

¹H-NMR (400 MHz, DMSO-*d*₆) δ 12.23 (s, 1H), 10.08 (s, 1H), 8.22 (d, *J* = 8.6 Hz, 1H), 7.99 (d, *J*= 8.6 Hz, 1H), 7.23 (s, 1H), 7.06 (d, *J* = 8.6 Hz, 1H), 6.76 (d, *J* = 8.8 Hz, 1H), 4.95 (s, 1H), 4.67 (dd, *J =* 12.1, 6.5 Hz, 1H), 4.49 (d, *J* = 12.1 Hz, 1H), 3.89 (s, 3H), 3.76 (t, *J* = 6.5 Hz, 2H), 3.73 - 3.68 (m, 1H), 3.54 - 3.46 (m, 1H), 3.35 - 3.34 (m, 2H), 3.03 - 2.88 (m, 4H), 1.88 - 1.62 (m, 4H), 0.30 (s, 4H).

MS m/z (ESI): 588.2 [M+H]⁺.

### Example 3: Synthesis of Compound 6-3

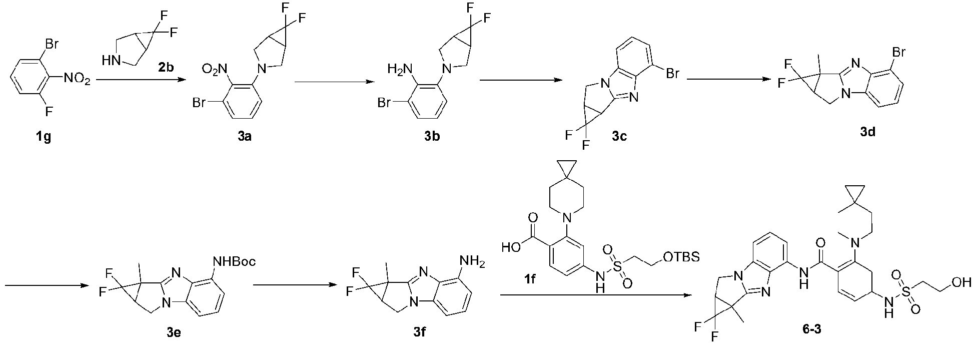

### Step 1: synthesis of compound 3a

Compound **3a** (760 mg) was obtained through synthesis and purification using a method identical to that described in step 5 of Example 1, with compounds **1g** (1.56 g, 7.07 mmol) and **2b** hydrochloride (1.0 g, 6.43 mmol) as starting materials.

### Step 2: synthesis of compound 3b

Compound **3b** (430 mg) was obtained through synthesis and purification using a method identical to that described in step 6 of Example 1, with compound **3a** (710 mg, 2.22 mmol) as starting material.

### Step 3: synthesis of compound 3c

Compound **3c** (140 mg) was obtained through synthesis and purification using a method identical to that described in step 7 of Example 1, with compound **3b** (290 mg, 1.0 mmol) as starting material.

### Step 4: synthesis of compound 3d

Lithium bis(trimethylsilyl)amide (1 M, 2.10 mmol, 2.10 mL) was slowly added dropwise to a solution of compound **3c** (120 mg, 421 µmol) in tetrahydrofuran (4 mL) at -70 °C in a nitrogen atmosphere, and the mixture was stirred at -78 °C for 10 min. A solution of iodomethane (179 mg, 1.26 mmol) in tetrahydrofuran (1 mL) was added dropwise to the reaction mixture, and the mixture was allowed to react sufficiently at -78 °C. A saturated ammonium chloride solution (10 mL) was added to quench the reaction, and ethyl acetate (50 mL × 3) was added for extraction. The organic phases were combined, dried over anhydrous sodium sulfate, and purified by column chromatography to give compound **3d** (50 mg).

### Step 5: synthesis of compound 3e

Compound **3e** (39 mg) was obtained through synthesis and purification using a method identical to that described in step 8 of Example 1, with compound **3d** (40 mg, 0.13 mmol) as starting material.

### Step 6: synthesis of compound 3f

Compound **3f** (23 mg) was obtained through synthesis and purification using a method identical to that described in step 9 of Example 1, with compound **3e** (36 mg, 0.11 mmol) as starting material. MS m/z (ESI): 236.1 [M+H]⁺.

### Step 7: synthesis of compound 6-3

Compound **6-3** (15 mg) was obtained through synthesis and purification using a method identical to that described in step 10 of Example 1, with compound **3f** (21 mg, 0.1 mmol) and compound **1f** (47 mg, 0.1 mmol) as starting materials.

¹H-NMR (400 MHz, DMSO-*d*₆) δ 12.38 (s, 1H), 8.39 (dd, *J=* 7.0, 2.1 Hz, 1H), 8.05 - 8.00 (m, 1H), 7.26 (s, 1H), 7.23 - 7.15 (m, 2H), 7.07 (d, *J* = 8.7 Hz, 1H), 4.92 (s, 1H), 4.55 (dd, *J* = 11.8, 6.4 Hz, 1H), 4.39 (d, *J* = 11.8 Hz, 1H), 3.76 (t, *J* = 6.5 Hz, 2H), 3.38 - 3.33 (m, 2H), 3.24 (dd, *J* = 12.7, 6.2 Hz, 1H), 3.07 - 2.92 (m, 4H), 1.96 - 1.62 (m, 7H), 0.37 - 0.25 (m, 4H).

MS m/z (ESI): 572.2 [M+H]⁺.

### Example 4 and Example 5: Synthesis of Compound 6-4 and Compound 6-5

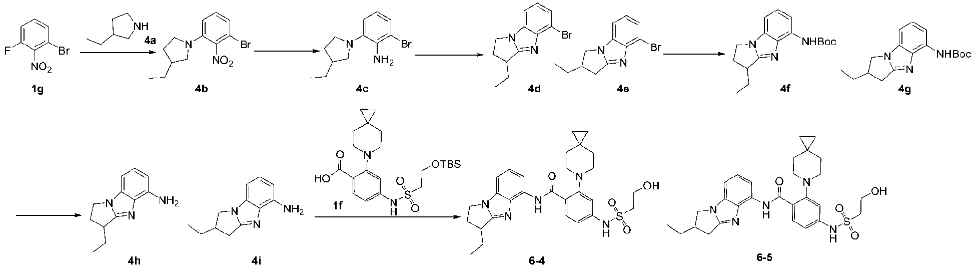

### Step 1: synthesis of compound 4b

Compound **4b** (342 mg) was obtained through synthesis and purification using a method identical to that described in step 5 of Example 1, with compounds **1g** (0.23 g, 1.05 mmol) and **4a** hydrochloride (170 mg, 1.25 mmol) as starting materials.

### Step 2: synthesis of compound 4c

Compound **4c** (249 mg) was obtained through synthesis and purification using a method identical to that described in step 6 of Example 1, with compound **4b** (300 mg, 1.0 mmol) as starting material.

### Step 3: synthesis of mixture 4d/4e

Mixture **4d/4e** (191 mg) was obtained through synthesis and purification using a method identical to that described in step 7 of Example 1, with compound **4c** (245 mg, 0.91 mmol) as starting material.

### Step 4: synthesis of mixture 4f/4g

Mixture **4f/4g** (269 mg) was obtained through synthesis and purification using a method identical to that described in step 8 of Example 1, with mixture **4d/4e** (160 mg, 0.6 mmol) as starting material.

### Step 5: synthesis of mixture 4h/4i

Mixture **4h/4i** (67 mg) was obtained through synthesis and purification using a method identical to that described in step 9 of Example 1, with mixture **4f/4g** (250 mg, 0.83 mmol) as starting material. MS m/z (ESI): 202.1 [M+H]⁺.

### Step 6: synthesis of compound 6-4/6-5

Mixture **6-4/6-5** was obtained through synthesis and purification using a method identical to that described in step 10 of Example 1, with mixture **4h/4i** (50 mg, 0.25 mmol) and compound **1f** (141 mg, 0.3 mmol) as starting materials, and it was then purified by preparative chromatography (acetonitrile:water, 20-85%, 0.1% ammonia water) to give:
compound **6-4** (4.3 mg)
¹H-NMR (400 MHz, DMSO-*d*₆) δ 12.25 (s, 1H), 8.35 (dd, *J* = 7.3, 1.7 Hz, 1H), 8.01 (d, *J* = 8.6 Hz, 1H), 7.24 (d, *J* = 2.2 Hz, 1H), 7.19 - 7.10 (m, 2H), 7.05 (dd, *J* = 8.6, 2.1 Hz, 1H), 4.23 - 4.16 (m, 1H), 4.11 - 4.03 (m, 1H), 3.76 (t, *J* = 6.5 Hz, 2H), 3.36 - 3.33 (m, 2H), 3.24 - 3.18 (m, 1H), 3.07 - 2.93 (m, 4H), 2.89 - 2.80 (m, 1H), 2.36 - 2.31 (m, 1H), 2.08 - 2.00 (m, 1H), 1.93 - 1.59 (m, 5H), 1.01 (t, *J =* 7.4 Hz, 3H), 0.30 (s, 4H).

MS m/z (ESI): 538.2 [M+H]⁺.

### compound 6-5 (2.8 mg)

¹H-NMR (400 MHz, DMSO-*d*₆) δ 12.32 (s, 1H), 8.29 (dd, *J =* 6.9, 2.1 Hz, 1H), 8.00 (d, *J =* 8.5 Hz, 1H), 7.24 (d, *J* = 2.2 Hz, 1H), 7.17 - 7.09 (m, 2H), 7.06 (dd, *J* = 8.6, 2.1 Hz, 1H), 4.33 (dd, *J* = 10.3, 7.8 Hz, 1H), 3.81 - 3.72 (m, 3H), 3.35 (t, *J* = 6.5 Hz, 2H), 3.16 - 3.09 (m, 1H), 3.08 - 3.02 (m, 1H), 3.02 - 2.93 (m, 4H), 2.69 - 2.62 (m, 1H), 1.77 (s, 4H), 1.69 - 1.62 (m, 2H), 0.97 (t, *J* = 7.4 Hz, 3H), 0.29 (s, 4H).

MS m/z (ESI): 538.2 [M+H]⁺.

### Example 6: Synthesis of Compound 6-6

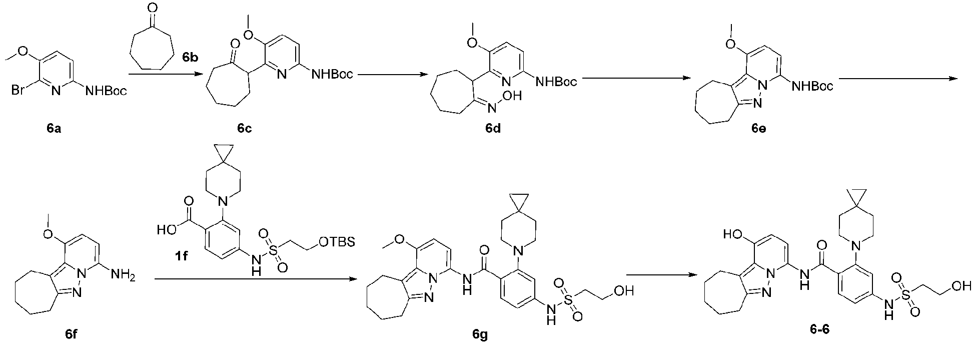

### Step 1: synthesis of compound 6c

Sodium tert-butoxide (950 mg, 9.90 mmol) was added to a solution of compound **6a** (1.00 g, 3.30 mmol), compound **6b** (1.11 g, 9.90 mmol), and XPhos-Pd-G3 (276 mg, 330 µmol) in dioxane (5 mL) in a nitrogen atmosphere, and the mixture was allowed to react sufficiently at 80 °C. After the reaction mixture was cooled, a saturated ammonium chloride solution (10 mL) was added, and ethyl acetate (60 mL × 3) was added for extraction. The organic phases were combined, dried over anhydrous sodium sulfate, and filtered, and the filtrate was concentrated to dryness by rotary evaporation. The residue was purified by flash column chromatography (petroleum ether:ethyl acetate = 100:0 to 90:10) to give compound **6c** (320 mg).

### Step 2: synthesis of compound 6d

Hydroxylamine hydrochloride (187 mg, 2.69 mmol) was added to a solution of compound **6c** (300 mg, 897 µmol) and sodium acetate (368 mg, 4.49 mmol) in methanol (5 mL), and the mixture was stirred at room temperature and allowed to react sufficiently. The reaction mixture was poured into water (25 mL), and ethyl acetate (50 mL × 3) was added for extraction. The organic phases were combined, dried over anhydrous sodium sulfate, and concentrated to dryness by rotary evaporation to give a crude product of compound **6d** (310 mg).

### Step 3: synthesis of compound 6e

Methylsulfonyl chloride (305 mg, 2.66 mmol) was slowly added dropwise to a solution of compound **6d** (310 mg, 887 µmol) and 2,4,6-trimethylpyridine (538 mg, 4.44 mmol) in dichloromethane (5 mL), and the mixture was stirred at room temperature and allowed to react sufficiently. The reaction mixture was poured into water (50 mL), and dichloromethane (20 mL × 3) was added for extraction. The organic phases were combined, dried over anhydrous sodium sulfate, and concentrated to dryness by rotary evaporation, and the residue was separated by column chromatography (petroleum ether:ethyl acetate = 100:0 to 95:5) to give compound **6e** (180 mg, yield: 61%).

### Step 4: synthesis of compound 6f

Compound **6f** (60 mg) was obtained through synthesis and purification using a method identical to that described in step 9 of Example 1, with compound **6e** (100 mg, 0.3 mmol) as starting material. MS m/z (ESI): 232.1 [M+H]⁺.

### Step 5: synthesis of compound 6g

Compound **6g** (26 mg) was obtained through synthesis and purification using a method identical to that described in step 10 of Example 1, with compound **6f** (46 mg, 0.2 mmol) and compound **1f** (94 mg, 0.2 mmol) as starting materials.

### Step 6: synthesis of compound 6-6

Boron tribromide (1.30 g, 5.19 mmol) was added dropwise to a solution of compound **6g** (21 mg, 37 µmol) in dichloromethane (2.5 mL), and the mixture was allowed to react sufficiently at 50 °C. After the reaction mixture was cooled, water (25 mL) was added to quench the reaction, and dichloromethane (25 mL × 3) was added for extraction. The organic phases were combined, dried over anhydrous sodium sulfate, and filtered, and the filtrate was concentrated to dryness by rotary evaporation. The residue was purified by preparative chromatography (acetonitrile:water, 20-85%, 0.1% ammonia water) to give compound **6-6** (11 mg).

¹H-NMR (400 MHz, DMSO-*d*₆) δ 12.65 (s, 1H), 10.18 (s, 1H), 10.06 (s, 1H), 8.01 (d, *J* = 8.6 Hz, 1H), 7.59 (d, *J* = 8.1 Hz, 1H), 7.27 (d, *J* = 2.1 Hz, 1H), 7.08 (dd, *J* = 8.6, 2.0 Hz, 1H), 6.39 (d, *J* = 8.2 Hz, 1H), 4.95 (s, 1H), 3.76 (t, *J =* 6.5 Hz, 2H), 3.39 - 3.36 (m, 2H), 3.13 - 3.05 (m, 2H), 3.03 - 2.91 (m, 6H), 1.91 - 1.84 (m, 2H), 1.84 - 1.53 (m, 8H), 0.31 (s, 4H).

MS m/z (ESI): 554.2 [M+H]⁺.

### Example 7: Synthesis of Compound 6-7

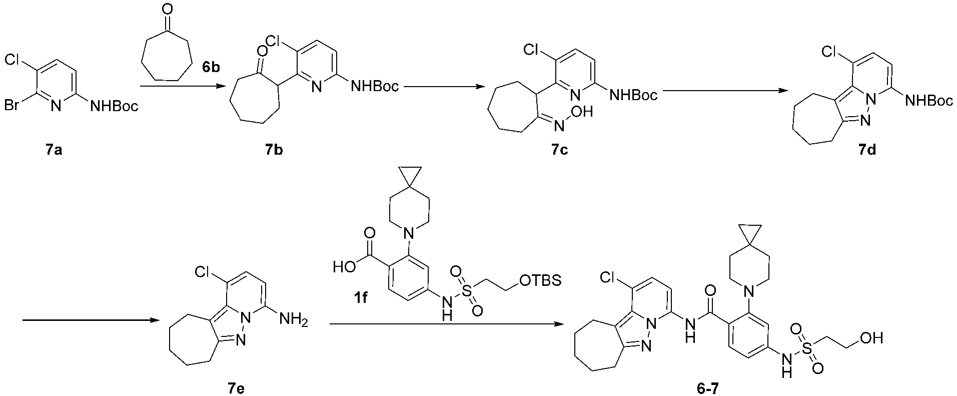

### Step 1: synthesis of compound 7b

Compound **7b** (440 mg) was obtained through synthesis and purification using a method identical to that described in step 1 of Example 6, with compounds **7a** (1.00 g, 3.25 mmol) and **6b** (1.09 g, 9.75 mmol) as starting materials.

### Step 2: synthesis of compound 7c

Compound **7c** (375 mg) was obtained through synthesis and purification using a method identical to that described in step 2 of Example 6, with compound **7b** (400 mg, 1.18 mmol) as starting material.

### Step 3: synthesis of compound 7d

Compound **7d** (145 mg) was obtained through synthesis and purification using a method identical to that described in step 3 of Example 6, with compound **7c** (333 mg, 0.94 mmol) as starting material.

### Step 4: synthesis of compound 7e

Compound **7e** (71 mg) was obtained through synthesis and purification using a method identical to that described in step 9 of Example 1, with compound **7d** (135 mg, 0.4 mmol) as starting material. MS m/z (ESI): 236.1 [M+H]⁺.

### Step 5: synthesis of compound 6-7

Compound **6-7** (31 mg) was obtained through synthesis and purification using a method identical to that described in step 10 of Example 1, with compound **7e** (47 mg, 0.2 mmol) and compound **1f** (94 mg, 0.2 mmol) as starting materials.

¹H-NMR (400 MHz, DMSO-*d*₆) δ 12.97 (s, 1H), 10.27 (s, 1H), 8.03 (d, *J =* 8.6 Hz, 1H), 7.79 (d, *J=* 8.3 Hz, 1H), 7.33 - 7.27 (m, 2H), 7.11 (dd, *J* = 8.6, 2.1 Hz, 1H), 4.95 (s, 1H), 3.76 (t, *J* = 6.4 Hz, 2H), 3.37 (t, *J* = 6.5 Hz, 2H), 3.23 - 3.15 (m, 2H), 3.07 - 2.94 (m, 6H), 1.94 - 1.84 (m, 2H), 1.84 - 1.52 (m, 8H), 0.32 (s, 4H).

MS m/z (ESI): 572.2 [M+H]⁺.

### Example 8: Synthesis of Compound 6-8

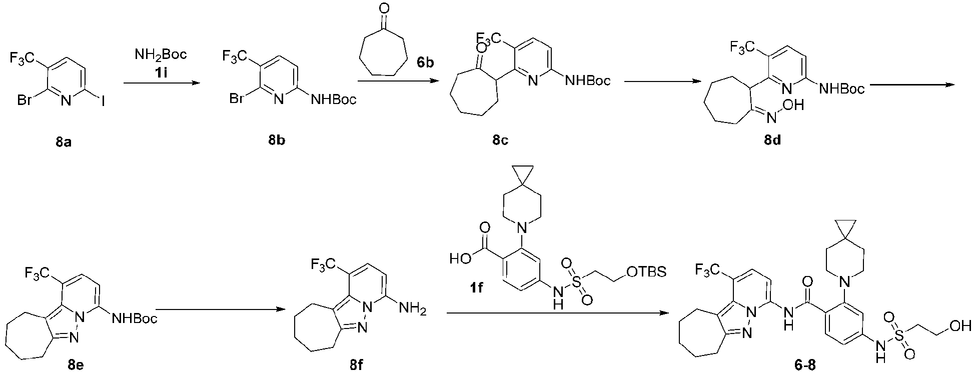

### Step 1: synthesis of compound 8b

Compound **8b** (650 mg) was obtained through synthesis and purification using a method identical to that described in step 8 of Example 1, with compounds **8a** (2.0 g, 5.7 mmol) and **1i** (0.7 g, 6.0 mmol) as starting materials.

### Step 2: synthesis of compound 8c

Compound **8c** (77 mg) was obtained through synthesis and purification using a method identical to that described in step 1 of Example 6, with compounds **8b** (0.64 g, 1.87 mmol) and **6b** (0.37 g, 3.3 mmol) as starting materials.

### Step 3: synthesis of compound 8d

Compound **8d** (67 mg) was obtained through synthesis and purification using a method identical to that described in step 2 of Example 6, with compound **8c** (75 mg, 0.2 mmol) as starting material.

### Step 4: synthesis of compound 8e

Compound **8e** (49 mg) was obtained through synthesis and purification using a method identical to that described in step 3 of Example 6, with compound **8d** (62 mg, 0.16 mmol) as starting material.

### Step 5: synthesis of compound 8f

Compound **8f** (29 mg) was obtained through synthesis and purification using a method identical to that described in step 9 of Example 1, with compound **8e** (41 mg, 0.11 mmol) as starting material. MS m/z (ESI): 270.1 [M+H]⁺.

### Step 6: synthesis of compound 6-8

Compound **6-8** (8.2 mg) was obtained through synthesis and purification using a method identical to that described in step 10 of Example 1, with compound **8f** (27 mg, 0.1 mmol) and compound **1f** (47 mg, 0.1 mmol) as starting materials.

¹H-NMR (400 MHz, DMSO-*d*₆) δ 13.17 (s, 1H), 8.03 (d, *J =* 8.7 Hz, 1H), 7.91 (d, *J =* 8.2 Hz, 1H), 7.72 (d, *J =* 8.3 Hz, 1H), 7.28 (d, *J =* 2.1 Hz, 1H), 7.09 (dd, *J =* 8.6, 2.1 Hz, 1H), 3.76 (t, *J =* 6.4 Hz, 2H), 3.39 - 3.36 (m, 2H), 3.10 - 2.97 (m, 6H), 2.89 - 2.82 (m, 2H), 1.93 - 1.85 (m, 2H), 1.84 - 1.53 (m, 8H), 0.32 (s, 4H).

MS m/z (ESI): 606.2 [M+H]⁺.

### Example 9 and Example 10: Synthesis of Compound 6-9 and Compound 6-10

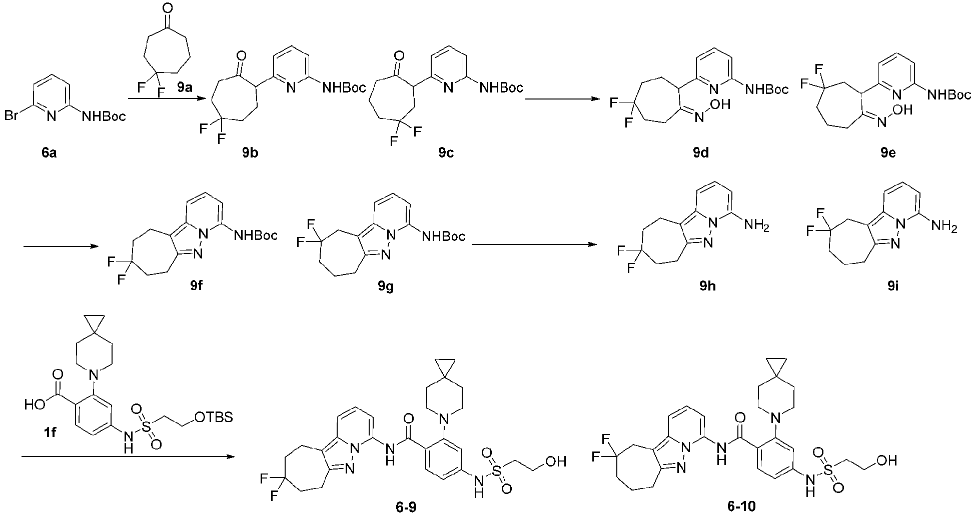

### Step 1: synthesis of mixture 9b/9c

Mixture **9b/9c** (1.9 g) was obtained through synthesis and purification using a method identical to that described in step 1 of Example 6, with compounds **6a** (1.8 g, 6.5 mmol) and **9a** (1.0 g, 6.75 mmol) as starting materials.

### Step 2: synthesis of mixture 9d/9e

Mixture **9d/9e** (1.6 g) was obtained through synthesis and purification using a method identical to that described in step 2 of Example 6, with mixture **9b/9c** (1.6 g, 4.7 mmol) as starting material. Step 3: synthesis of mixture **9f/9g**

Mixture **9f/9g** (445 mg) was obtained through synthesis and purification using a method identical to that described in step 3 of Example 6, with mixture **9d/9e** (1.6 g, 4.5 mmol) as starting material.

### Step 4: synthesis of mixture 9h/9i

Mixture **9h/9i** (117 mg) was obtained through synthesis and purification using a method identical to that described in step 9 of Example 1, with mixture **9f/9g** (380 mg, 1.13 mmol) as starting material. MS m/z (ESI): 238.1 [M+H]⁺.

### Step 6: synthesis of compound 6-9 and compound 6-10

Mixture **6-9/6-10** was obtained through synthesis and purification using a method identical to that described in step 10 of Example 1, with mixture **9h/9i** (95 mg, 0.4 mmol) and compound **1f** (230 mg, 0.5 mmol) as starting materials, and it was then purified by preparative chromatography (acetonitrile:water, 20-85%, 0.1% ammonia water) to give:
compound **6-9** (15 mg)
¹H NMR (400 MHz, DMSO-*d*₆) δ 13.02 (s, 1H), 10.21 (s, 1H), 8.06 (d, *J* = 8.6 Hz, 1H), 7.88 (dd, *J* = 7.6, 1.2 Hz, 1H), 7.40 - 7.30 (m, 2H), 7.25 (dd, *J =* 8.9, 7.5 Hz, 1H), 7.12 (dd, *J =* 8.6, 2.1 Hz, 1H), 4.94 (s, 1H), 3.77 (t, *J =* 6.5 Hz, 2H), 3.37 (t, *J =* 6.5 Hz, 2H), 3.10 - 2.96 (m, 6H), 2.92 - 2.81 (m, 2H), 2.30 - 2.12 (m, 4H), 1.76 (s, 4H), 0.33 (s, 4H).

MS m/z (ESI): 574.1 [M+H]⁺.

### compound 6-10 (8.0 mg)

¹H NMR (400 MHz, DMSO-*d*₆) δ 12.99 (s, 1H), 10.23 (s, 1H), 8.05 (d, *J =* 8.6 Hz, 1H), 7.88 (dd, *J* = 7.6, 1.2 Hz, 1H), 7.44 (dd, *J* = 8.9, 1.2 Hz, 1H), 7.31 (d, *J* = 2.2 Hz, 1H), 7.26 (dd, *J* = 8.8, 7.5 Hz, 1H), 7.11 (dd, *J* = 8.6, 2.1 Hz, 1H), 4.93 (s, 1H), 3.77 (t, *J* = 6.4 Hz, 2H), 3.46 (t, *J* = 13.6 Hz, 2H), 3.37 (t, *J* = 6.5 Hz, 2H), 3.13 - 3.04 (m, 2H), 3.01 (t, *J* = 5.4 Hz, 4H), 2.44 - 2.34 (m, 2H), 1.94 - 1.60 (m, 6H), 0.32 (s, 4H).

MS m/z (ESI): 574.2 [M+H]⁺.

### Example 11: Synthesis of Compound 6-11

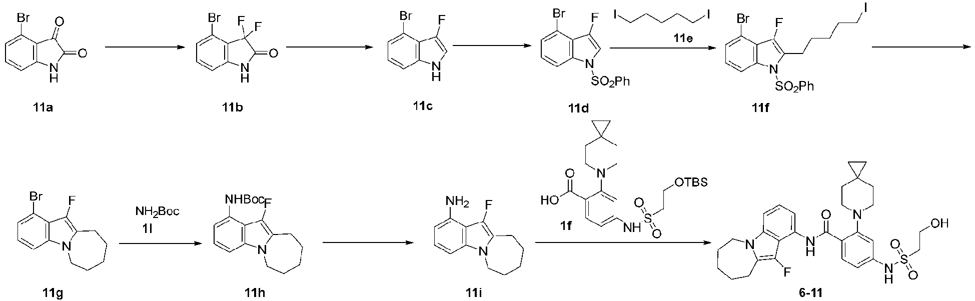

### Step 1: synthesis of compound 11b

DAST (diethylaminosulfur trifluoride) (1.78 g, 11.1 mmol) was slowly added to a solution of compound **11a** (1.0 g, 4.42 mmol) in dichloromethane (10 mL) at 0 °C, and the mixture was slowly warmed to room temperature and stirred and allowed to react sufficiently. Methanol (5 mL) was added to the reaction mixture to quench the reaction, and the reaction mixture was concentrated under reduced pressure. The residue was purified by column chromatography (petroleum ether:ethyl acetate = 4:1) to give compound **11b** (0.90 g).

### Step 2: synthesis of compound 11c

Sodium borohydride (3.7 g, 98 mmol) and boron trifluoride diethyl etherate (13.9 g, 98 mmol) were added to a solution of compound **11b** (8.1 g, 32.7 mmol) in tetrahydrofuran (50 mL) at 0 °C, and the mixture was warmed to room temperature and then allowed to react sufficiently. The reaction mixture was added to ice water (200 mL), and ethyl acetate (50 mL × 3) was added for extraction. The combined organic phases were dried over anhydrous sodium sulfate and concentrated, and the residue was purified by column chromatography (petroleum ether:ethyl acetate = 4:1) to give compound **11c** (5.60 g).

### Step 3: synthesis of compound 11d

Sodium hydride (972 mg, 24.3 mmol, 60% dispersion in mineral oil) was added in portions to a solution of compound **11c** (4.0 g, 18.7 mmol) in tetrahydrofuran (50 mL) at 0 °C, and the mixture was stirred at 0 °C. Benzenesulfonyl chloride (3.30 g, 18.7 mmol) was then added to the reaction mixture. The mixture was warmed to room temperature and then stirred and allowed to react sufficiently. The reaction mixture was slowly poured into ice water (200 mL), and ethyl acetate (100 mL × 3) was added for extraction. The combined organic phases were dried over anhydrous sodium sulfate and concentrated, and the residue was separated and purified by column chromatography (petroleum ether:ethyl acetate = 3:1) to give compound **11d** (5.60 g).

### Step 4: synthesis of compound 11f

Compound **11d** (1.00 g, 2.82 mmol) and tetramethylethylenediamine (656 mg, 5.65 mmol) were added to tetrahydrofuran (15 mL) in a nitrogen atmosphere, and lithium diisopropylamide (2 M, 1.69 mL) was added dropwise to the mixture at 0 °C. The mixture was stirred at 0 °C, and compound **11e** (1.10 g, 3.39 mmol) was added to the system. The mixture was allowed to react sufficiently at that temperature. The reaction mixture was poured into a saturated ammonium chloride solution (60 mL), and ethyl acetate (100 mL × 3) was added for extraction. The combined organic phases were dried over anhydrous sodium sulfate and concentrated, and the residue was separated and purified by column chromatography (petroleum ether:ethyl acetate = 5:1) to give compound **11f** (960 mg).

### Step 5: synthesis of compound 11g

Compound **11f** (540 mg, 981 µmol) and sodium tert-butoxide (189 mg, 1.96 mmol) were added to dioxane (5 mL), and the mixture was allowed to react at 80 °C for 4 h. The reaction mixture was poured into water, and ethyl acetate (100 mL × 3) was added for extraction. The combined organic phases were dried over anhydrous sodium sulfate and concentrated, and the residue was separated and purified by column chromatography (petroleum ether:ethyl acetate = 6:1) to give compound **11g** (190 mg).

### Step 6: synthesis of compound 11h

Compound **11h** (50 mg) was obtained through synthesis and purification using a method identical to that described in step 8 of Example 1, with compounds **11g** (170 mg, 602 µmol) and **1l** (282 mg, 2.41 mmol) as starting materials.

### Step 7: synthesis of compound 11i

Compound **11i** (15 mg) was obtained through synthesis and purification using a method identical to that described in step 9 of Example 1, with compound **11h** (45 mg, 141 µmol) as starting material.

### Step 8: synthesis of compound 6-11

Compound **6-11** (1.5 mg) was obtained through synthesis and purification using a method identical to that described in step 10 of Example 1, with compounds **11i** (10 mg, 46 µmol) and **1f** (20 mg, 43 µmol) as starting materials.

¹H NMR (400 MHz, DMSO-*d*₆) δ 11.17 (s, 1H), 9.92 (s, 1H), 7.86 (d, *J* = 8.5 Hz, 1H), 7.80 (d, *J* = 7.7 Hz, 1H), 7.28 (dd, *J* = 8.5, 2.3 Hz, 1H), 7.17 (d, *J* = 2.1 Hz, 1H), 7.10 (t, *J* = 8.1 Hz, 1H), 7.03 (dd, *J =* 8.5, 2.0 Hz, 1H), 4.96 (s, 1H), 4.29 - 4.17 (m, 2H), 3.77 (t, *J =* 6.5 Hz, 2H), 3.40 - 3.35 (m, 2H), 3.08 - 2.93 (m, 4H), 2.93 - 2.81 (m, 2H), 1.86 - 1.76 (m, 2H), 1.73 - 1.62 (m, 4H), 1.49 - 1.38 (m, 4H), 0.28 (s, 4H).

MS m/z (ESI): 555.2 [M+H]⁺.

### Example 12: Synthesis of Compound 6-12

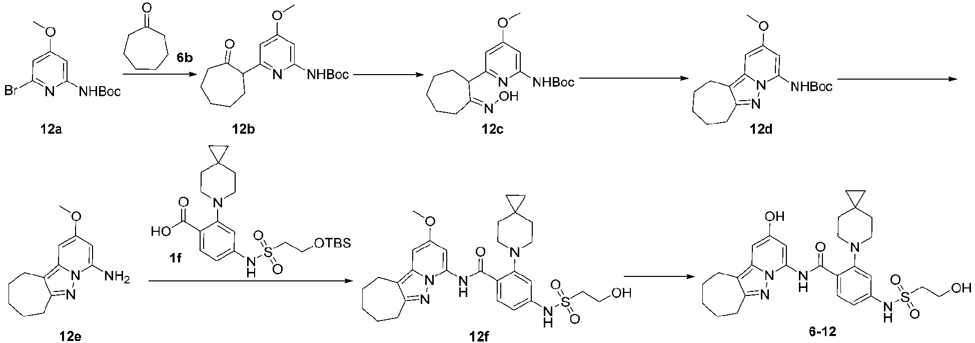

Compound **6-12** (3.6 mg) was obtained through synthesis and purification using a method identical to that described in Example 6, with compounds **12a** and **6b** as starting materials.

MS m/z (ESI): 554.2 [M+H]⁺.

### Example 14: Synthesis of Compound 6-14

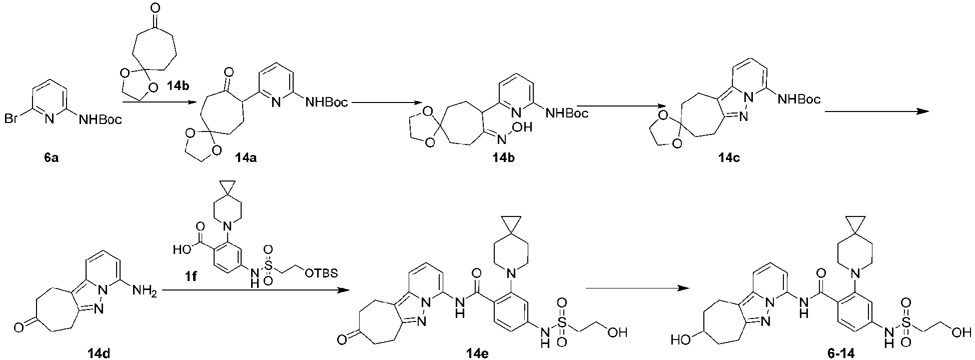

### Step 1: synthesis of compound 14a

Compound **14a** (127 mg) was obtained through synthesis and purification using a method identical to that described in step 1 of Example 6, with compounds **6a** (300 mg, 1.10 mmol) and **14b** (375 mg, 2.20 mmol) as starting materials.

### Step 2: synthesis of compound 14b

Compound **14b** (133 mg) was obtained through synthesis and purification using a method identical to that described in step 2 of Example 6, with compound **14a** (127 mg, 352 µmol) as starting material.

### Step 3: synthesis of compound 14c

Compound **14c** (45.4 mg) was obtained through synthesis and purification using a method identical to that described in step 3 of Example 6, with compound **14b** (133 mg, 352 µmol) as starting material.

### Step 4: synthesis of compound 14d

Compound **14d** (14.6 mg) was obtained through synthesis and purification using a method identical to that described in step 9 of Example 1, with compound **14c** (45.5 mg, 126 µmol) as starting material.

### Step 5: synthesis of compound 14e

Compound **14e** (22 mg) was obtained through synthesis and purification using a method identical to that described in step 10 of Example 1, with compounds **14d** (14.6 mg, 68 µmol) and **1f** (32.1 mg, 68.0 µmol) as starting materials.

### Step 6: synthesis of compound 6-14

Compound **14e** (22 mg, 40 µmol) was added to methanol (3 mL), and sodium borohydride (5.5 mg, 144 µmol) was added at 0 °C. The mixture was allowed to react sufficiently at 0 °C. Hydrochloric acid (2 mL, 1 N) was added to the system, and ethyl acetate (1 mL × 3) was added for extraction. The organic phases were combined, washed with saturated brine, dried over anhydrous magnesium sulfate, filtered, and concentrated to dryness by rotary evaporation to give compound **6-14** (6.1 mg). MS m/z (ESI): 554.3 [M+H]⁺.

### Example 15: Synthesis of Compound 6-15

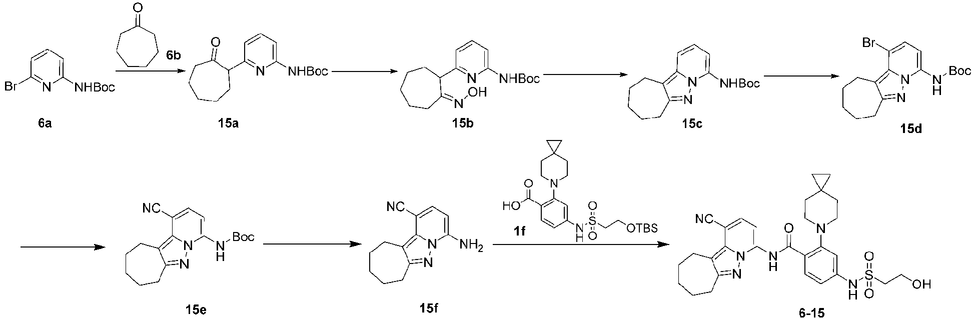

### Step 1: synthesis of compound 15a

Compound **15a** (1.5 g) was obtained through synthesis and purification using a method identical to that described in step 1 of Example 6, with compounds **6a** (2.7 g, 10 mmol) and **6b** (1.35 g, 12 mmol) as starting materials.

### Step 2: synthesis of compound 15b

Compound **15b** (1.5 g) was obtained through synthesis and purification using a method identical to that described in step 2 of Example 6, with compound **15a** (1.5 g, 5 mmol) as starting material.

### Step 3: synthesis of compound 15c

Compound **15c** (0.6 g) was obtained through synthesis and purification using a method identical to that described in step 3 of Example 6, with compound **15b** (1.5 g, 4.7 mmol) as starting material.

### Step 4: synthesis of compound 15d

Compound **15c** (100 mg, 332 µmol) was added to dichloromethane (4 mL) and n-hexane (1 mL), and a solution of NBS (35.4 mg, 199 µmol) in dichloromethane (5 mL) was added dropwise at -60 °C. The mixture was allowed to react sufficiently at that temperature. A saturated sodium thiosulfate solution (10 mL) was added to the system, and dichloromethane (5 mL × 2) was added for extraction. The organic phases were combined, washed with saturated brine, dried over anhydrous magnesium sulfate, and filtered, and the filtrate was concentrated to dryness by rotary evaporation. The residue was purified by flash column chromatography (petroleum ether:ethyl acetate = 4:1) to give compound **15d** (44 mg).

### Step 5: synthesis of compound 15e

Compound **15d** (44 mg, 116 µmol), zinc cyanide (8.2 mg, 70 mmol), and bis(diphenylphosphino)ferrocene (6.42 mg, 11.6 µmol) were added to DMF (0.5 mL) and water (0.02 mL), followed by the addition of Pd₂(dba)₃ (5.30 mg, 5.8 µmol). The mixture was allowed to react sufficiently at 120 °C. After the reaction mixture was cooled, diluted ammonia water (3 mL) was added to the system, and ethyl acetate (2 mL × 3) was added for extraction. The organic phases were combined, washed with saturated brine, dried over anhydrous magnesium sulfate, and filtered, and the filtrate was concentrated to dryness by rotary evaporation. The residue was purified by flash column chromatography (petroleum ether:ethyl acetate = 2:1) to give compound **15e** (27.2 mg).

### Step 6: synthesis of compound 15f

Compound **15f** (11.8 mg) was obtained through synthesis and purification using a method identical to that described in step 9 of Example 1, with compound **15e** (27.2 mg, 83.4 µmol) as starting material.

### Step 7: synthesis of compound 6-15

Compound **6-15** (6.9 mg) was obtained through synthesis and purification using a method identical to that described in step 10 of Example 1, with compounds **15f** (9.8 mg, 43 µmol) and **1f** (20 mg, 43 µmol) as starting materials.

MS m/z (ESI): 563.2 [M+H]⁺.

### Example 16: Synthesis of Compound 6-16

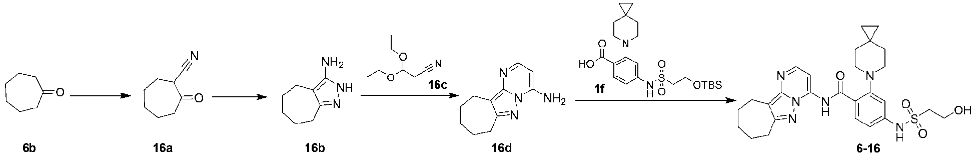

### Step 1: synthesis of compound 16a

Compound **6b** (571 mg, 5.1 mmol) and DIPEA (713 mg, 5.5 mmol) were added to diethyl ether (10 mL), and 9-iodo-9-borabicyclo[3.3.1]nonane (1 M in hexane, 5.50 mL, 5.50 mmol) was added dropwise at 0 °C. The mixture was allowed to react at room temperature, and then a solution of trimethylsilyl cyanide (1.63 g, 5.99 mmol) in diethyl ether (10 mL) was added. The mixture was allowed to react sufficiently at room temperature. The reaction mixture was filtered through silica gel and concentrated to dryness by rotary evaporation to give compound **16a** (697 mg, crude product).

### Step 2: synthesis of compound 16b

Compound **16a** (697 mg, 5.09 mmol) was added to hydrazine hydrate (5 mL) and absolute ethanol (15 mL), and the mixture was allowed to react sufficiently at 80 °C. The reaction mixture was concentrated, and the residue was purified by flash column chromatography (acetonitrile:water = 0-50%) to give compound **16b** (300 mg).

### Step 3: synthesis of compound 16d

Compounds **16b** (50 mg, 331 µmol) and **16c** (95 mg, 662 µmol) were added to absolute ethanol (1 mL), followed by the addition of hydrochloric acid (0.2 mL, 2 N). The mixture was allowed to react sufficiently at 60 °C. The reaction mixture was concentrated to dryness by rotary evaporation, and the residue was purified by reversed-phase column chromatography (acetonitrile:water = 0-50%) to give compound **16d** (30.1 mg).

### Step 4: synthesis of compound 6-16

Compound **6-16** (6.3 mg) was obtained through synthesis and purification using a method identical to that described in step 10 of Example 1, with compounds **16d** (8.6 mg, 43 µmol) and **1f** (20 mg, 43 µmol) as starting materials.

¹H-NMR (400 MHz, DMSO-*d*₆) δ 13.64 (s, 1H), 8.36 (d, *J* = 2.7 Hz, 1H), 8.34 (s, 1H), 7.94 (d, *J =* 8.8 Hz, 1H), 7.82 (d, *J =* 5.0 Hz, 1H), 7.08 (d, *J =* 2.1 Hz, 1H), 6.89 (d, 8.7 Hz, 1H), 3.74 (t, *J =* 6.6 Hz, 2H), 3.12 (t, *J =* 6.6 Hz, 2H), 3.09 - 2.94 (m, 6H), 2.90 - 2.82 (m, 2H), 1.94 - 1.84 (m, 2H), 1.84 - 1.63 (m, 6H), 0.36 (s, 4H).

MS m/z (ESI): 539.2 [M+H]⁺.

### Example 17: Synthesis of Compound 6-17

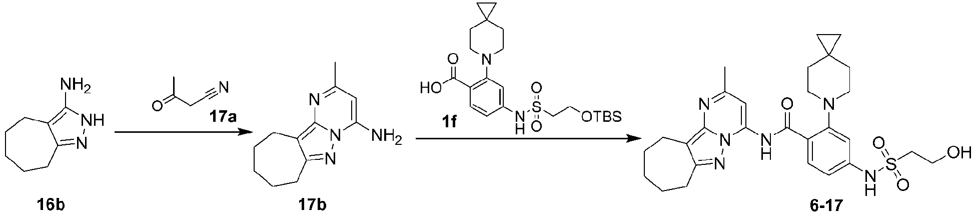

### Step 1: synthesis of compound 17b

Compound **17b** (25.7 mg) was obtained through synthesis and purification using a method identical to that described in step 3 of Example 16, with compounds **16b** (50.0 mg, 331 µmol) and **17a** (54.9 mg, 662 µmol) as starting materials.

### Step 2: synthesis of compound 6-17

Compound **6-17** (8.2 mg) was obtained through synthesis and purification using a method identical to that described in step 10 of Example 1, with compounds **17b** (9.2 mg, 43 µmol) and **1f** (20 mg, 43 µmol) as starting materials.

¹H-NMR (400 MHz, DMSO-*d*₆) δ 13.39 (s, 1H), 8.09 (d, *J* = 8.6 Hz, 1H), 7.71 (s, 1H), 7.35 (s, 1H), 7.16 (d, *J* = 8.6 Hz, 1H), 3.78 (t, *J* = 6.4 Hz, 2H), 3.02 (t, *J =* 5.5 Hz, 2H), 3.02 - 2.94 (m, 6H), 2.89 - 2.79 (m, 2H), 1.92 - 1.82 (m, 2H), 1.80 - 1.60 (m, 6H), 0.36 (s, 4H).

MS m/z (ESI): 553.3 [M+H]⁺.

### Example 18: Synthesis of Compound 6-18

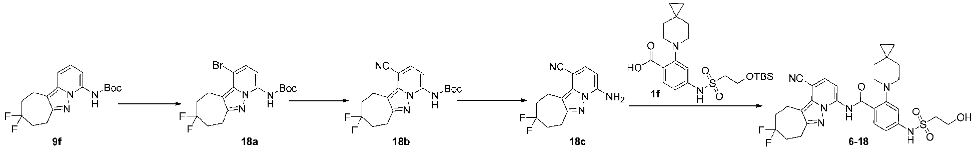

Compound **6-18** (2.1 mg) was obtained through synthesis and purification using a method identical to that described in Example 15, with compound **9f** as starting material.

¹H NMR (400 MHz, DMSO-*d*₆) δ 13.45 (s, 1H), 8.07 (d, *J =* 8.7 Hz, 1H), 8.00 (d, *J =* 8.2 Hz, 1H), 7.97 (d, *J =* 8.2 Hz, 1H), 7.34 (d, *J* = 2.1 Hz, 1H), 7.14 (dd, *J* = 8.7, 2.1 Hz, 1H), 3.76 (t, *J* = 6.4 Hz, 2H), 3.38 (t, *J* = 6.4 Hz, 2H), 3.16 - 3.06 (m, 4H), 3.06 - 2.96 (m, 4H), 2.32 - 2.18 (m, 4H), 1.85 - 1.64 (m, 4H), 0.35 (s, 4H).

MS m/z (ESI): 599.2 [M+H]⁺.

### Example 19: Synthesis of Compound 6-19

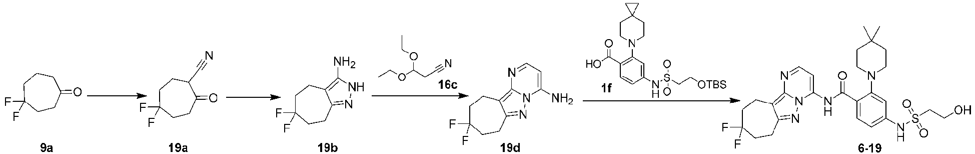

Compound **6-19** (3.5 mg) was obtained through synthesis and purification using a method identical to that described in Example **16,** with compound **9a** (5.00 g, 33.7 mmol) as starting material.

¹H NMR (400 MHz, DMSO-*d*₆) δ 13.58 (s, 1H), 10.36 (s, 1H), 8.44 (d, *J* = 5.0 Hz, 1H), 8.09 (d, *J* = 8.8 Hz, 1H), 7.86 (d, *J* = 5.0 Hz, 1H), 7.35 (s, 1H), 7.15 (d, *J* = 8.5 Hz, 1H), 4.91 (s, 1H), 3.77 (t, *J* = 6.4 Hz, 2H), 3.38 - 3.36 (m, 2H), 3.10 - 3.04 (m, 2H), 3.04 - 2.94 (m, 4H), 2.94 - 2.86 (m, 2H), 2.29 - 2.13 (m, 4H), 1.93 - 1.68 (m, 4H), 0.36 (s, 4H).

MS m/z (ESI): 575.2 [M+H]⁺.

### Example 20: Synthesis of Compound 6-20

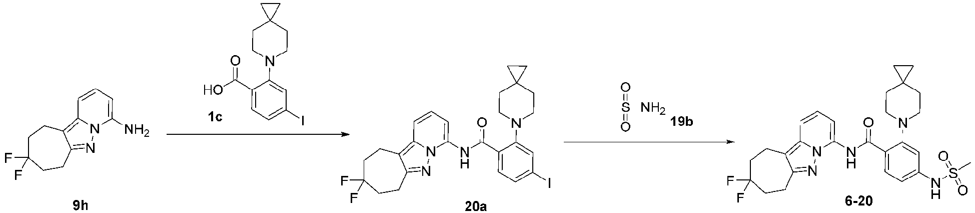

### Step 1: synthesis of compound 20a

Compound **20a** (0.80 g) was obtained through synthesis and purification using a method identical to that described in step 10 of Example 1, with compound **9h** (400 mg, 1.69 mmol) and compound **1c** (590 mg, 1.65 mmol) as starting materials.

### Step 2: synthesis of compound 6-20

Compound **20a** (100 mg, 173 µmol), methanesulfonamide **19b** (21 mg, 225 µmol), (1R,2R)-N,N'-dimethyl-1,2-cyclohexanediamine (25 mg, 173 µmol), cuprous iodide (17 mg, 87 µmol), and potassium phosphate (184 mg, 867 µmol) were added to DMF (2 mL) in a nitrogen atmosphere. The mixture was allowed to react sufficiently at 100 °C. After being cooled, the reaction mixture was poured into water (10 mL), and ethyl acetate (10 mL × 3) was added for extraction. The organic phases were combined, dried over anhydrous sodium sulfate, and filtered, and the filtrate was concentrated to dryness by rotary evaporation. The residue was purified by reversed-phase column chromatography (acetonitrile:water = 30-95%) to give compound **6-20** (60.1 mg).

¹H NMR (400 MHz, DMSO-*d*₆) δ 13.02 (s, 1H), 10.28 (s, 1H), 8.07 (d, *J* = 8.6 Hz, 1H), 7.89 (dd, *J* = 7.5, 1.2 Hz, 1H), 7.37 (dd, *J* = 8.9, 1.2 Hz, 1H), 7.30 (d, *J* = 2.2 Hz, 1H), 7.25 (dd, *J* = 8.9, 7.5 Hz, 1H), 7.12 (dd, *J* = 8.6, 2.1 Hz, 1H), 3.13 (s, 3H), 3.09 - 2.98 (m, 6H), 2.90 - 2.83 (m, 2H), 2.31 - 2.11 (m, 4H), 1.91 - 1.62 (m, 4H), 0.33 (s, 4H).

MS m/z (ESI): 544.2 [M+H]⁺.

### Example 21: Synthesis of Compound 6-21

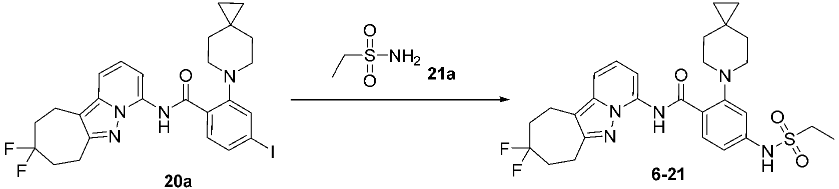

Compound **6-21** (51 mg) was obtained through synthesis and purification using a method identical to that described in step 2 of Example **20,** with compound **20a** (100 mg, 173 µmol) and ethyl sulfonamide **21a** (25 mg, 225 µmol) as starting materials.

¹H NMR (400 MHz, DMSO-*d*₆) δ 13.01 (s, 1H), 10.27 (s, 1H), 8.04 (d, *J =* 8.6 Hz, 1H), 7.88 (dd, *J* = 7.5, 1.2 Hz, 1H), 7.37 (dd, *J* = 8.7, 1.2 Hz, 1H), 7.31 (d, *J* = 2.2 Hz, 1H), 7.25 (dd, *J* = 8.8, 7.5 Hz, 1H), 7.11 (dd, *J* = 8.6, 2.1 Hz, 1H), 3.22 (q, *J* = 7.3 Hz, 2H), 3.12 - 2.96 (m, 6H), 2.90 - 2.82 (m, 2H), 2.30 - 2.11 (m, 4H), 1.85 - 1.64 (m, 4H), 1.21 (t, *J =* 7.3 Hz, 3H), 0.33 (s, 4H).

MS m/z (ESI): 558.2 [M+H]⁺.

### Example 22: Synthesis of Compound 6-22

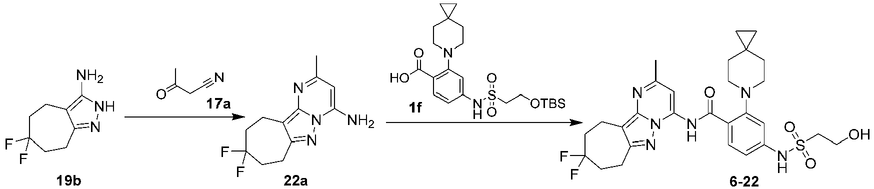

Compound **6-22** (8.5 mg) was obtained through synthesis and purification using a method identical to that described in Example **17,** with compounds **19b** (100 mg, 535 µmol) and **17a** (83 mg, 1.0 mmol) as starting materials.

MS m/z (ESI): 589.2 [M+H]⁺.

### Example 23: Synthesis of Compound 6-23

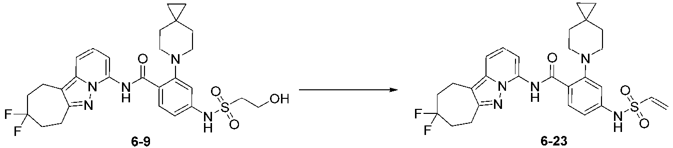

Compound **6-9** (28 mg, 50 µmol) and triethylamine (15 mg, 150 µmol) were dissolved in dichloromethane (1 mL), and methanesulfonic anhydride (17 mg, 100 µmol) was added dropwise. The mixture was allowed to react sufficiently at room temperature. The volatiles were removed under reduced pressure, and the residue was purified by reversed-phase column chromatography (acetonitrile:water = 45-99%) to give compound **6-23** (9 mg).

MS m/z (ESI): 556.2 [M+H]⁺.

### Evaluations of Biological Activity

### Test Example 1: KIF18A Enzyme Activity Assay

Based on the ADP-Glo^{™} Kinase Assay, the ADP amount generated in the reaction was determined to reflect the enzyme activity of KIF18A.

### 1. Experimental instruments and materials

| Instrument | Manufacturer | Model |
|---|---|---|
| Oscillator | Boxun | BSD-YX3400 |
| Plate reader | PerkinElmer | Envision |
| Centrifuge | Eppendorf | Eppendorf Mixmate |
| Compound dilution and loading instrument | PerkinElmer | Echo |

Human KIF18A (1-376aa) protease used in the experiments, expressed by Shanghai Panchao Biotechnology Co., Ltd., was stored at -80 °C (no more than 5 freeze-thaw cycles).

The assay kit (ADP-Glo^{™} Kinase Assay) was purchased from Promega (Cat. No. V9101) and stored at -30 °C. The kit determines the enzyme activity by measuring ADP generated in enzyme reaction. The KIF18A enzyme and ATP generate ADP. The remaining ATP in this reaction is consumed by the ADP-Glo reagent, while the ADP generated by the KIF18A enzyme reaction is converted back to ATP by the assay reagent. The ATP then reacts with luciferin in the presence of Ultra-Glo^{™} luciferase, producing luminescence. The luminescent signal is positively correlated with the enzyme activity of KIF18A.

Other reagents and consumables required for the experiment are as follows

| Reagent | Brand | Cat. No. |
|---|---|---|
| ATP | Promega | V915A |
| Tris | Teknova Inc | T0664 |
| Pluronic F-68 | Thermo Fisher | 24040032 |
| Microtubule | Cytoskeleton | MT002 |
| Taxol | Cytoskeleton | TXD01 |
| Reagent reservoir | Corning | 4877 |
| White 384-well plate | Corning | 3765 |

### 2. Experimental procedure

Compounds were each subjected to 3-fold serial dilution starting from 10 µM to obtain 10 concentration points. The compounds and pure DMSO (control) were pipetted into wells of a 384-well plate using an Echo instrument, and the total volume of compound and DMSO was 100 nL; sample concentrations of gradient dilution were obtained by the instrument based on different ratios. Preparation of a KIF18A enzyme reaction buffer: 15 nM Tris, 10 mM MgCl₂, 0.01% Pluronic F-68, 1 µM Taxol, and 50 µg/mL Microtubule. KIF18A and the enzyme reaction buffer were mixed, and then the mixed solution was added to the 384-well plate and incubated at room temperature for 15 min. ATP (Km 70 µM) was then added to the mixed solution, and the mixed solution was incubated at room temperature for 15 min. 10 µL of ADP-Glo^{™} Reagent was well mixed with 10 µL of the mixed solution, and the mixture was incubated at room temperature for 40 min. Finally, 20 µL of ADP-Glo^{™} Detection Reagent was added, and the mixture was incubated at room temperature for 30 min. The chemiluminescent signal in each well was measured using an Envision plate reader (PerkinElmer, emission wavelength: 400-700 nm). In the experiment, 1 column of wells with no compound and no enzyme (chemiluminescence value was taken as [RLU]background) and 1 column of wells with enzyme but no compound (chemiluminescence value was taken as [RLU]enzyme) were provided. The chemiluminescence value of the compound adding group was taken as [RLU]cpd. The inhibition rate of the compound on proliferation was calculated according to the following equation: inhibition rate (%) = ([RLU]enzyme - [RLU]cpd)/([RLU]enzyme - [RLU]background) × 100%, and the inhibitory activity IC₅₀ value of the compound on the enzyme activity was calculated using a four-parameter Logistic Model method. In the following equation, x represents the logarithmic form of the concentration of compound, and f (x) represents an effect value (inhibition rate of enzyme activity at the concentration): F(x) = (A + ((B - A)/(1 + ((C/x)^D)))). A. B, C, and D are four parameters. IC₅₀ values were further calculated as the concentration of compound required for 50% inhibition of enzyme activity in the best-fit curve using Xlfit, and the KIF18A inhibitory activity of the compounds of the present disclosure is shown in Table 1.

**Table 1. KIF 18A inhibitory activity of compounds of the present disclosure**

| Compound No. | KIF18A inhibitory activity IC₅₀ (nM) | KIF18A inhibitory activity IC₅₀ (nM) |
|---|---|---|
| 6-1 | ++++ | 52.3 |
| 6-2 | ++++ | 21.4 |
| 6-3 | ++++ | 21.3 |
| 6-4 | ++++ | 25.5 |
| 6-5 | ++++ | 22.1 |
| 6-6 | ++++ | 29.5 |
| 6-7 | ++++ | 44.7 |
| 6-8 | ++++ | 124.5 |
| 6-9 | ++++ | 14.6 |
| 6-10 | ++++ | 19.1 |
| 6-11 | ++++ | 110 |
| 6-12 | ++++ | 86 |
| 6-14 | ++++ | 74.8 |
| 6-15 | ++++ | 81.1 |
| 6-16 | ++++ | 42.3 |
| 6-17 | ++++ | 51.4 |
| 6-18 | ++++ | 125 |
| 6-19 | ++++ | 33.7 |
| 6-20 | ++++ | 122 |
| 6-21 | ++++ | 182 |
| 6-22 | ++++ | 48.9 |
| 6-23 | +++ | 286 |

In the table above,
"++++" indicates that the IC₅₀ range of the test compound for enzyme inhibitory activity is < 200 nM;
"+++" indicates that the IC₅₀ range of the test compound for enzyme inhibitory activity is 200-500 nM.

### Test Example 2. Test on Inhibition of Proliferation of OVCAR-3 Cells by Compounds of Present Disclosure

**Cells and materials:** The human ovarian cancer cell line OVCAR3 was purchased from ATCC (Cat. No. HTB-161^{™}), RPMI 1640 medium (Gibco#A1049101), penicillin-streptomycin (Gibco#15140122), and 0.25% Trypsin-EDTA (Gibco#25200056) were purchased from Gibco (U.S.), bovine insulin (Yeasen#40107ES60) was purchased from Yeasen, 384-well plates (Corning#CLS3765) were purchased from Corning (U.S.), Cell-Titer Glo reagent (Promega#G7568) was purchased from Promega (U.S.), and Echo was purchased from PerkinElmer.

**Cell culture:** OVCAR3 cells were cultured in RPMI 1640 complete medium (RPMI 1640 medium containing 20% fetal bovine serum, 10 µg/mL bovine insulin, and 1% penicillin-streptomycin) in an incubator (37 °C, 5% CO₂), and the cells were able to be used in experiments when in the logarithmic growth phase.

**Cell proliferation activity test:** The Cell-Titer Glo reagent was used to test the compound for inhibition of proliferation of the OVCAR3 cell strain. The compound formulated with DMSO and pure DMSO (control) were each added to a 384-well plate using an Echo instrument; the compound was subjected to 3-fold serial dilution starting from 30 µM to obtain 11 concentration points, and the volume of the compound or DMSO added was 100 nL.

OVCAR3 cells were digested and resuspended in RPMI 1640 complete medium, and the suspension was added to the 384-well plate (1000 cells/50 µL/well) to be well mixed with the compound. The mixture was incubated in an incubator (37 °C, 5% CO₂) for 3 days. The Cell-Titer Glo reagent was added at 25 µL/well, and the resulting mixture was well mixed by shaking and then incubated for 10 min. The Cell-Titer Glo readings were measured with a Multimode Plate Reader (brand: PerkinElmer; model: SIC-PV-018).

A negative control group (Bottom) was set, and the negative control group was medium wells with 0.2% DMSO added and was defined as 100% proliferation inhibition; the positive control group (Top) was OVCAR3 cell wells with 0.2% DMSO added.

### Data analysis:

The percent proliferation inhibition (%Inhibition) was calculated, and the curves were fit using the four-parameter equation, Y = Bottom + (Top - Bottom)/(1 + (IC₅₀/X)^HillSlope), to obtain the proliferation inhibition IC₅₀ of the compound.
Percent inhibition: %Inhibition = (1 - (Signal - Bottom)/(Top - Bottom)) × 100%.
Signal: Cell-Titer Glo reading of wells with compound added;
Bottom: Cell-Titer Glo reading of negative control wells;
Top: Cell-Titer Glo reading of positive control wells.

### Experimental results:

The inhibitory activity of the compounds of present disclosure on OVCAR3 proliferation is shown in Table 2.

**Table 2. Inhibitory activity of compounds of the present disclosure on OVCAR3 proliferation**

| Compound No. | OVCAR3 proliferation inhibition IC₅₀ (nM) | OVCAR3 proliferation inhibition IC₅₀ (nM) |
|---|---|---|
| 6-1 | ++++ | 73.0 |
| 6-2 | ++++ | 6.62 |
| 6-3 | ++++ | 29.1 |
| 6-7 | ++++ | 70.4 |
| 6-9 | ++++ | 19.8 |
| 6-10 | ++++ | 88.1 |

In the table above, "++++" indicates that the IC₅₀ range of the test compound for OVCAR3 proliferation inhibitory activity is < 200 nM.

### Test example 3: Test on Inhibition of CYP Enzyme by Compounds of Present Disclosure

The metabolic reactions of representative substrates of 5 major human CYP subtypes (CYP1A2, CYP2C9, CYP2C19, CYP2D6, and CYP3A4/5) were evaluated using 150-donor pooled human liver microsomes (purchased from Coming, Cat. No. 452117). The effects of different concentrations of the test compounds on the metabolic reactions of phenacetin (CYP1A2), diclofenac sodium (CYP2C9), S-mephenytoin (CYP2C19), bufuralol hydrochloride (CYP2D6), and midazolam (CYP3A4/5) were assessed by liquid chromatography-tandem mass spectrometry (LC/MS/MS).

A 200 µL reaction system containing 30 µM phenacetin, 10 µM diclofenac sodium, 35 µM S-mephenytoin, 5 µM bufuralol hydrochloride, 3 µM midazolam, 1 mM reduced nicotinamide adenine dinucleotide phosphate (NADPH), test compounds (at concentrations of 0.1 µmol/L, 0.3 µmol/L, 1 µmol/L, 3 µmol/L, 10 µmol/L, and 30 µmol/L, respectively) or positive compound or blank control, and pooled human liver microsomes (0.2 mg/mL) (in 100 mmol/L phosphate-buffered saline, pH 7.4, containing 0.3 vol % of DMSO, 0.6 vol % of acetonitrile, and 0.1 vol % of methanol) was incubated at 37 °C for 5 min. Then, 200 µL of an acetonitrile solution containing 3% formic acid and 40 nM internal standard verapamil was added, and the mixture was centrifuged at 4000 rpm for 50 min. The mixture was cooled on ice for 20 min and centrifuged at 4000 rpm for 20 min to precipitate protein. 200 µL of the supernatant was taken for LC/MS/MS analysis.

The peak area was calculated based on the chromatogram.

The residual activity rate (%) was calculated using the following equation:$peak area rate = peak area of metabolite / peak area of internal standard$ 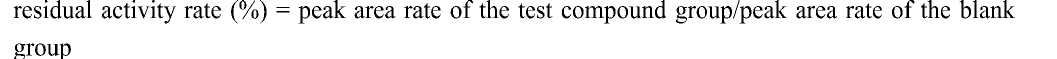 residual activity rate (%) = peak area rate of the test compound group/peak area rate of the blank group

The half maximal inhibitory concentrations (IC₅₀) against CYP were calculated using Excel XLfit 5.3.1.3.

The test results show that the compounds of the present disclosure had no significant inhibitory effect on multiple CYP subtypes, indicating that the compounds of the present disclosure are expected to have relatively good drug safety and relatively low drug-drug interactions.

### Test Example 4. Caco-2 Permeability Assay

The apparent permeability coefficient (Pₐₚₚ) of the analytical drug was determined by liquid chromatography-tandem mass spectrometry (LC/MS/MS) using a Caco-2 cell model.

In this test example: Caco-2 cells were purchased from American Type Culture Collection (ATCC); 4-hydroxyethyl piperazine ethane sulfonic acid (HEPES) was purchased from Beijing Solarbio Science & Technology Co., Ltd.; Hanks' balanced salt solution (HBSS) and non-essential amino acids (NEAA) were purchased from ThermoFisher Scientific; penicillin, streptomycin, and trypsin/EDTA were purchased from Solarbio; fetal bovine serum (FBS) and DMEM medium were purchased from Coming; HTS-96-well Transwell plates and other sterile consumables were purchased from Coming; Millicell resistance measuring system was purchased from Millipore; Cellometer^{®}K2 was purchased from Nexcelom Bioscience; Infinite 200 PRO microplate reader was purchased from Tecan; MTS2/4 orbital shaker was purchased from IKA Labortechnik.

### Step 1. cell culture and plating

Caco-2 was cultured in a cell culture flask. The incubator was set to be at 37 °C and 5% CO₂, with the relative humidity guaranteed to be 95%. Cells could be used for inoculation in the Transwell when at a confluency of 70-90%. Prior to cell inoculation, 50 µL of cell medium was added to each well of the upper Transwell chamber and 25 mL of cell medium was added to the lower culture plate. The culture plate was incubated in an incubator (37 °C, 5% CO₂) for 1 h and then inoculated with cells. After cell digestion, the cell suspension was pipetted and transferred to a round-bottomed centrifuge tube for centrifugation at 120 g for 5 min. The cells were resuspended in the medium to a final concentration of 6.86 × 10⁵ cells/mL. The cell suspension was then added to the upper chamber of the 96-well Transwell culture plate at 50 µL/well, with a final inoculation density of 2.4 × 10⁵ cells/cm². The medium was first changed 24 h after inoculation; the cells were cultured for 14-18 days, and the medium was changed every other day. The medium was changed as follows: Transwell chambers were separated from the receiver plate, medium in the receiver plate was discarded, then medium in the Transwell chambers was discarded, and finally, 75 µL of fresh medium was added to each chamber, and 25 mL of fresh medium was added to the receiver plate.

### Step 2. evaluation of integrity of monolayer cell membrane

After approximately 14 days of culture, the Caco-2 cells reached the confluency and completed differentiation. In this case, the cells could be used for permeability assay. The resistance of the monolayer membrane was measured using a resistance meter (Millipore, USA), and the resistance of each well was recorded. After the measurement, the Transwell culture plate was put back into the incubator. Calculation of resistance: measured resistance value (ohms) × membrane area (cm²) = TEER value (ohm· cm²); if TEER value < 230 ohms· cm², the well could not be used for permeability assay.

### Step 3. preparation of solution

2.38 g of HEPES and 0.35 g of sodium bicarbonate were dissolved in 900 mL of purified water, and then 100 mL of 10× HBSS was added. The mixture was stirred well, and the pH was adjusted to 7.4. Finally, the mixture was filtered to obtain 1 L of transport buffer (HBSS, 10 mM HEPES, pH 7.4). A 1 mM DMSO stock solution of test compound was diluted with the transport buffer to obtain 5 µM test solution. The control compound digoxin or minoxidil was diluted with DMSO to 2 mM and then diluted with the transport buffer described above to 10 µM to obtain a control compound test solution. In addition, DMSO was also diluted with the transport buffer described above to obtain a receiving end solution containing 0.5% DMSO.

### Step 4. drug permeability assay

The Transwell culture plate was taken out from the incubator. The monolayer cell membrane was rinsed twice with the transport buffer (10 mM HEPES, pH 7.4) and incubated at 37 °C for 30 min.

The transport rate of the compound from the apical side to the basolateral side was determined. 125 µL of test solution was added to each well of the upper chamber (apical side), and then 50 µL of solution was immediately transferred from the apical side to 200 µL of acetonitrile containing an internal standard (0.1 µM tolbutamide) to serve as an initial sample from the apical side to the basolateral side. 235 µL of receiving end solution was added to each well of the lower chamber (basolateral side).

The transport rate of the compound from the basolateral side to the apical side was determined. 285 µL of receiving end solution was added to each well of the upper chamber (apical side), and then 50 µL of solution was immediately transferred from the apical side to 200 µL of acetonitrile containing an internal standard (0.1 µM tolbutamide) to serve as an initial sample from the basolateral side to the apical side. 75 µL of test solution was added to each well of the lower chamber (basolateral side).

After the combination of the upper and lower transport devices, incubation was performed at 37 °C for 2 h.

After incubation, 50 µL of sample was taken from each well of the upper and lower chambers of the Transwell culture plate to new sample tubes. 200 µL of acetonitrile containing an internal standard (0.1 µM tolbutamide) was added to each sample tube, and then the mixture was vortexed for 10 min and centrifuged at 3220 g for 40 min. Then, 150 µL of supernatant was pipetted and diluted with 150 µL of water for LC-MS/MS analysis. All samples were prepared in triplicate.

The integrity of monolayer cell membranes after 2 h of incubation was evaluated by leakage of fluorescein, and the fluorescein stock solution was diluted with the transport buffer (10 mM HEPES, pH 7.4) to a final concentration of 100 µM. 100 µL of fluorescein solution was added to each well of the upper Transwell plate, and 300 µL of transport buffer (10 mM HEPES, pH 7.4) was added to each well of the lower receiver plate. After incubation at 37 °C for 30 min, 80 µL of solution was pipetted from the upper and lower layers of each well into a new 96-well plate. Fluoremetry was performed using a microplate reader at an excitation wavelength of 485 nm and an emission wavelength of 530 nm.

### Step 5. data analysis

All calculations were performed using Microsoft Excel. Peak areas were determined by using extracted ion chromatograms.

**The apparent permeability coefficient** (Pₐₚₚ, cm/s × 10⁻⁶) was calculated according to the following equation: ${P}_{app} = \frac{{V}_{A}}{Area \times time} \times \frac{{\left[drug\right]}_{receiver}}{{\left[drug\right]}_{initial , donor}}$

In the equation: V_{A} was the volume of the receiving end solution (Ap→B1 was 0.3 mL, and Bl→Ap was 0.1 mL); Area was the area of the membrane of the Transwell 96-well plate (0.143 cm²); time was the time for incubation (s); [drug]_{receiver} was the concentration of the drug at the receiving end; [drug]ᵢₙᵢₜᵢₐₗ, _{donor} was the initial concentration of the drug at the administration end.

**The efflux ratio** was calculated according to the following equation: $Efflux ratio = \frac{{P}_{app \left(B\to A\right)}}{{P}_{app \left(A\to B\right)}}$

In the equation, P_{app (B-A)} was the apparent permeability coefficient from the basolateral side to the apical side, and

P_{app (A-B)} was the apparent permeability coefficient from the apical side to the basolateral side.

**The recovery rate ("Percentage recovery" (%))** was calculated according to the following equation: $Percentage recovery \left(%\right) = \frac{{\left[drug\right]}_{receiver} \times {V}_{A} + {\left[drug\right]}_{donor} \times {V}_{D}}{{\left[drug\right]}_{initial , donor} \times {V}_{D}} \times 100 %$

In the equation, V_{A} was the solution volume at the receiving end (mL), and V_{D} was the solution volume at the administration end (mL).

**The leakage rate (Percentage leakage(%) or LY(%))** was calculated according to the following equation: $Percentage LY leakage \left(%\right) = \left(\frac{{l}_{receiver} \times 0.3}{{l}_{receiver} \times 0.3 + {l}_{donor} \times 0.1}\right) \times 100 %$

In the equation, *I_{receiver}* represented the fluorescence density of the receiving wells (0.3 mL), and I*_{donor}* represented the fluorescence density of the dosing wells (0.1 mL); the leakage rate was expressed as LY (%). LY <1.5% indicated that the monolayer cell membrane was intact. For individual LY > 1.5% cases, the final data could be accepted based on scientific judgment if the Pₐₚₚ value was close to that in other replicates.

The test results show that the compounds of the present disclosure exhibited relatively excellent *in vitro* membrane permeability, indicating that they are expected to have good oral absorption ability.

### Test Example 5: Assay on In Vivo Pharmacokinetics of Compounds of Present Disclosure in Rats

By using SD rats as test animals, drug concentrations in plasma of the rats at different time points after intravenous injection and intragastric administration of the compounds of present disclosure were determined by LC/MS/MS. *In vivo* pharmacokinetic behaviors of the compounds of present disclosure in rats were studied to evaluate the pharmacokinetic characteristics of the compounds. There were three healthy 6-8-week-old male SD rats in each group.

Intravenous injection: A certain amount of drug was weighed out, and then 10 vol % of N,N-dimethylacetamide, 33 vol % of triethylene glycol, and 57 vol % of physiological saline were added to prepare 1 mg/mL colorless clear and transparent liquid;

Intragastric administration: A certain amount of drug was weighed out, and then 0.5 wt % of hydroxypropyl methylcellulose, 0.1 vol % of tween 80, and 99.6 vol % of physiological saline were added to prepare 1 mg/mL white suspension.

After fasting overnight, SD rats were administered either intravenously (at a dose of 1 mg/kg) or intragastrically (at a dose of 5 mg/kg).

The compounds of the present disclosure were injected intravenously to the rats, and then 0.2 mL of blood was collected from jugular veins at 0.083 h, 0.25 h, 0.5 h, 1 h, 2 h, 4 h, 8 h, and 24 h after the administration and placed in a test tube containing EDTA-K2. The blood was then centrifuged at 4 °C and 4000 rpm for 5 min to separate plasma, and the plasma was stored at -75 °C. Alternatively, the compounds of the present disclosure were administered intragastrically to the rats, and then 0.2 mL of blood was collected from jugular veins at 0.25 h, 0.5 h, 1 h, 2 h, 4 h, 8 h, and 24 h after the administration and placed in a test tube containing EDTA-K2. The blood was then centrifuged at 4 °C and 3500 rpm for 10 min to separate plasma, and the plasma was stored at -75 °C.

Determination of the content of the test compounds in rat plasma after intragastric administration of the compounds at different concentrations: 30 µL of rat plasma at each time point after administration was taken, 200 µL (50 ng/mL) of a solution of internal standard dexamethasone in acetonitrile was added, and then the resulting mixture was vortexed for 30 s and centrifuged at 4 °C and 4700 rpm for 15 min. The supernatant of plasma samples was subjected to 3-fold dilution with water, and 2.0 µL of diluted solution was taken for LC-MS/MS analysis.

The test results show that the compounds of the present disclosure exhibited excellent pharmacokinetic properties, including relatively low apparent clearance, relatively long half-life, relatively high plasma exposure, and the like.

## Claims

1. A compound represented by formula (II) or a pharmaceutically acceptable salt thereof: 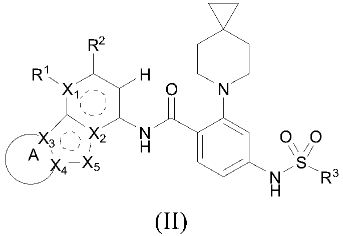 wherein in formula (II):
A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from halogen, OH, C₁-C₆ alkyl, O(C₁-C₄ alkyl), NH₂, NH(C₁-C₄ alkyl), or N(C₁-C₄ alkyl)₂, wherein the C₁-C₆ alkyl is optionally substituted with one or more groups independently selected from halogen, cyano, OH, NH₂, or O(C₁-C₄ alkyl);
X₁, X₂, X₃, X₄, and X₅ are each independently CR^{a} or nitrogen, wherein R^{a} are each independently selected from being absent, H, or halogen;
R¹ is absent, H, C₁-C₆ alkyl, cyano, C₁-C₆ alkoxy, (CH₂)ᵣ-C(O)R^{d}, halogen, OH, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂;
R² is H, OH, C₁-C₆ alkyl, cyano, C₁-C₆ alkoxy, C(O)R^{d}, halogen, C₁-C₆ haloalkyl, or C₁-C₆ haloalkoxy;
R^{d} is selected from OH, C₁-C₆ alkyl, O(C₁-C₄ alkyl), NH₂, NH(C₁-C₄ alkyl), or N(C₁-C₄ alkyl)₂; and
R³ is selected from C₁-C₆ alkyl or C₂-C₆ alkenyl, wherein the C₁-C₆ alkyl is optionally substituted with one or more halogen or OH.

2. The compound or the pharmaceutically acceptable salt thereof according to claim 1, wherein
R¹ is absent, H, C₁-C₆ alkyl, cyano, C₁-C₆ alkoxy, OH, halogen, (CH₂)ᵣ-C₃-C₁₀ cycloalkyl, (CH₂)ᵣ-C₅-C₁₀ cycloalkenyl, (CH₂)ᵣ-4- to 10-membered heterocyclyl, (CH₂)ᵣ-C₆-C₁₀ aryl, or (CH₂)ᵣ-5- to 10-membered heteroaryl, wherein r is 0, 1, 2, or 3, and the C₁-C₆ alkyl, C₁-C₆ alkoxy, C₃-C₁₀ cycloalkyl, C₅-C₁₀ cycloalkenyl, 4- to 10-membered heterocyclyl, C₆-C₁₀ aryl, or 5- to 10-membered heteroaryl is optionally substituted with one or more groups independently selected from halogen, CN, OH, NH₂, C₁-C₆ alkyl, C₃-C₆ cycloalkyl, 4- to 7-membered heterocyclyl, O(C₁-C₄ alkyl), NH(C₁-C₄ alkyl), or N(C₁-C₄ alkyl)₂; and
R³ is selected from C₁-C₆ alkyl, wherein the C₁-C₆ alkyl is optionally substituted with one or more halogen or OH;
the compound represented by formula (II) does not include the following compounds: 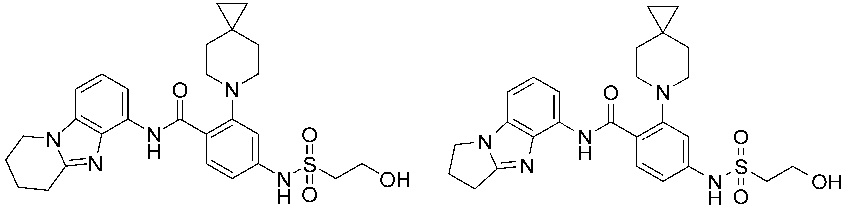 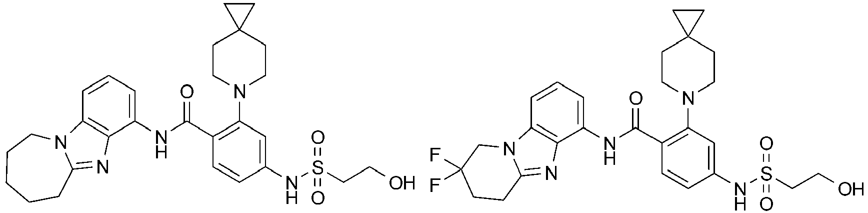 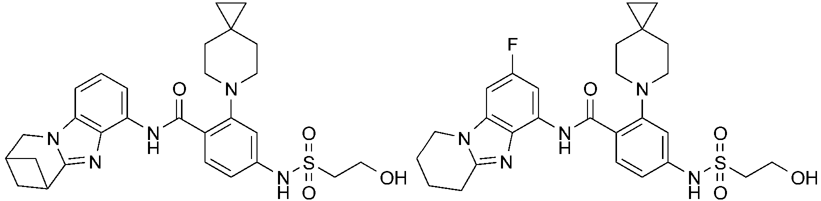 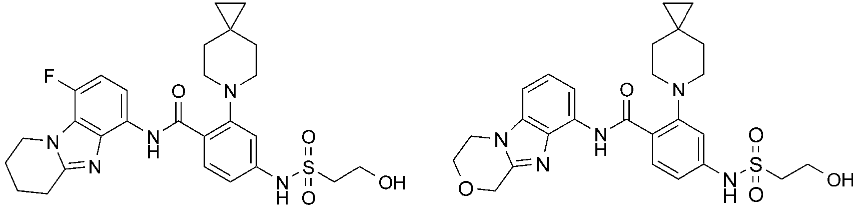 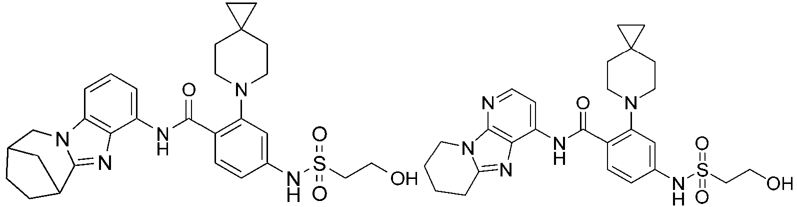 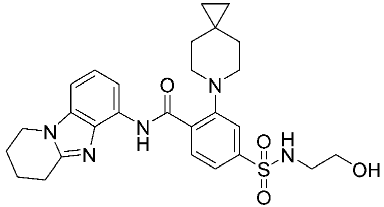 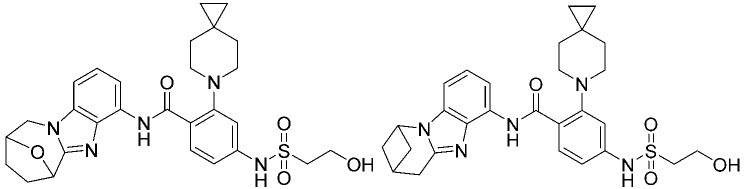 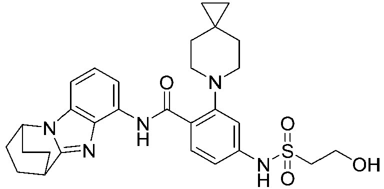 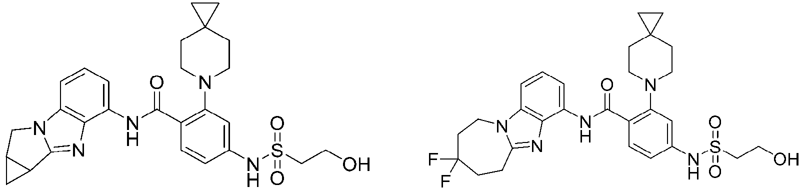 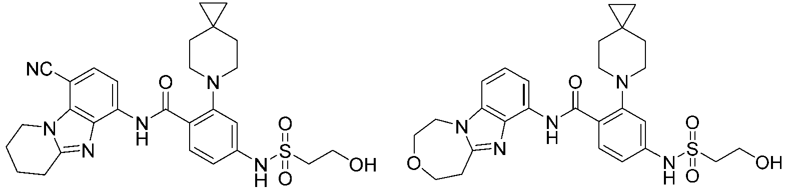 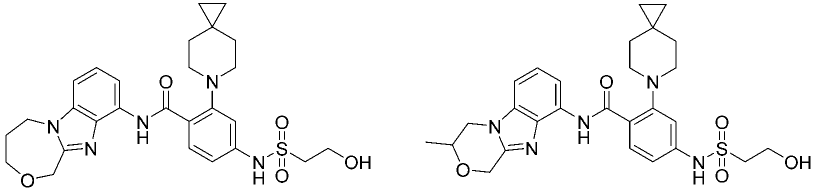 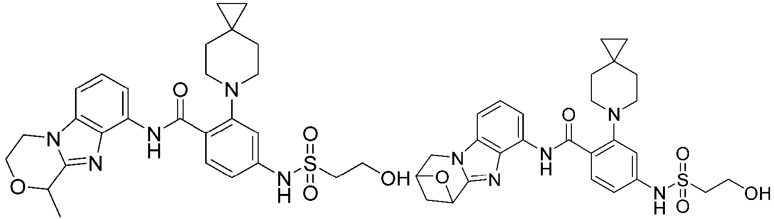 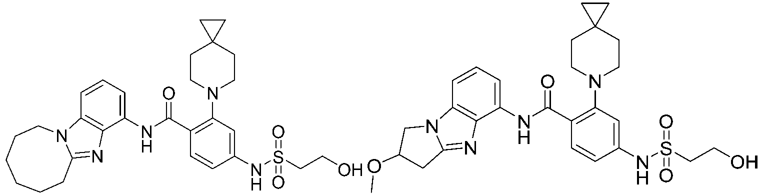 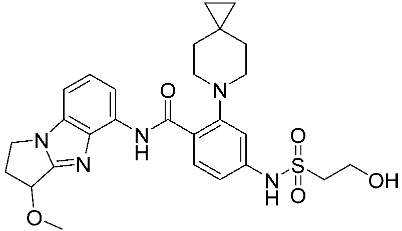 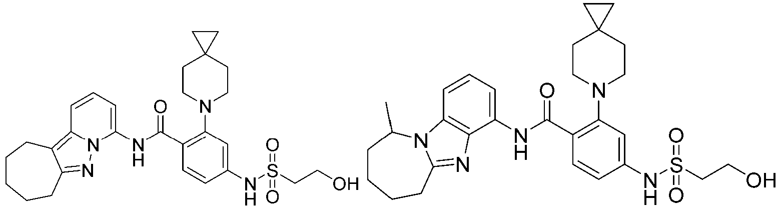 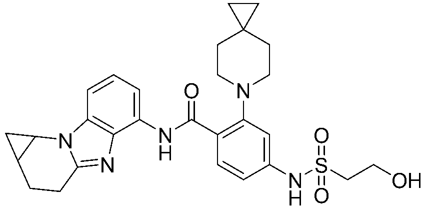 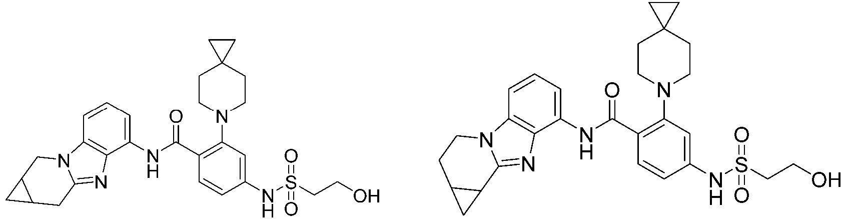 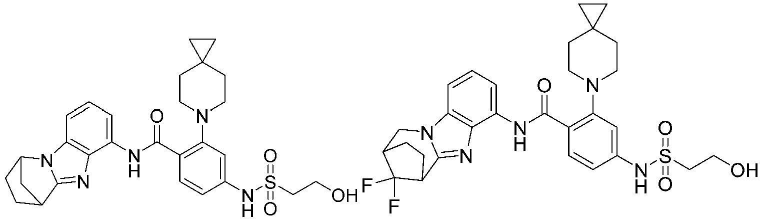 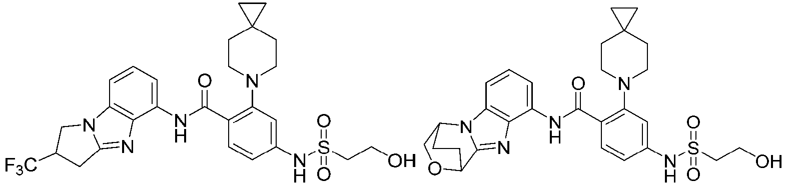 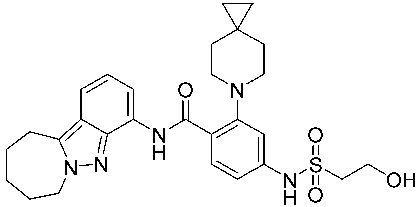 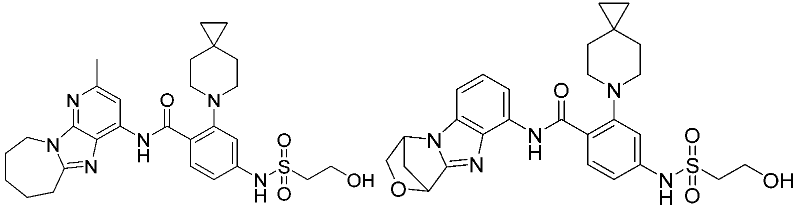 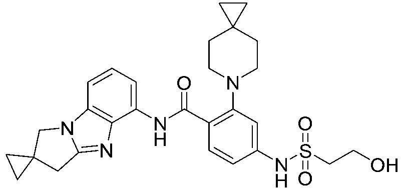 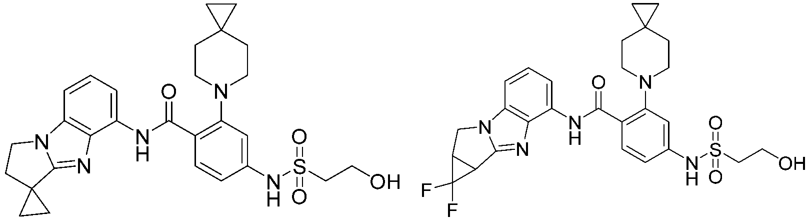 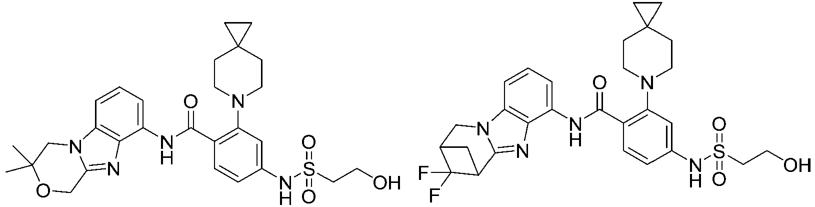 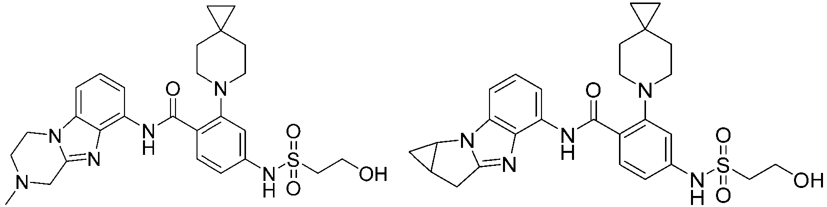 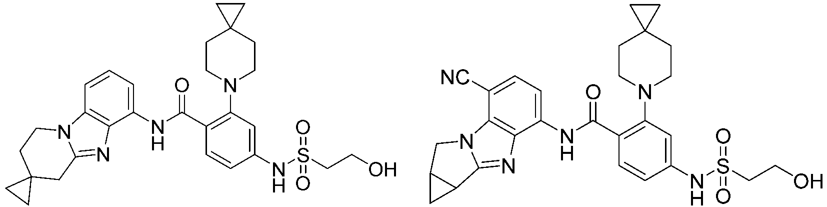 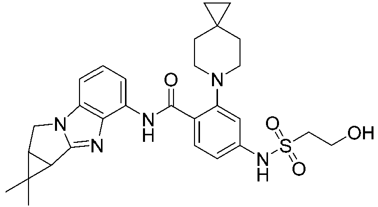 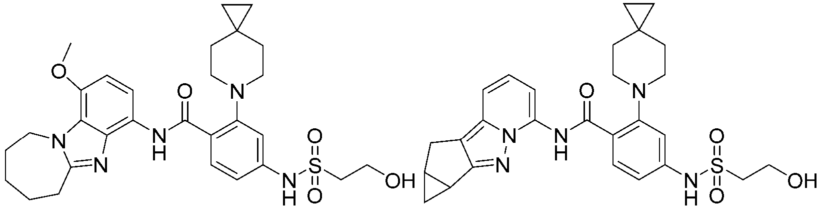 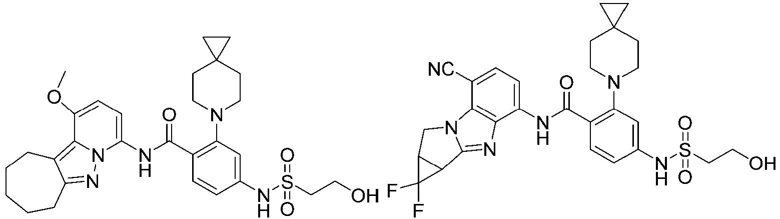 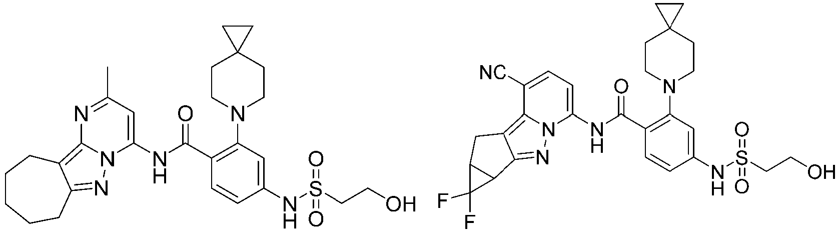 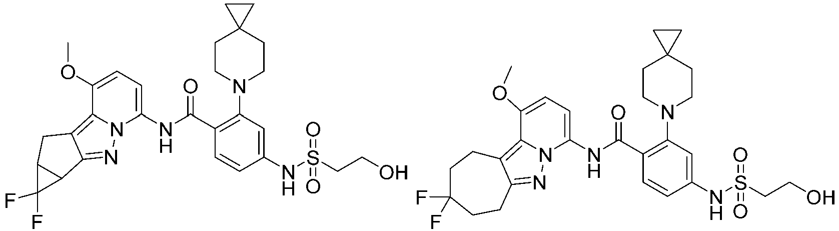 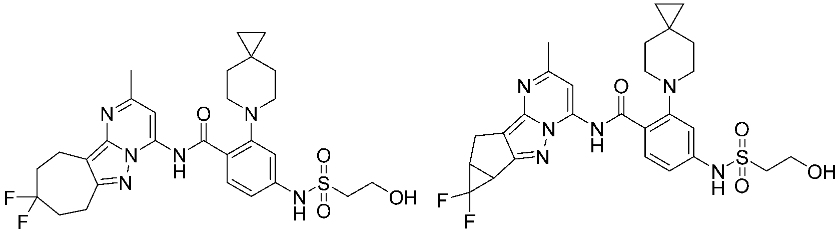 and

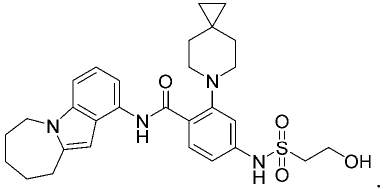

3. The compound or the pharmaceutically acceptable salt thereof according to claim 1 or 2, wherein both X₂ and X₅ are N.

4. The compound or the pharmaceutically acceptable salt thereof according to any one of claims 1-3, wherein the compound represented by formula (II) or the pharmaceutically acceptable salt thereof is selected from a compound represented by formula (III') or a pharmaceutically acceptable salt thereof:

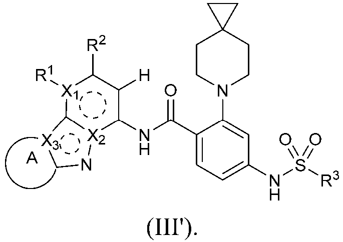

5. The compound or the pharmaceutically acceptable salt thereof according to claim 4, wherein X₁ is C or N, X₂ is N, and X₃ is C.

6. The compound or the pharmaceutically acceptable salt thereof according to any one of claims 1-5, wherein the compound does not include the following compounds:

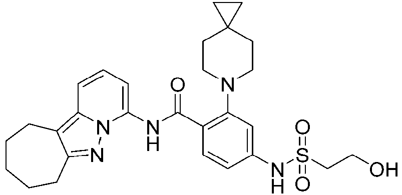

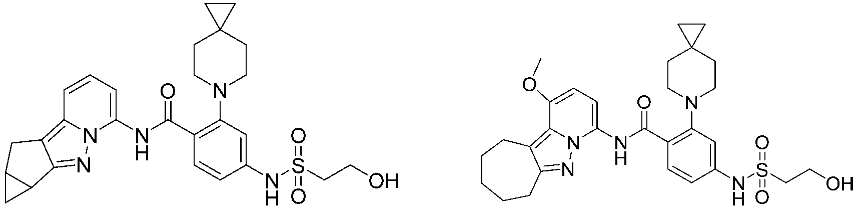

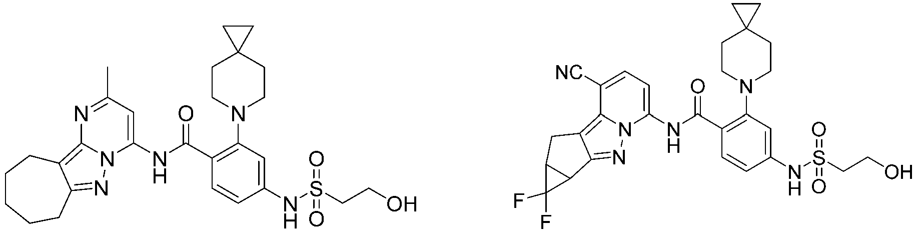

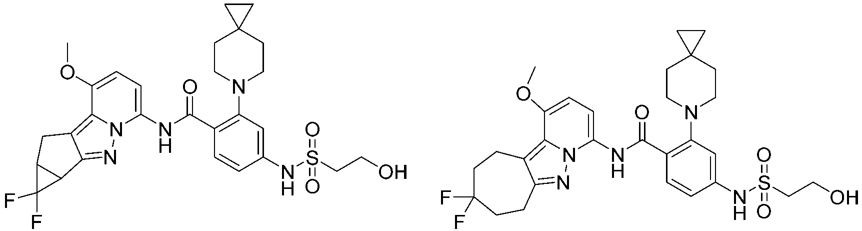

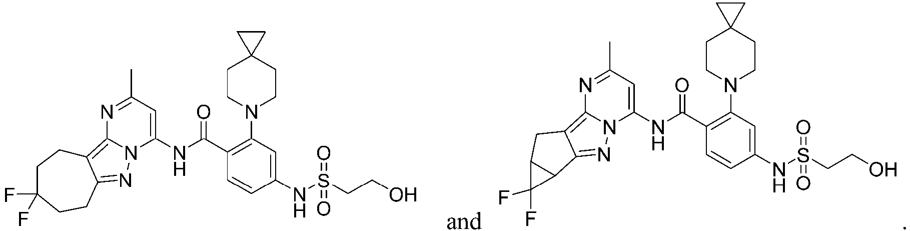

7. The compound or the pharmaceutically acceptable salt thereof according to any one of claims 1-6, wherein R¹ is absent, H, OH, C₁-C₆ alkyl, halogen, cyano, C₁-C₆ alkoxy, 5- to 10-membered heteroaryl, or C(O)R^{d}, wherein the C₁-C₆ alkyl is optionally substituted with one or more halogen, and the 5- to 10-membered heteroaryl is optionally substituted with one or more C₁-C₆ alkyl; or
R¹ is absent, H, OH, C₁-C₆ alkyl, halogen, cyano, C₁-C₆ alkoxy, or 5- to 10-membered heteroaryl, wherein the C₁-C₆ alkyl is optionally substituted with one or more halogen, and the 5- to 10-membered heteroaryl is optionally substituted with one or more C₁-C₆ alkyl; or
R¹ is absent, H, OH, C₁-C₆ alkyl, halogen, cyano, or C₁-C₆ alkoxy, wherein the C₁-C₆ alkyl is optionally substituted with one or more halogen; or
R¹ is absent, H, OH, methoxy, halogen, CN, CF₃, or 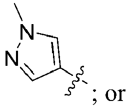
R¹ is absent, H, OH, methoxy, halogen, CN, or CF₃.

8. The compound or the pharmaceutically acceptable salt thereof according to any one of claims 1-7, wherein R² is H, OH, C₁-C₆ alkyl, cyano, C₁-C₆ alkoxy, or C(O)R^{d}; or
R² is H, OH, C₁-C₆ alkyl, cyano, or C₁-C₆ alkoxy; or
R² is H, OH, or C₁-C₄ alkyl.

9. The compound or the pharmaceutically acceptable salt thereof according to any one of claims 1-8, wherein R³ is selected from C₁-C₄ alkyl or C₂-C₃ alkenyl, wherein the C₁-C₄ alkyl is optionally substituted with one or more halogen or OH; or
R³ is selected from C₁-C₄ alkyl optionally substituted with one or more OH; or
R³ is selected from methyl, ethyl, vinyl, or ; or
R³ is selected from methyl, ethyl, or .

10. The compound or the pharmaceutically acceptable salt thereof according to any one of claims 1-9, wherein A is a monocyclic or bicyclic ring selected from a 4- to 10-membered carbocyclic ring or a 4- to 10-membered heterocyclic ring, wherein the 4- to 10-membered carbocyclic ring or 4- to 10-membered heterocyclic ring is optionally substituted with one or more R^{b}, and each R^{b} is independently selected from OH, F, or C₁-C₄ alkyl, wherein the C₁-C₄ alkyl is optionally substituted with one or more groups independently selected from F or O(C₁-C₄ alkyl).

11. A compound or a pharmaceutically acceptable salt thereof, wherein the compound is selected from one of the following structures:

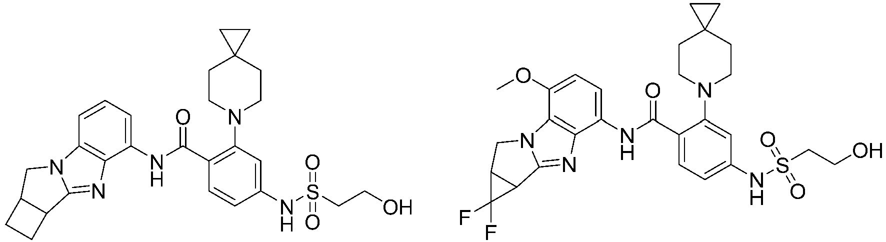

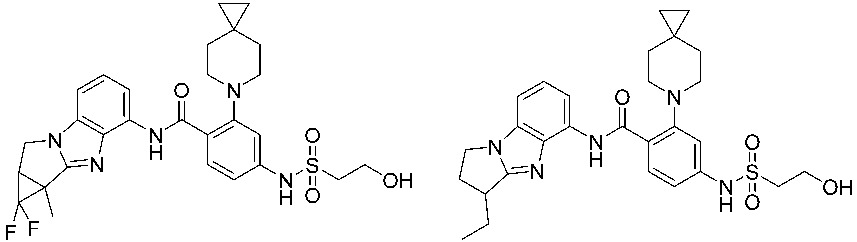

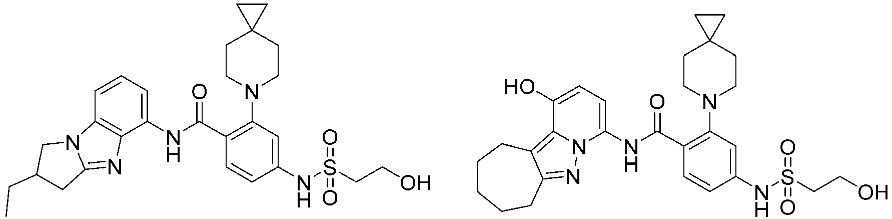

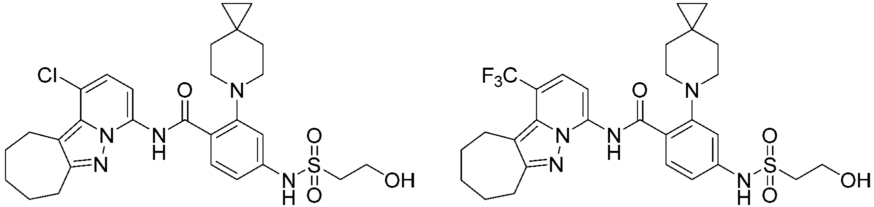

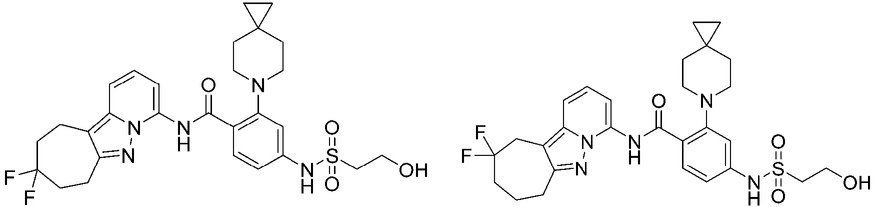

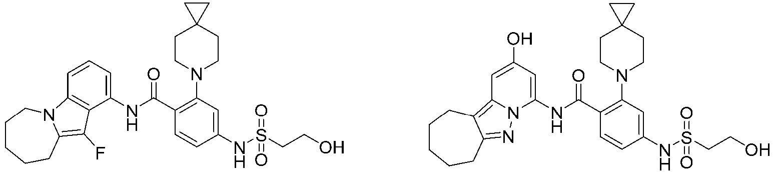

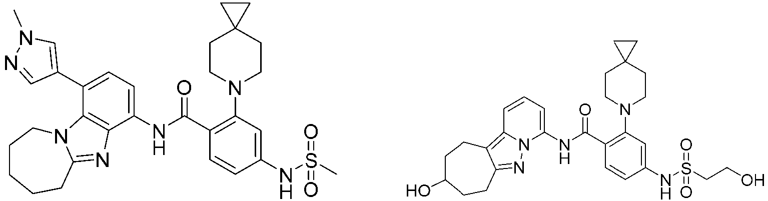

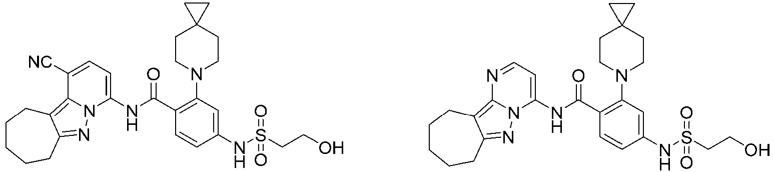

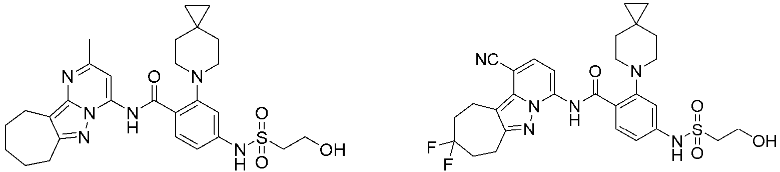

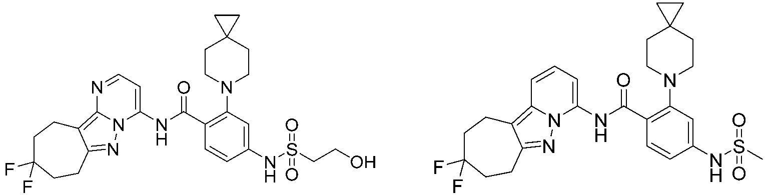

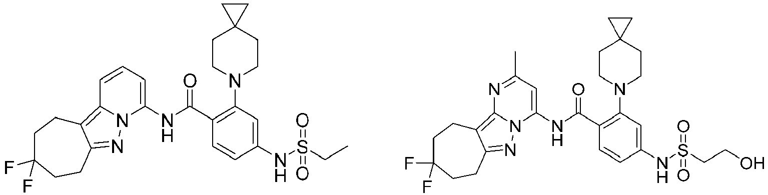

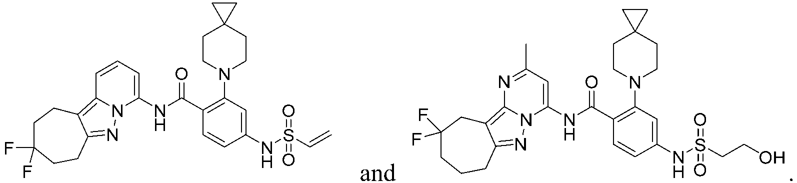

12. A pharmaceutical composition, comprising the compound or the pharmaceutically acceptable salt thereof according to any one of claims 1-11 and a pharmaceutically acceptable excipient.

13. Use of the compound or the pharmaceutically acceptable salt thereof according to any one of claims 1-11 or the pharmaceutical composition according to claim 12 in the preparation of a medicament for preventing or treating a KIF18A-related disease.
